(19) Europäisches Patentamt
European Patent Office
Office européen des brevets

(11) Veröffentlichungsnummer : **0 365 484 B1**

# (12) EUROPÄISCHE PATENTSCHRIFT

(45) Veröffentlichungstag der Patentschrift :
**07.01.93 Patentblatt 93/01**

(51) Int. Cl.$^5$ : **C07C 311/51,** C07D 307/22, C07D 333/38, C07D 211/90, A01N 47/28

(21) Anmeldenummer : **89810778.4**

(22) Anmeldetag : **11.10.89**

## (54) Sulfamoylphenylharnstoffe.

(30) Priorität : **20.10.88 CH 3914/88**

(43) Veröffentlichungstag der Anmeldung :
**25.04.90 Patentblatt 90/17**

(45) Bekanntmachung des Hinweises auf die Patenterteilung :
**07.01.93 Patentblatt 93/01**

(84) Benannte Vertragsstaaten :
**AT BE CH DE ES FR GB GR IT LI LU NL SE**

(56) Entgegenhaltungen :
**CH-A- 482 396**
**CH-A- 602 615**
**DE-B- 1 618 445**

(73) Patentinhaber : **CIBA-GEIGY AG**
**Klybeckstrasse 141**
**CH-4002 Basel (CH)**

(72) Erfinder : **Burckhardt, Urs, Dr.**
**Rittergasse 29**
**CH-4051 Basel (CH)**
Erfinder : **Soliman, Raafat, Prof. Dr.**
**45 Mahmoud El Deeb Street**
**Zizinia Alexandria (EG)**
Erfinder : **Töpfl, Werner, Dr.**
**Dorneckstrasse 68**
**CH-4143 Dornach (CH)**
Erfinder : **Waespe, Hans-Rudolf, Dr.**
**Feldstrasse 86**
**CH-4123 Allschwil (CH)**

EP 0 365 484 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-Acylsulfamoylphenylharnstoffe, welche sich zum Schützen von Kulturpflanzen vor der phytotoxischen Wirkung von Acylcyclohexandionherbiziden, Sulfonylharnstoffherbiziden, Chloracetanilidherbiziden und Aryloxyphenoxypropionsäureherbiziden eignen. Weiter betrifft die Erfindung die Herstellung der neuen Wirkstoffe und deren Verwendung zum Schutz von Kulturpflanzen. Ferner bilden den neuen Wirkstoff enthaltende Mittel und herbizide Mittel, die eine Kombination von Herbizid und Gegenmittel enthalten, sowie entsprechende Unkrautbekämpfungsmethoden weitere Erfindungsgegenstände. Schliesslich betrifft die Erfindung auch durch Behandlung mit N-Acylsulfamoylphenylharnstoffen gegen Herbizidwirkung geschütztes Kulturpflanzensaatgut.

Es ist bekannt, dass Herbizide aus den Stoffklassen der Sulfonylharnstoffe, Halogenacetanilide und Aryloxyphenoxypropionsäurederivate bei der Anwendung in wirksamer Dosis gelegentlich neben den zu bekämpfenden Unkräutern auch die Kulturpflanzen in gewissem Masse schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, das heisst die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, das heisst ein bestimmtes Gegenmittel eignet sich oft nur für eine bestimmte Kulturpflanze und einige wenige herbizide Stoffklassen.

So beschreibt die Britische Patentschrift 1,277,557 die Behandlung von Samen oder Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). Im US-Patent 4,618,361 werden Benzoxazinderivate mit Schutzwirkung vor der herbiziden Wirkung von Halogenacetaniliden und Sulfonylharnstoffen offenbart. Zum Schutz gegen Sulfonylharnstoffherbizide werden als Gegenmittel in der EP-A-122 231 Benzoyloximäther und in der EP-A-147 365 Phenylglyoxylsäurenitril-oxim, Naphthalindicarbonsäureanhydrid, ein Thiazolcarbonsäureester sowie Dichloracetamide vorgeschlagen. Ferner können Maispflanzen gemäss der DE-OS 2,402,983 wirksam vor Schädigung durch Chloracetanilide geschützt werden, indem man dem Boden als Gegenmittel ein N-disubstituiertes Dichloracetamid zuführt. Derartige Verbindungen werden gemäss DE-OS 2,828,265 und 2,828,293 auch als Antidotes gegen herbizide Acetanilide verwendet.

Es wurde nun gefunden, dass sich überraschenderweise eine Gruppe von N-Acylsulfamoylphenylharnstoffen hervorragend dazu eignet, Kulturpflanzen gegen schädigende Wirkung von Sulfonylharnstoffherbiziden, Chloracetanilidherbiziden oder Aryloxyphenoxypropionsäureherbiziden zu schützen. Diese N-Acylsulfamoylphenylharnstoffe werden daher im folgenden auch als "Gegenmittel", "Antidot" oder "Safener" bezeichnet.

Die erfindungsgemäss vorgeschlagenen neuen N-Acylsulfamoylphenylharnstoffe entsprechen der allgemeinen Formel I

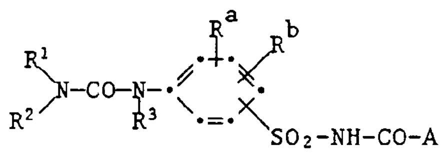

(I)

worin

A für einen Rest aus der Gruppe

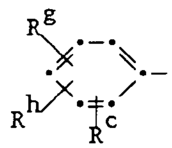 , 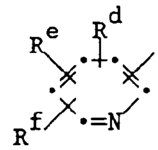 , 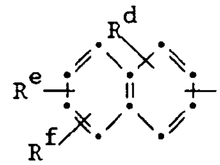 ,

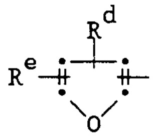 oder 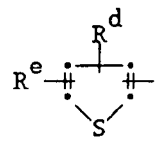 ,

$R^1$ und $R^2$ unabhängig voneinander für Wasserstoff, $C_1$-$C_8$-Alkyl, $C_3$-$C_8$-Cycloalkyl, $C_3$-$C_6$-Alkenyl, $C_3$-$C_6$-Alkinyl,

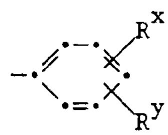

oder durch $C_1$-$C_4$-Alkoxy oder

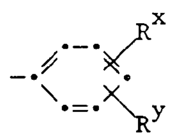

substituiertes $C_1$-$C_4$-Alkyl, oder
$R^1$ und $R^2$ gemeinsam für eine $C_4$-$C_6$-Alkylenbrücke oder eine durch Sauerstoff, Schwefel, SO, $SO_2$, NH oder -N($C_1$-$C_4$-Alkyl)- unterbrochene $C_4$-$C_6$-Alkylenbrücke,
$R^3$ für Wasserstoff oder $C_1$-$C_4$-Alkyl,
$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $-COOR^j$, $-CONR^kR^m$, $-COR^n$, $-SO_2NR^kR^m$ oder $-OSO_2$-$C_1$-$C_4$-Alkyl, oder $R^a$ und $R^b$ gemeinsam für eine $C_3$-$C_4$-Alkylenbrücke, die durch Halogen oder $C_1$-$C_4$-Alkyl substituiert sein kann, oder eine $C_3$-$C_4$-Alkenylenbrücke, die durch Halogen oder $C_1$-$C_4$-Alkyl substituiert sein kann, oder eine $C_4$-Alkadienylenbrücke, die durch Halogen oder $C_1$-$C_4$-Alkyl substituiert sein kann,
$R^g$ und $R^h$ unabhängig voneinander für Wasserstoff, Halogen, $C_1$-$C_4$-Alkyl, Trifluormethyl, Methoxy, Methylthio oder $-COOR^j$ stehen, wobei
$R^c$ Wasserstoff, Halogen, $C_1$-$C_4$-Alkyl oder Methoxy,
$R^d$ Wasserstoff; Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $-COOR^j$ oder $-CONR^k R^m$,
$R^e$ Wasserstoff, Halogen, $C_1$-$C_4$-Alkyl, $-COOR^j$, Trifluormethyl oder Methoxy, oder $R^d$ und $R^e$ gemeinsam für eine $C_3$-$C_4$-Alkylenbrücke,
$R^f$ Wasserstoff, Halogen oder $C_1$-$C_4$-Alkyl,
$R^x$ und $R^y$ unabhängig voneinander Wasserstoff, Halogen, $C_1$-$C_4$-Alkyl, $C_1$-$C_4$-Alkoxy, $C_1$-$C_4$-Alkylthio, $-COOR^4$, Trifluormethyl, Nitro oder Cyan,
$R^j$, $R^k$ und $R^m$ unabhängig voneinander Wasserstoff oder $C_1$-$C_4$-Alkyl,
$R^k$ und $R^m$ gemeinsam eine $C_4$-$C_6$-Alkylenbrücke oder eine durch Sauerstoff,
NH oder -N($C_1$-$C_4$-Alkyl)- unterbrochene $C_4$-$C_6$-Alkylenbrücke, und
$R^n$ $C_1$-$C_4$-Alkyl, Phenyl oder durch Halogen, $C_1$-$C_4$-Alkyl, Methoxy, Nitro oder Trifluormethyl substituiertes Phenyl bedeuten,
sowie die Verbindungen

1 - [4-(N-3-Chlormethylbenzoyl-sulfamoyl)-phenyl]-3-methyl-harnstoff,
1-[4-(N-2-Äthoxybenzoyl-sulfamoyl)-phenyl]-3-methyl-harnstoff,
1-[4-(N-3-Äthoxybenzoyl-sulfamoyl)-phenyl]-3-methyl-harnstoff,
1-[4-(N-4-Äthoxybenzoyl-sulfamoyl)-phenyl]-3-methyl-harnstoff,

1-[4-(N-2-Methylsulfonylbenzoyl-sulfamoyl)-phenyl]-3-methyl-harnstoff,
1-[4-(N-2-Nitrobenzoyl-sulfamoyl)-phenyl]-3-methyl-harnstoff,
1-[4-(N-3-Nitrobenzoyl-sulfamoyl)-phenyl]-3-methyl-harnstoff,
1-[4-(N-4-Nitrobenzoyl-sulfamoyl)-phenyl]-3-methyl-harnstoff,
1-[4-(N-2-Acetylbenzoyl-sulfamoyl)-phenyl]-3-methyl-harnstoff,
1-[4-(N-4-Acetylbenzoyl-sulfamoyl)-phenyl]-3-methyl-harnstoff,
1-[4-(N-3-Cyanobenzoyl-sulfamoyl)-phenyl]-3-methyl-harnstoff,
1-[4-(N-4-Cyanobenzoyl-sulfamoyl)-phenyl]-3-methyl-harnstoff,
1-[4-(N-3,4-Dinitrobenzoyl-sulfamoyl)-phenyl]-3-methyl-harnstoff,
1-[4-(N-3,5-Dinitrobenzoyl-sulfamo yl)-phenyl]-3-methyl-harnstoff,
1-[4-(N-2-Chlor-3-nitrobenzoyl-sulfamoyl)-phenyl]-3-methyl-harnstoff,
1-[4-(N-2-Chlor-4-nitrobenzoyl-sulfamoyl)-phenyl] -3-methyl-harnstoff,
1-[4-(N-2-Chlor-5-nitrobenzoyl-sulfamoyl)-phenyl] -3-methyl-harnstoff,
1 -[4-(N-3-Chlor-2-nitrobenzoyl-sulfamoyl)-phenyl]-3-methyl-harnstoff,
1-[4-(N-4-Chlor-2-nitrobenzoyl-sulfamoyl)-phenyl]-3-methyl-harnstoff,
1-[4-(N-3-Chlor-3-nitrobenzoyl-sulfamoyl)-phenyl]-3-methyl-harnstoff,
1-[4-(N-5-Chlor-2-nitrobenzoyl-sulfamoyl)-phenyl]-3-methyl-harnstoff,
1-[4-(N-2-Methyl-3-nitrobenzoyl-su lfamoyl)-phenyl]-3-methyl-harnstoff,
1-[4-(N-2-Methyl-5-nitrobenzoyl-sulfamoyl)-phenyl]-3-methyl-harnstoff,
1-[4-(N-2-Methyl-6-nitrobenzoyl-sulfamoyl)-phenyl]-3-methyl-harnstoff,
1-[4-(N-3-Methyl-2-nitrobenzoyl-sulfamoyl)-phenyl]-3-methyl-harnstoff,
1-[4-(N-3-Methyl-4-nitrobenzoyl-sulfamoyl)-phenyl]-3-methyl-harnstoff,
1-[4-(N-4-Methyl-3-nitrobenzoyl-sulfamoyl)-phenyl]-3-methyl-harnstoff,
1-[4-(N-5-Methyl-2-nitrobenzoyl-sulfamoyl)-phenyl] -3-methyl-harnstoff und
1-[4-(N-4-Fluor-3-nitrobenzoyl-sulfamoyl)-phenyl]-3-methyl-harnstoff.

Unter Halogen ist in den Definitionen Fluor, Chlor, Brom und Jod, vorzugsweise jedoch Fluor, Chlor und Brom, insbesondere Chlor zu verstehen. In den Definitionen ist unter Alkyl geradkettiges oder verzweigtes Alkyl zu verstehen; zum Beispiel Methyl, Aethyl, n-Propyl, i-Propyl oder die vier isomeren Butyl. Längerkettige Alkylgruppen umfassen die Isomeren von Pentyl, Hexyl, Heptyl oder Oktyl, wobei jeweils die unverzweigten Ketten bevorzugt sind. Unter Alkoxy ist zu verstehen: Methoxy, Aethoxy, n-Propyloxy, i-Propyloxy oder die vier isomeren Butyloxyreste, insbesondere aber Methoxy, Aethoxy oder i-Propyloxy. Durch Alkoxy substituiertes Alkyl steht vorzugsweise für Methoxymethyl, Aethoxymethyl, Methoxyäthyl und Aethoxyäthyl, insbesondere aber Methoxyäthyl. Durch gegebenenfalls substituiertes Phenyl substituiertes Alkyl steht vorzugsweise für Abkömmlinge von Phenyläthyl oder Benzyl. $C_3$-$C_6$-Alkenyl und $C_3$-$C_6$-Alkinylreste in der Definition von $R^1$ und $R^2$ zeichnen sich dadurch aus, dass sie über ein gesättigtes Kohlenstoffatom an das sie tragende Stickstoffatom gebunden sind. Typisch Alkenyl- und Alkinylreste sind Allyl, 2-Butenyl, Methallyl, 3-Butenyl, Propargyl, 2-Butinyl, 3-Butinyl oder 2-Pentenyl. Beispiele für Cycloalkyl sind Cyclopropyl, Cyclopentyl, Cyclohexyl oder Cycloheptyl, vorzugsweise aber Cyclopentyl und Cyclohexyl. Heterocyclen welche gemeinsam von $R^1$ und $R^2$ oder gemeinsam vom $R^5$ und $R^6$ mit dem sie tragenden Stickstoffatom gebildet werden sind Pyrrolidin, Piperidin, Pyrazolidin, Imidazolidin, Oxazolidin, Thiazolidin, Morpholin, Thiomorpholin, Piperazin oder Hexahydroazepin sowie bei schwefelhaltigen Ringen deren Oxidationsprodukte. In Alkylthio, Alkylsulfinyl oder Alkylsulfonyl hat Alkyl die oben aufgezählten konkreten Bedeutungen.

Wenn $R^a$ und $R^b$ gemeinsam für eine $C_3$-$C_4$-Alkylenbrücke, $C_3$-$C_4$-Alkenylenbrücke oder $C_4$-Alkadienylenbrücke, die jeweils durch Halogen oder $C_1$-$C_4$-Alkyl substituiert sein kann, stehen, werden zusammen mit dem Phenylring, an welchem die Brücke gebunden ist, zweikernige Systeme gebildet wie beispielsweise 1,2,3,4-Tetrahydronaphtalin, 1-Chlor-2-Methyl-3,4-dihydronaphtalin, Indan, 1,2-Dihydronaphtalin, Inden, Naphtalin, 2-Methylnaphtalin, 1-n-Butylnaphtalin, 2-Ethylnaphtalin oder 1-Chlornaphtalin.

Wenn die Substituenten $R^d$ und $R^e$ gemeinsam für eine $C_3$-$C_4$-Alkylenbrücke stehen, so werden zusammen mit dem Ringsystem, an welches sie gebunden sind, mehrkernige Systeme gebildet wie etwa 2,3-Tetramethylenthiophen, 2,3-Trimethylenthiophen, 2,3-Tetramethylenfuran, 3,4-Tetramethylenpyridin oder

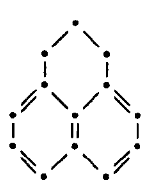

Wegen ihrer guten Wirkung als Herbizidantagonisten sind solche Verbindungen hervorzuheben, in denen entweder

a) $R^b$ für Wasserstoff steht, oder

b) die Sulfamoylgruppe die 4-Stellung des Phenylring besetzt, oder

c) $R^2$ und $R^3$ für Wasserstoff stehen, oder

d) A für die Gruppe

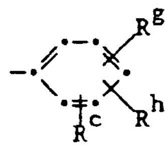

steht.

Eine bevorzugte Gruppe von Verbindungen der Formel I bilden diejenigen, worin $R^b$ Wasserstoff bedeutet und die Sulfamoylgruppe die 4-Stellung am Phenylring einnimmt. Weiter bevorzugt sind die Verbindungen, worin $R^2$, $R^3$ und $R^b$ Wasserstoff bedeuten und die Sulfamoylgruppe die 4-Stellung am Phenylring einnimmt.

Unter den Verbindungen der Untergruppe d) sind weiter diejenigen bevorzugt, worin $R^2$, $R^3$ und $R^b$ für Wasserstoff stehen und die Sulfamoylgruppe die 4-Stellung am Phenylring besetzt.

Ganz besonderes Interesse verdienen unter den erfindungsgemässen Verbindungen solche, die der Unterformel Ia

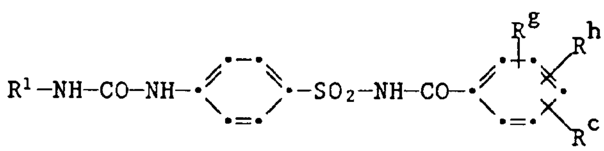

(Ia)

worin $R^1$, $R^c$, $R^g$ und $R^h$ die unter Formel I gegebenen Bedeutungen haben, zuzuordnen sind.

Eine weitere Bevorzugung hinsichtlich ihrer Wirkung geniessen die Verbindungen der Formel Ia, worin $R^c$ Wasserstoff bedeutet. Eine weitere speziell zu erwähnende Untergruppe davon stellen die Verbindungen der Formel Ia dar, in denen die Reste $R^1$, $R^g$ und $R^h$ für $C_1$-$C_4$-Alkylgruppen stehen.

Als bevorzugte erfindungsgemässe Einzelverbindungen aus dem Umfang der Formel I sind zu nennen:
1-[4-(N-4-Methylbenzoyl-sulfamoyl)-phenyl]-3-methylharnstoff,
1-[4(N-3-Methylbenzoyl-sulfamoyl)-phenyl]-3-methylharnstoff,
1-[4-(N-4-tert.Butylbenzoyl-sulfamoyl)-phenyl]-3-methylharnstoff,
1-[4-(N-3-Trifluormethylbenzoyl-sulfamoyl)-phenyl]-3-methylharnstoff,
1-[4-(N-4-Nitrobenzoyl-sulfamoyl)-phenyl]-3-methylharnstoff,
1-[4-(N-2,3-Dimethylbenzoyl-sulfamoyl)-phenyl]-3-methylharnstoff,
1-[4-(N-3,4-Dimethylbenzoyl-sulfamoyl)-phenyl]-3-methylharnstoff,
1-[4-(N-3,4-Dimethylbenzoyl-sulfamoyl)-phenyl]-3,3-dimethylharnstoff,
1-[4-(N-3,4-Dimethylbenzoyl-sulfamoyl)-phenyl]-3-äthylharnstoff,
1-[4-(N-3,4-Dimethylbenzoyl-sulfamoyl)-phenyl]-3-allylharnstoff,
1-[4-(N-3,4-Dimethylbenzoyl-sulfamoyl)-phenyl]-3-phenylharnstoff,
1-[4-(N-3,5-Dimethylbenzoyl-sulfamoyl)-phenyl]-3-methylharnstoff,
1-[4-(N-3,4-Dichlorbenzoyl-sulfamoyl)-phenyl]-3-methylharnstoff,
1-[4-(N-3,4-Dimethoxybenzoyl-sulfamoyl)-phenyl]-3-methylharnstoff,
1-[4-(N-3,4-Dimethoxybenzoyl-sulfamoyl)-phenyl]-3,3-dimethylharnstoff,
1-[4-(N-2,4,5-Trimethoxybenzoyl-sulfamoyl)-phenyl]-3-methylharnstoff,
1-[4-(N-1-Naphthylcarbonyl-sulfamoyl)-phenyl]-3-methylharnstoff,
1-[4-(N-2-Furylcarbonyl-sulfamoyl)-phenyl]-3-methylharnstoff,
1-[4-(N-2-Furylcarbonyl-sulfamoyl)-phenyl]-3,3-dimethylharnstoff,
1-[4-(N-2-Thienylcarbonyl-sulfamoyl)-phenyl]-3-methylharnstoff,
1-[4-(N-Piperonyloyl-sulfamoyl)-phenyl]-3-methylharnstoff,
1-[4-(N-3-Methylbenzoyl-sulfamoyl)-phenyl]-3,3-dimethylharnstoff,
1-[4-(N-3-Trifluormethylbenzoyl-sulfamoyl)-phenyl]-3-cyclopropylharnstoff,

1-[3-(N-3,4-Dimethylbenzoyl-sulfamoyl)-phenyl]-3-methylharnstoff, und
1-[3-(N-2-Furylcarbonyl-sulfamoyl)-phenyl]-3,3-dimethylharnstoff,

Die Verbindungen der Formel I, worin $R^1$ die unter Formel I gegebenen Bedeutungen mit Ausnahme von Wasserstoff hat und $R^2$ Wasserstoff bedeutet, werden hergestellt, indem man ein Sulfamoylanilin der Formel V

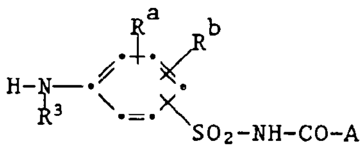

(V)

worin A, $R^3$, $R^a$ und $R^b$ die unter Formel I gegebenen Bedeutungen haben, mit einem Isocyanat der Formel VI

$R^1$-N=C=O (VI)

worin $R^1$ die unter Formel I gegebenen Bedeutungen mit Ausnahme von Wasserstoff hat, umsetzt.

Durch Umsetzung mit Alkylierungsreagenzien der Formel

Lg - $R^2$

worin Lg für eine Abgangsgruppe wie Halogen, Tosyl, $C_6H_5$-$SO_3$- oder $CH_3OSO_3$- steht, und $R^2$ die unter Formel I gegebenen Bedeutungen mit Ausnahme von Wasserstoff hat, können solche Verbindungen der Formel I erzeugt werden, in denen $R^2$ eine von Wasserstoff verschiedene Bedeutung hat. Die Umsetzung der Anilinderivate der Formel V mit dem Isocyanat der Formel VI kann sowohl lösungsmittelfrei als auch in Gegenwart eines aprotischen inerten organischen Lösungsmittels erfolgen. Die Umsetzungen werden vorteilhafterweise in einem Lösungsmittel vorgenommen. Geeignete Lösungsmittel sind Kohlenwasserstoffe wie Benzol, Toluol, Xylol oder Cyclohexan, Tetrachlorkohlenstoff oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, N,N-Dimethylaminopyridin, Pyridin, 1,4-Diazabicyclo[2.2.2]-octan, 1,5-Diazabicyclo-[4.3.0]non-5-en oder 1,8-Diazabicyclo[5.4.0]undec-7-en geeignet. Die Endprodukte der Formel I können entweder direkt als Kristallisat oder durch Einengen und/oder Verdampfen des Lösungsmittels isoliert und durch Umkristallisation oder Zerreiben des festen Rückstandes in Lösungsmitteln, in denen sie sich nicht gut lösen, gereinigt werden.

Nach einem zweiten Verfahren erhält man die Wirkstoffe der Formel I, indem man einen Sulfamoylphenylharnstoff der Formel VII

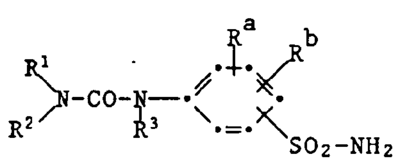

(VII)

worin $R^1$, $R^2$, $R^3$, $R^a$ und $R^b$ die unter Formel I gegebenen Bedeutungen haben, mit einem Carbonsäurehalogenid der Formel VIII

Hal-CO-A (VIII)

worin A die unter Formel I gegebenen Bedeutungen hat und Hal Chlor oder Brom bedeutet, acyliert.

Die Reaktion des Sulfamoylphenylharnstoffs der Formel VII mit dem Acylierungsreagenz der Formel VIII wird mit Vorteil in Gegenwart eines säurebindenden Mittels in einem inerten organischen Lösungsmittel ausgeführt. Geeignete Lösungmittel sind Kohlenwasserstoffe wie Benzol, Toluol, Xylol oder Cyclohexan, Tetrachlorkohlenstoff oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20°

und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, N,N-Dimethylaminopyridin, Pyridin, 1,4-Diazabicyclo[2.2.2]-octan, 1,5-Diazabicyclo-[4.3.0]non-5-en oder 1,8-Diazabicyclo[5.4.0]-undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden. Diese Basen können gleichzeitig als säurebindende Mittel eingesetzt werden. Die Endprodukte der Formel I können entweder direkt als Kristallisat oder durch Einengen und/oder Verdampfen des Lösungsmittels isoliert und durch Umkristallisation oder Zerreiben des festen Rückstandes in Lösungsmitteln, in denen sie sich nicht gut lösen, gereinigt werden.

Die Ausgangsverbindungen der Formeln V, VI, VII und VIII sind allgemein bekannt. Die Verbindungen der Formeln VI und VII sind teilweise im Handel erhältlich. Verbindungen der Formeln V und VIII, für welche in der Literatur noch keine konkreten Herstellungsverfahren beschrieben worden sind, können gemäss den folgenden Reaktionsschemata unter den für die einzelnen Reaktionsschritte üblichen Bedingungen hergestellt werden:

Schema 1

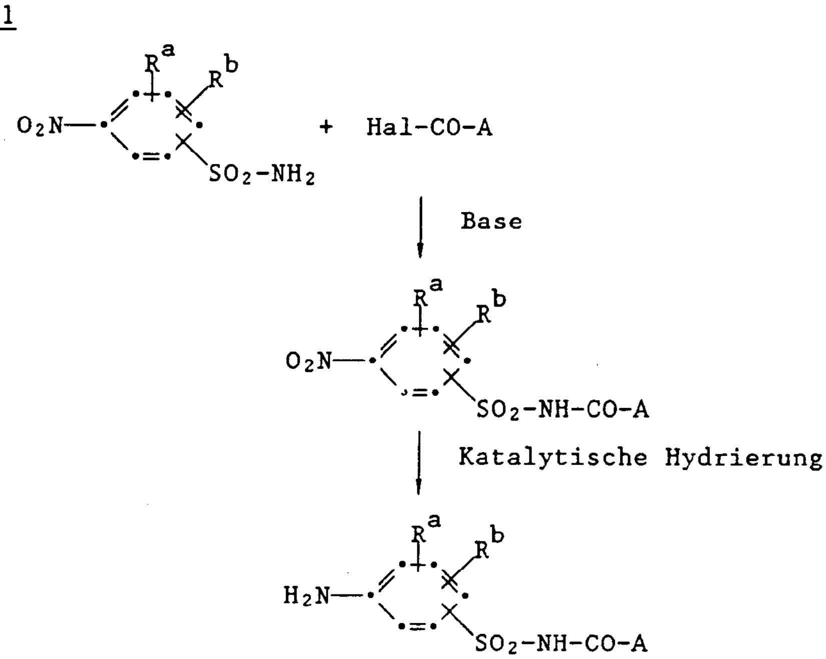

Schema 2

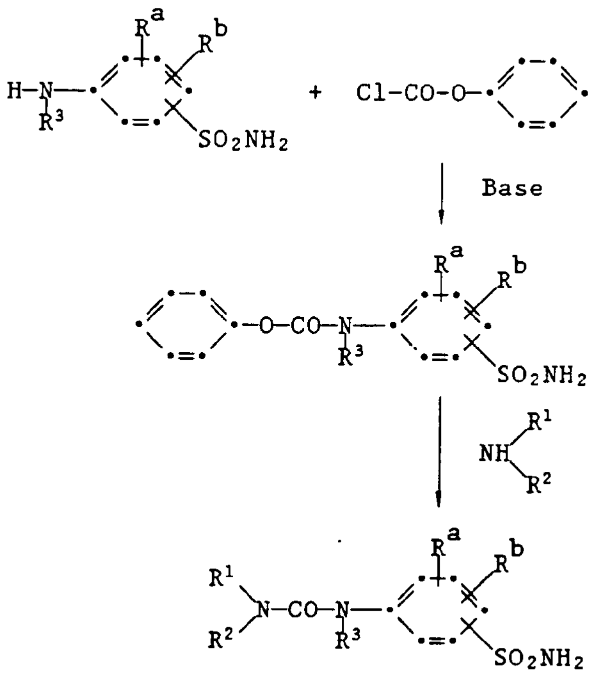


Schema 3

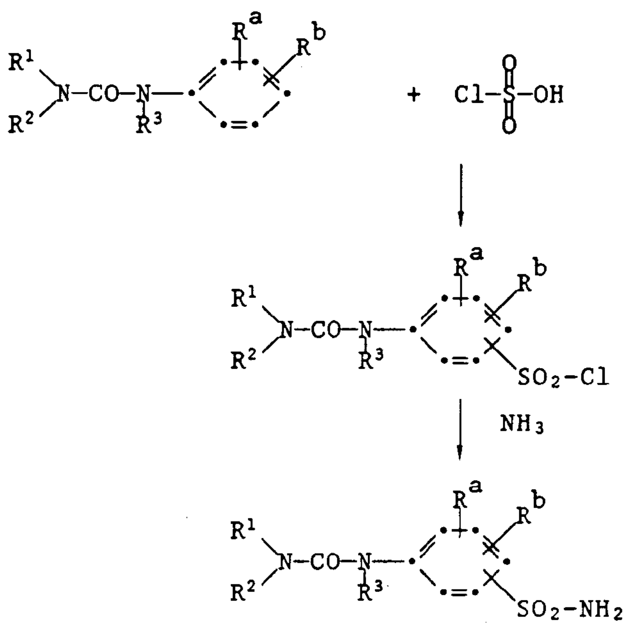


Die Verbindungen der Formel I sind unter normalen Laborbedingungen stabile Substanzen. Sie zersetzen sich bei der Lagerung bei Raumtemperatur nicht.

Ein Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation des Herbizids erfolgen. Die Behandlung der Pflanze kann jedoch auch durch gleichzeitige Applikation von Herbizid und Gegenmittel (Tankmischung) erfolgen. Die premergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbizid von 5:1 bis 1:50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1-10 g Gegenmittel benötigt. In der Regel wird mit 0,1-5 g Gegenmittel pro kg Samen bereits die volle Schutzwirkung erreicht. Falls das Gegenmittel kurz vor der Aussaat durch Samenquellung (seed soaking) appliziert werden soll, so werden zweckmässig Lösungen des Gegenmittels verwendet, welche den Wirkstoff in einer Konzentration von 1-10000 ppm enthalten. In der Regel wird mit Konzentrationen des Gegenmittels von 100-1000 ppm die volle Schutzwirkung erreicht.

In der Regel liegt zwischen protektiven Massnahmen, wie Samenbeizung und Behandlung von Stecklingen mit einem Gegenmittel der Formel I und der möglichen späteren Feldbehandlung mit Herbiziden ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Herbizide enthalten, vor deren Einfluss die Kulturpflanze geschützt werden soll. Ebenfalls Gegenstand der Erfindung ist mit Wirkstoffen der Formel I vorbehandeltes Vermehrungsgut von Kulturpflanzen wie Saatgut, Setzlinge oder Stecklinge. Insbesondere geeignet sind die Wirkstoffe der Formel I zur Behandlung von Saatgut von Getreide, Soja und vorzugsweise Sorghum, Mais und Reis.

Als Kulturpflanzen gelten im Rahmen vorliegender Erfindung beispielsweise sämtliche Getreidearten, wie Weizen, Roggen, Gerste und Hafer, daneben vor allem Reis, Kulturhirse, Mais und Soja. Vorzugsweise werden von den Wirkstoffen der Formel I Hirse (Sorghum), Mais und Reis gegen die Wirkung von Acylcyclohexandionherbiziden, Sulfonylharnstoffherbiziden, Chloracetanilidherbiziden oder Aryloxyphenoxypropionsäureherbiziden geschützt.

Hervorragende Schutzwirkung gegen Acylcyclohexandionherbizide, Sulfonylharnstoffherbizide, gegen Aryloxyphenoxypropionsäureherbizide und gegen Chloracetanilidherbizide werden bei Anwendung der Antidotes der Formel I in Mais, Sorghum und Reis beobachtet. Insbesondere ist dabei der günstige Effekt der Verbindung 1-[4-(N-3,4-Dimethylbenzoyl-sulfamoyl)-phenyl]-3-methylharnstoff herauszuheben.

Sulfonylharnstoffherbizide, deren schädigende Wirkung gegenüber Kulturpflanzen mit Hilfe der N-Acylsulfamoylphenylharnstoffe der Formel I aufgehoben werden kann, sind in letzter Zeit in grosser Zahl bekannt geworden. Aus der Vielzahl der Publikationen, welche sich der Offenbarung von herbizid wirksamen Sulfonylharnstoffderivaten widmen, sollen beispielhaft das US-Patent 4 127 405 sowie die publizierten Europäischen Patentanmeldungen EP-A-7687, EP-A-30142, EP-A-44807, EP-A-44808, EP-A-51466, EP-A-70802, EP-A-84020, EP-A-87780, EP-A-102925, EP-A-108708, EP-A-120814, EP-A-136061, EP-A-184385, EP-A-206995 und EP-A-237292 genannt sein.

Typische Vertreter von herbiziden Sulfonylharnstoffderivaten sind unter der Formel II

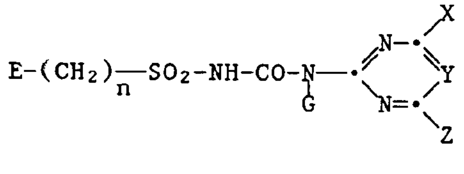

(II)

zusammengefasst, worin

E eine Gruppe

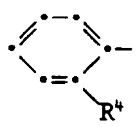 , 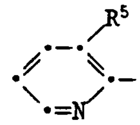 , 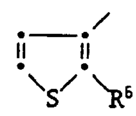 ,

oder

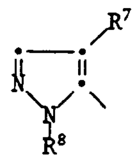 ,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten.

Unter die Formel II fallen folgende herbizide Einzelwirkstoffe:
N-(3-Trifluormethylpyridin-2-ylsulfonyl)-N′-(4,6-dimethoxypyrimidin-2-yl)-harnstoff,
N-(3-Dimethylcarbamoylpyridin-2-ylsulfonyl)-N′-(4,6-dimethoxypyrimidin-2-yl)-harnstoff,
N-(1-Methyl-4-äthoxycarbonylpyrazol-2-ylsulfonyl)-N′-(4,6-dimethoxypyrimidin-2-yl)-harnstoff,
N-(2-Methoxycarbonylthien-3-ylsulfonyl)-N′-(4-methoxy-6-methyl-1,3,5-triazin-2-yl)-harnstoff,
N-(2-Methoxycarbonylbenzylsulfonyl)-N′-(4,6-dimethoxypyrimidin-2-yl)-harnstoff,
N-(2-Methoxycarbonylphenylsulfonyl)-N′-(4,6-bis-difluormethoxypyrimidin-2-yl)-harnstoff,
N-(2-Methoxycarbonylphenylsulfonyl)-N′-(4-äthoxy-6-methylamino-1,3,5-triazin-2-yl)-harnstoff,
N-(2-Methoxycarbonylphenylsulfonyl)-N′-(4-methoxy-6-methyl-1,3,5-triazin-2-yl)-harnstoff,
N-(2-Aethoxycarbonylphenylsulfonyl)-N′-(4-chlor-6-methoxypyrimidin-2-yl)-harnstoff,
N-(2-Methoxycarbonylphenylsulfonyl)-N′-(4-methoxy-6-methyl-1,3,5-triazin-2-yl)- N′-methylharnstoff,
N-(2-Methoxycarbonylphenylsulfonyl)-N′-(4,6-dimethoxypyrimidin-2-yl)-harnstoff,
N-(2-Chlorphenylsulfonyl)-N′-(4-methoxy-6-methyl-1,3,5-triazin-2-yl)-harnstoff,
N-[2-(2-Chloräthoxy)-phenylsulfonyl]-N′-(4-methoxy-6-methyl-1,3,5-triazin-2-yl)-harnstoff, und
N-[2-(2-Methoxyäthoxy)-phenylsulfonyl]-N′-(4,6-dimethoxy-1,3,5-triazin-2-yl)-harnstoff.

Halogenacetanilide, deren schädigende Wirkung gegenüber Kulturpflanzen mit Hilfe der N-Acylsulfamoylphenylharnstoffe der Formel I aufgehoben werden kann, sind ebenfalls bereits in grosser Zahl bekannt geworden. Solche Halogenacetanilide können durch die folgende allgemeine Formel III beschrieben werden:

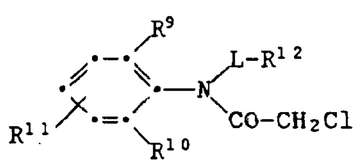 (III)

worin
L eine $C_1$-$C_4$-Alkylenbrücke,
$R^9$ , $R^{10}$ und $R^{11}$ unabhängig voneinander Wasserstoff, Halogen, $C_1$-$C_4$-Alkyl,
$C_1$-$C_4$-Alkoxy, $C_1$-$C_4$-Halogenalkyl, $C_2$-$C_5$-Alkoxyalkyl oder

$C_2$-$C_5$-Alkylthioalkyl, und
$R^{12}$ $C_1$-$C_4$-Alkoxy, -COOH, $C_1$-$C_4$-Alkoxycarbonyl, -$CONH_2$,
$C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet.

Unter die Formel III fallen insbesondere die folgenden herbiziden Chloracetanilidderivate:
N-Aethoxymethyl-N-chloracetyl-2-äthyl-6-methylanilin,
N-Chloracetyl-N-methoxymethyl-2,6-diäthylanilin,
N-Chloracetyl-N-(2-methoxyäthyl)-2,6-dimethylanilin,
N-Chloracetyl-N-(2-n-propoxyäthyl)-2,6-dimethylanilin,
N-Chloracetyl-N-(2-isopropoxyäthyl)-2,6-dimethylanilin,
N-Chloracetyl-N-(2-methoxyäthyl)-2-äthyl-6-methylanilin,
N-Chloracetyl-N-(methoxyäthyl)-2,6-diäthylanilin,
N-(2-Aethoxyäthyl)-N-chloracetyl-2-äthyl-6-methylanilin,
N-Chloracetyl-N-(2-methoxy-1-methyläthyl)-2-methylanilin,
N-Chloracetyl-N-(2-methoxy-1-methyläthyl)-2,6-dimethylanilin,
N-Chloracetyl-N-(2-methoxy-1-methyläthyl)-2,6-diäthylanilin,
N-Chloracetyl-N-(2-methoxy-1-methyläthyl)-2-äthyl-6-methylanilin,
N-(2-Aethoxyäthyl)-N-chloracetyl-2,6-diäthylanilin,
N-Chloracetyl-N-(2-n-propoxyäthyl)-2-äthyl-6-methylanilin,
N-Chloracetyl-N-(2-n-propoxyäthyl)-2,6-diäthylanilin,
N-Chloracetyl-N-(2-isopropoxyäthyl)-2-äthyl-6-methylanilin,
N-Aethoxycarbonylmethyl-N-chloracetyl-2,6-dimethylanilin
N-Aethoxycarbonylmethyl-N-chloracetyl-2,6-diäthylanilin
N-Chloracetyl-N-methoxycarbonylmethyl-2,6-dimethylanilin,
N-Chloracetyl-N-(2,2-diäthoxyäthyl)-2,6-dimethylanilin,
N-Chloracetyl-N-(2-methoxy-1-methyläthyl)-2,3-dimethylanilin,
N-(2-Aethoxyäthyl)-N-chloracetyl-2-methylanilin,
N-Chloracetyl-N-(2-methoxyäthyl)-2-methylanilin,
N-Chloracetyl-N-(2-methoxy-2-methyläthyl)-2,6-dimethylanilin,
N-(2-Aethoxy-2-methyläthyl)-N-chloracetyl-2-äthyl-6-methylanilin,
N-Chloracetyl-N-(1-äthyl-1-methoxyäthyl)-2,6-dimethylanilin,
N-Chloracetyl-N-(2-methoxyäthyl)-2-methoxy-6-methylanilin,
N-n-Butoxymethyl-N-chloracetyl-2-tert.-butylanilin,
N-(2-Aethoxyäthyl-2-methyläthyl)-2,6-dimethylanilin,
N-Chloracetyl-N-(-2-methoxyäthyl)-2-chlor-6-methylanilin,
N-(2-Aethoxyäthyl)-N-chloracetyl-2-chlor-6-methylanilin,
N-(2-Aethoxyäthyl)-N-chloracetyl-2,3,6-trimethylanilin,
N-Chloracetyl-1-(2-methoxyäthyl)-2,3,6-trimethylanilin,
N-Chloracetyl-N-cyanomethyl-2,6-dimethylanilin,
N-Chloracetyl-N-(1,3-dioxolan-2-ylmethyl-2,6-dimethylanilin,
N-Chloracetyl-N-(1,3-dioxolan-2-ylmethyl)-2-äthyl-6-methylanilin,
N-Chloracetyl-N-(1,3-dioxan-2-ylmethyl)-2-äthyl-6-methylanilin,
N-Chloracetyl-N-(2-furylmethyl)-2,6-dimethylanilin,
N-Chloracetyl-N-(2-furylmethyl)-2-äthyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diäthylanilin,

N-(2-n-Butoxyäthyl)-N-chloracetyl-2,6-diäthylanilin,
N-Chloracetyl-N-(2-methoxy-1,2-dimethyläthyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-äthyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diäthylanilin,
N-Benzoylmethyl-N-chloracetyl-2,6-diäthylanilin,
N-Benzoylmethyl-N-chloracetyl-2-äthyl-6-methylanilin,
N-Chloracetyl-N-(5-methyl-1,3,4-oxadiazol-2-yl)-2,6-diäthylanilin,
N-Chloracetyl-N-(5-methyl-1,3,4-oxadiazol-2-yl)-2-äthyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diäthylanilin.

Aryloxyphenoxypropionsäureherbizide, deren schädigende Wirkung gegenüber Kulturpflanzen mit Hilfe der N-Acylsulfamoylphenylharnstoffe der Formel I aufgehoben werden kann, sind in grosser Zahl bekannt. Solche Aryloxyphenoxypropionsäurederivate können durch die folgende allgemeine Formel IV beschrieben werden:

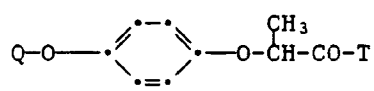

(IV)

worin
Q für den Rest

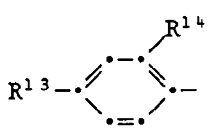 , 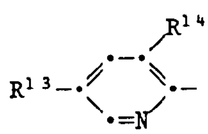 , 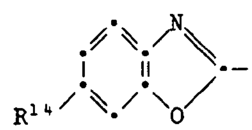 ,

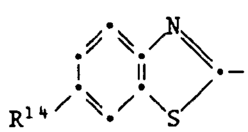 oder 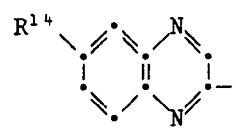

und T für
$-NR^{15}R^{16}$, $-N(CN)R^{17}$, $-OR^{18}$, $SR^{18}$ oder $-O-N=CR^{19}R^{20}$ stehen, wobei

$R^{13}$ Halogen oder Trifluormethyl,

$R^{14}$ Wasserstoff oder Halogen,

$R^{15}$ und $R^{16}$ unabhängig voneinander Wasserstoff, $C_1$-$C_8$-Alkoxy, $C_1$-$C_8$-Alkyl, Phenyl oder Benzyl, $R^{15}$ und $R^{16}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R^{17}$ $C_1$-$C_4$-Alkyl, $C_3$-$C_4$-Alkenyl, $C_3$-$C_4$-Alkinyl oder $C_2$-$C_4$-Alkoxyalkyl

$R^{18}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$, -CON($C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{21}$, $COSR^{21}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3- bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten.

Unter die Formel IV fallen insbesondere die folgenden herbiziden Aryloxyphenoxypropionsäurederivate:
2-[4-(5-Chlor-3-fluorpyridin-2-yloxy)-phenoxy]-propionsäure-propargylester
2-[4-(3,5-Dichlorpyridin-2-yloxy)-phenoxy]-propionsäure-propargylester,
2-[4-(3,5-Dichlorpyridin-2-yloxy)-phenoxy]-thiopropionsäure-propargylester,
2-[4-(5-Chlor-3-fluorpyridin-2-yloxy)-phenoxy]-propionsäure-methylester,
2-[4-(5-Chlor-3-fluorpyridin-2-yloxy)-phenoxy]-propionsäure-butylester,
2-[4-(5-Trifluormethylpyridin-2-yloxy)-phenoxy]-propionsäure-butylester,
2-[4-(5-Trifluormethylpyridin-2-yloxy)-phenoxy]-propionsäure-methylester,
2-[4-(6-Chlorchinoxalin-2-yloxy)-phenoxy]-propionsäure-äthylester und
2-[4-(6-Chlorbenzoxazolin-2-yloxy)-phenoxy]-propionsäure-äthylester.

Acylcyclohexandionderivate, deren schädigende Wirkung gegenüber Kulturpflanzen mit Hilfe der N-Acylsulfamoylphenylharnstoffe der Formel I aufgehoben werden kann, sind beispielsweise in der publizierten europäischen Patentanmeldung Nr. 0 243 313 beschrieben. Solche Acylcyclohexandionderivate können durch die folgende allgemeine Formel IX

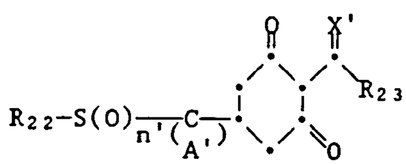
(IX)

worin A′ eine 2-7-gliedrige Alkylenbrücke, eine 3-7-gliedrige Alkenylenbrücke, welche ein- oder mehrfach ungesättigt sein kann,

n′ Null, eins oder zwei

$R_{22}$ $C_1$-$C_4$-Alkyl oder Benzyl

$R_{23}$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl wobei der Phenylring durch Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,

X′ Sauerstoff oder ein Rest $-NOR_{24}$ und

$R_{24}$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl bedeuten.

Unter die Formel IX fallen insbesondere die folgenden herbiziden Acylcyclohexandionderivate:
5-(1-Methylthiocyclobutan-1-yl)-2-(2,4-dichlorobenzoyl)-cyclohexan-1,3-dion,
5-(1-Methylthiocyclobutan-1-yl)-2-n-butyryl-cyclohexan-1,3-dion,
5-(1-Methylthiocyclobutan-1-yl)-cyclopropylcarbonyl-cyclohexan-1,3-dion,
5-(1-Methylthiocyclobutan-1-yl)-2-(2,3-dichlorobenzoyl)-cyclohexan-1,3-dion,
5-(1-Methylsulfonylcyclobutan-1-yl)-2-n-butyryl-cyclohexan-1,3-dion,
5-(1-Methylthiocyclopropan-1-yl)-2-propionyl-cyclohexan-1,3-dion,
5-(1-Aethylthiocyclopropan-1-yl)-2-propionyl-cyclohexan-1,3-dion und
5-(1-Methylthiocyclopropan-1-yl)-2-n-butyryl-cyclohexan-1,3-dion.
5-(1-Methylthiocyclobutan-1-yl)-2-(1-äthoximino-n-butyryl)-cyclohexan-1,3-dion,
5-(1-Methylthiocyclopropan-1-yl)-2-(1-äthoximino-n-butyryl)-cyclohexan-1,3-dion,
5-(1-Methylthiocyclohexan-1-yl)-2-(1-äthoximino-n-butyryl)-cyclohexan-1,3-dion,
5-(1-Methylthiocyclobutan-1-yl)-2-(1-allyloxyimino-n-butyryl)-cyclohexan-1,3-dion und
5-(1-Methylthiocyclopentan-1-yl)-2-(1-äthoximino-n-butyryl)-cyclohexan-1,3-dion,
5-(1-Methylthiocyclopropan-1-yl)-2-[1-(trans-3-chlorallyloximino)-n-butyryl]-cyclohexan-1,3-dion,
5-(1-Methylthiocyclopropan-1-yl)-2-[1-(trans-3-chlorallyloximino)-propionyl]-cyclohexan-1,3-dion,
5-(1-Methylthiocyclopropan-1-yl)-2-[1-(cis-3-chlorallyloximino)-propionyl]-cyclohexan-1,3-dion und
5-(1-Aethylthiocyclopropan-1-yl)-2-[1-(trans-3-chlorallyloximino)-propionyl]-cyclohexan-1,3-dion.

Die Antidotes der Formel I eignen sich ganz besonders, um Kulturpflanzen vor den herbiziden Wirkungen der Herbizide der Formeln II, III oder IV zu schützen.

Agrochemische Mittel, welche in einer gemeinsamen Formulierung neben dem Antidote der Formel I ein Sulfonylharnstoffherbizid, ein Chloracetanilidherbizid oder ein Aryloxyphenoxypropionsäureherbizid enthalten sind zum Einsatz als selektive Herbizide in Nutzpflanzenkulturen geeignet. Vorzugsweise enthalten die erfindungsgemässen herbiziden Mittel neben einem Antidote der Formel I einen Sulfonylharnstoff der Formel II, ein Chloracetanilid der Formel III, ein Acylcyclohexandionderivat der Formel IX oder ein Aryloxyphenoxypropionsäurederivat der Formel IV.

Die angewendete Menge an Gegenmittel, sofern sie nicht auf die Samen gebeizt wird, schwankt zwischen etwa 0,01 und etwa 5 Gewichtsteilen pro Gewichtsteil Herbizid. In der Praxis ermittelt man jeweils, von Fall zu Fall, das heisst je nach verwendetem Herbizid-Typ, welches Verhältnis in Bezug auf optimale Wirkung bei der speziellen Kulturpflanze das geeignetste ist.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Für die Verwendung von Verbindungen der Formel I oder sie enthaltender Mittel zum Schützen von Kulturpflanzen gegen schädigende Wirkungen von Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (bei einer 25 %igen Formulierung: 40 g bis 2 kg Spritzpulver) pro 100 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit 50-3200 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 400 g Aktivsubstanz pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:30) wird verwendet, wobei die Aufwandmenge an Herbizid 0,001 bis 10 kg pro Hektar beträgt. Solche Tankmischung wird vorzugsweise vor oder nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Di-oktylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside in guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali, Erdalkali- oder gegebenenfalls substituierten Ammoniumsalze von höheren Fettsäuren ($C_{10}$-$C_{22}$), wie zum Beispiel die Na-oder K-Salze der Oel- oder Stearinsäure, oder von natürlichen Fettsäuregemischen, die beispielsweise aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, zum Beispiel das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 Kohlenstoffatomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenolpolyäthoxyäthanol, Polyäthylenglykol und Oktylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 Kohlenstoffatomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:
"1985 International Mc Cutcheon's Emulsifiers & Detergents", Glen Rock NJ USA, 1985",
"H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatstoffes und 0 bis 25 %, insbesondere 0,1 bis 25 % eines Tensides.

Insbesondere setzen sich bevorzugte Formulierungen folgendermassen zusammen: (% = Gewichtsprozent)

<u>Emulgierbare Konzentrate</u>

| | | |
|---|---|---|
| Aktives Wirkstoffgemisch | 1 bis 20 %, bevorzugt | 5 bis 10 % |
| oberflächenaktives Mittel: | 5 bis 30 %, vorzugsweise | 10 bis 20 % |
| flüssiges Trägermittel: | 50 bis 94 %, vorzugsweise | 70 bis 85 % |

<u>Stäube:</u>

| | | |
|---|---|---|
| Aktives Wirkstoffgemisch: | 0,1 bis 10 %, vorzugsweise | 0,1 bis 1 % |
| festes Trägermittel: | 99,9 bis 90 %, vorzugsweise | 99,9 bis 99 %. |

<u>Suspension-Konzentrate</u>

| | | |
|---|---|---|
| Aktives Wirkstoffgemisch: | 5 bis 75 %, vorzugsweise | 10 bis 50 % |
| Wasser: | 94 bis 25 %, vorzugsweise | 88 bis 30 % |
| oberflächenaktives Wasser: | 1 bis 40 %, vorzugsweise | 2 bis 30 % |

<u>Benetzbare Pulver</u>

| | | |
|---|---|---|
| Aktives Wirkstoffgemisch: | 0,5 bis 90 %, vorzugsweise | 1 bis 80 % |
| oberflächenaktives Mittel: | 0,5 bis 20 %, vorzugsweise | 1 bis 15 % |
| festes Trägermaterial: | 5 bis 95 %, vorzugsweise | 15 bis 90 % |

<u>Granulate</u>

| | | |
|---|---|---|
| aktives Wirkstoffgemisch: | 0,5 bis 30 %, vorzugsweise | 3 bis 15 % |
| festes Trägermittel: | 99,5 bis 70 %, vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,25 bis 5 kg AS/ha.

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele:

Beispiel H1: 1-[4-(N-3,4-Dimethylbenzoyl-sulfamoyl)-phenyl]-3-methylharnstoff.

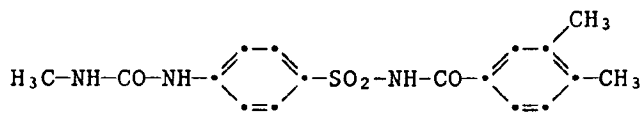

a) N-(4-Sulfamoylphenyl)-O-phenylcarbamat.

176 g Sulfanilamid werden in 750 ml Dioxan suspendiert und mit 79 g Pyridin versetzt. Zu dieser Mischung lässt man bei 15°C 160 g Chlorameisensäurephenylester zutropfen. Die Reaktionssuspension wird für 1 Stunde bei 50°C gerührt, abgekühlt und in 2 Liter Wasser eingerührt. Das ausgefallene Produkt wird abgetrennt und getrocknet. Man erhält 277 g N-(4-Sulfamoylphenyl)-O-phenylcarbamat, Smp. 220-223°C.

b) 1-(4-Sulfamoylphenyl)-3-methylharnstoff.

Zu einer Suspension von 350 g N-(4-Sulfamoylphenyl)-O-phenylcarbamat in 1500 ml Aethanol lässt man bei 80°C 330 ml einer 33 %iger äthanolischen Methylaminlösung zutropfen. Aus der anfänglich klaren Lösung kristallisiert das Produkt innerhalb von 1,5 Stunden aus. Nach Abkühlen der Reaktionsmischung trennt man den Niederschlag ab und trocknet ihn. Man erhält so 256 g 1-(4-Sulfamoylphenyl)-3-methylharnstoff, Smp. 211-213°C.

c) Einer Suspension von 73,3 g 1-(4-Sulfamoylphenyl)-3-methylharnstoff in 600 ml Acetonitril setzt man 56,7 g 3,4-Dimethylbenzoylchlorid und 3,9 g 4-Dimethylaminopyridin zu. Zu dieser Mischung lässt man 98,1 g Triäthylamin zutropfen. Die Temperatur der Mischung steigt dabei von der Raumtemperatur auf 45°C an und die dispersen Bestandteile gehen in Lösung. Nach kurzer Zeit setzt Kristallisation ein. Nach weiteren 2,5 Stunden rührt man das Gemisch in 2,5 Liter Eiswasser und 200 ml 2N Schwefelsäure ein. Das ausgefallene Produkt wird abgetrennt, mit Aether gewaschen und getrocknet. Man erhält 114,4 g 1-[4-(N-3,4-Dimethylbenzoyl-sulfamoyl)-phenyl]-3-methylharnstoff, Smp. 236-238°C (unter Zersetzung).

Beispiel H2: 1-[3-(N-2-Furylcarbonyl-sulfamoyl)-phenyl]-3,3-dimethylharnstoff.

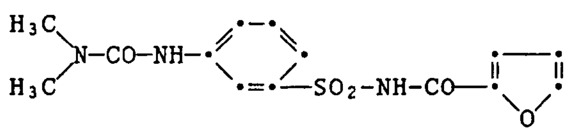

a) N-(3-Sulfamoylphenyl)-O-phenylcarbamat.

Zu einer Suspension von 125 g 3-Aminobenzolsulfonamid und 57,9 g Pyridin in 500 ml Dioxan lässt man bei 15°C 113 g Chlorameisensäurephenylester zutropfen. Die Mischung wird für eine Stunde bei Raumtemperatur und für eine Stunde bei 50°C gerührt und anschliessend in 2 Liter Wasser eingerührt. Das ausgefallene Produkt wird abgetrennt und getrocknet. Man erhält so 201 g N-(3-sulfamoylphenyl)-O-phenylcarbamat, Smp. 184-186°C (unter Zersetzung).

b) 1-(3-Sulfamoylphenyl)-3,3-dimethylharnstoff.

Ein Gemisch aus 205 g N-(3-Sulfamoylphenyl)-O-phenylcarbamat und 158 g einer 40 %-igen wässrigen Lösung von Dimethylamin wird für 3 Stunden in 500 ml Aethanol zum Rückfluss erhitzt. Das Lösungsmittel wird unter vermindertem Druck abdestilliert. Der Rückstand wird mit Wasser versetzt und das entstandene Phenol wird mit Wasserdampf abgetrieben. Der kristalline Rückstand wird nach dem Abkühlen des Destillationskolbens abgetrennt, mit kaltem Methanol gewaschen und getrocknet. Man erhält so 136 g 1-(3-Sulfamoylphenyl)-3,3-dimethylharnstoff, Smp. 193-194°C.

c) Der Suspension von 10,1 g 1-(3-Sulfamoylphenyl)-3,3-dimethylharnstoff in 60 ml Acetonitril setzt man 6,1 g 2-Furancarbonsäurechlorid und 0,1 g 4-Dimethylaminopyridin zu. Zu dieser Mischung lässt man bei Raumtemperatur 9,5 g Triäthylamin zutropfen. Das Reaktionsgemisch wird für 15 Stunden bei Raumtemperatur und für eine Stunde bei 60°C gerührt. Nach Abkühlen der Mischung lässt man 4,5 g Methansulfonsäure zutropfen und nimmt sie in 1 Liter Wasser auf. Das abgeschiedene Oel kristallisiert nach kurzer Zeit. Das Kristallisat wird abgetrennt, mit Methanol gewaschen und getrocknet. Man erhält so 11,3 g 1-[3-(N-2-Furylcarbonylsulfamoyl)-phenyl]-3,3-dimethylharnstoff, Smp. 216-217°C (unter Zersetzung).

Beispiel H3: 1-[4-(N-3,4-Dimethylbenzoyl-sulfamoyl)-phenyl]-3-allylharnstoff.

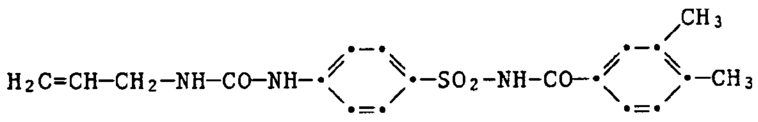

Eine Suspension von 12,2 g 4-(N-3,4-Dimethylbenzoyl-sulfamoyl)-anilin in 20 ml Acetonitril wird tropfenweise mit 4,1 g Triäthylamin versetzt. Zunächst entsteht eine klare Lösung aus der ein Salz ausfällt. Nach Zugabe von 4,6 g Allylisocyanat wird die Mischung für 2 Stunden bei Raumtemperatur und für 2 Stunden bei 70°C gerührt. Nachdem das Gemisch abgekühlt ist, wird es in einem Gemisch von 1 Liter Eiswasser und 4,5 g konzentrierter Schwefelsäure aufgenommen. Der entstandene Niederschlag wird abgetrennt, mit Aether gewaschen und getrocknet. Man erhält so 15,1 g 1-[4-(N-3,4-Dimethylbenzoyl-sulfamoyl)-phenyl]-3-allylharnstoff, Smp. 194-201°C (unter Zersetzung).

Beispiel H4: 1-[4-(N-2,3-Dimethylbenzoyl-sulfamoyl)-phenyl]-3-methylharnstoff.

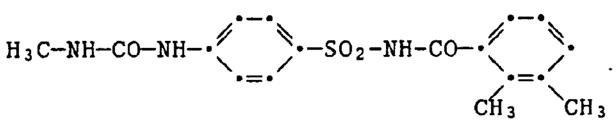

Eine Suspension von 6,1 g 4-(N-2,3-Dimethylbenzoyl-sulfamoyl)-anilin in 50 ml Acetonitril wird mit 2,8 g Triäthylamin versetzt. Zu diesem Gemisch lässt man 1,3 ml Methylisocyanat zutropfen. Die Reaktionsmischung wird für 2 Stunden bei Raumtemperatur und für 6 Stunden bei 50°C gerührt. Nach Abkühlen auf Raumtemperatur wird das Gemisch in 200 ml Eiswasser und 15 ml 2N Schwefelsäure aufgenommen. Das ausgefallene Produkt wird abgetrennt und getrocknet. Man erhält 1-[4-(N-2,3-Dimethylbenzoylsulfamoyl)-phenyl]-3-methylharnstoff, Smp. 242-244°C.

In analoger Weise erhält man die in den folgenden Tabellen aufgelisteten Zwischen- und Endprodukte.

Tabelle 1:

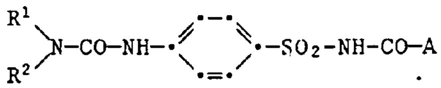

| Verb. Nr. | $R^1R^2N$- | A | Smp. [°C] |
|---|---|---|---|
| 1.001 | Amino | Phenyl | |
| 1.002 | Methylamino | Phenyl | 237-238° (Zers.) |
| 1.003 | Dimethylamino | Phenyl | |
| 1.004 | Methylamino | 2-Tolyl | 234-236° (Zers.) |
| 1.005 | Methylamino | 3-Tolyl | 216-218° (Zers.) |
| 1.006 | Methylamino | 4-Tolyl | 247-249° (Zers.) |
| 1.007 | Methylamino | 4-Aethyl-phenyl | |
| 1.008 | Methylamino | 4-n-Propyl-phenyl | |
| 1.009 | Methylamino | 4-i-Propyl-phenyl | |
| 1.010 | Methylamino | 4-n-Butyl-phenyl | |
| 1.011 | Methylamino | 4-tert-Butyl-phenyl | 250-252° (Zers.) |
| 1.012 | Methylamino | 2-Chlor-phenyl | 239-241° (Zers.) |
| 1.013 | Methylamino | 3-Chlor-phenyl | 222-224° (Zers.) |
| 1.014 | Methylamino | 4-Chlor-phenyl | 246-247° (Zers.) |
| 1.015 | Methylamino | 2-Fluor-phenyl | 227-229° (Zers.) |
| 1.016 | Methylamino | 3-Fluor-phenyl | 234-235° (Zers.) |
| 1.017 | Methylamino | 4-Fluor-phenyl | |
| 1.018 | Methylamino | 2-Brom-phenyl | 244-245° (Zers.) |
| 1.019 | Methylamino | 3-Brom-phenyl | |
| 1.020 | Methylamino | 4-Brom-phenyl | |
| 1.021 | Methylamino | 2-Jod-phenyl | |
| 1.022 | Methylamino | 3-Jod-phenyl | |

Tabelle 1: Fortsetzung

| Verb. Nr. | $R^1R^2N$- | A | Smp. [°C] |
|---|---|---|---|
| 1.023 | Methylamino | 4-Jod-phenyl | |
| 1.024 | Methylamino | 3-Chlormethyl-phenyl | |
| 1.025 | Methylamino | 2-Trifluormethyl-phenyl | |
| 1.026 | Methylamino | 3-Trifluormethyl-phenyl | 231-233° (Zers.) |
| 1.027 | Methylamino | 4-Trifluormethyl-phenyl | |
| 1.028 | Methylamino | 2-Methoxy-phenyl | 197-198° (Zers.) |
| 1.029 | Methylamino | 3-Methoxy-phenyl | |
| 1.030 | Methylamino | 4-Methoxy-phenyl | |
| 1.031 | Methylamino | 2-Aethoxy-phenyl | |
| 1.032 | Methylamino | 3-Aethoxy-phenyl | |
| 1.033 | Methylamino | 4-Aethoxy-phenyl | |
| 1.034 | Methylamino | 2-Methylmercapto-phenyl | |
| 1.035 | Methylamino | 2-Methylsulfonyl-phenyl | |
| 1.036 | Methylamino | 2-Methoxycarbonyl-phenyl | |
| 1.037 | Methylamino | 4-Methoxycarbonyl-phenyl | |
| 1.038 | Methylamino | 2-Nitro-phenyl | |
| 1.039 | Methylamino | 3-Nitro-phenyl | |
| 1.040 | Methylamino | 4-Nitro-phenyl | 237-238° (Zers.) |
| 1.041 | Methylamino | 2-Acetyl-phenyl | |
| 1.042 | Methylamino | 4-Acetyl-phenyl | |
| 1.043 | Methylamino | 3-Cyan-phenyl | |
| 1.044 | Methylamino | 4-Cyan-phenyl | |
| 1.045 | Methylamino | 2,3-Dimethyl-phenyl | 242-244° (Zers.) |
| 1.046 | Methylamino | 2,4-Dimethyl-phenyl | 239-241° (Zers.) |
| 1.047 | Methylamino | 2,5-Dimethyl-phenyl | 223-224° (Zers.) |
| 1.048 | Methylamino | 2,6-Dimethyl-phenyl | |
| 1.049 | Amino | 3,4-Dimethyl-phenyl | 151-153° (Zers.) |

Tabelle 1: Fortsetzung

| Verb. Nr. | $R^1R^2N-$ | A | Smp. [°C] |
|---|---|---|---|
| 1.050 | Methylamino | 3,4-Dimethyl-phenyl | 236-238° (Zers.) |
| 1.051 | Dimethylamino | 3,4-Dimethyl-phenyl | 233-235° (Zers.) |
| 1.052 | Aethylamino | 3,4-Dimethyl-phenyl | > 230° (Zers.) |
| 1.053 | Propylamino | 3,4-Dimethyl-phenyl | 211-213° (Zers.) |
| 1.054 | Isopropylamino | 3,4-Dimethyl-phenyl | 235-236° (Zers.) |
| 1.055 | Cyclopropylamino | 3,4-Dimethyl-phenyl | 229-231° (Zers.) |
| 1.056 | Butylamino | 3,4-Dimethyl-phenyl | 200-202° (Zers.) |
| 1.057 | Isobutylamino | 3,4-Dimethyl-phenyl | 222-223° (Zers.) |
| 1.058 | sec. Butylamino | 3,4-Dimethyl-phenyl | 215-218° (Zers.) |
| 1.059 | tert. Butylamino | 3,4-Dimethyl-phenyl | 223-225° (Zers.) |
| 1.060 | Allylamino | 3,4-Dimethyl-phenyl | 194-201° (Zers.) |
| 1.061 | Propargylamino | 3,4-Dimethyl-phenyl | 231-233° (Zers.) |
| 1.062 | Cycloheylamino | 3,4-Dimethyl-phenyl | |
| 1.063 | Phenylamino | 3,4-Dimethyl-phenyl | 230-231° (Zers.) |
| 1.064 | 2-Methoxy-äthylamino | 3,4-Dimethyl-phenyl | 166-169° (Zers.) |
| 1.065 | Benzylamino | 3,4-Dimethyl-phenyl | 234-236° (Zers.) |
| 1.066 | Pyrrolidinyl | 3,4-Dimethyl-phenyl | |
| 1.067 | Piperidinyl | 3,4-Dimethyl-phenyl | |
| 1.068 | Hexahydroazepinyl | 3,4-Dimethyl-phenyl | |
| 1.069 | Morpholinyl | 3,4-Dimethyl-phenyl | |
| 1.070 | 2-Methyl-piperidinyl | 3,4-Dimethyl-phenyl | |
| 1.071 | 3-Methyl-piperidinyl | 3,4-Dimethyl-phenyl | |

Tabelle 1: Fortsetzung

| Verb. Nr. | $R^1R^2N$- | A | Smp. [°C |
|---|---|---|---|
| 1.072 | 4-Methyl-piperidinyl | 3,4-Dimethyl-phenyl | |
| 1.073 | 2,6-Dimethyl-piperidinyl | 3,4-Dimethyl-phenyl | |
| 1.074 | N-Methyl-piperazinyl | 3,4-Dimethyl-phenyl | |
| 1.075 | Thiomorpholinyl | 3,4-Dimethyl-phenyl | |
| 1.076 | Methylamino | 3,5-Dimethyl-phenyl | 240-242° (Zers.) |
| 1.077 | Methylamino | 2,3-Dichlor-phenyl | |
| 1.078 | Methylamino | 2,4-Dichlor-phenyl | |
| 1.079 | Methylamino | 2,5-Dichlor-phenyl | |
| 1.080 | Methylamino | 2,6-Dichlor-phenyl | |
| 1.081 | Methylamino | 3,4-Dichlor-phenyl | 240-241° (Zers.) |
| 1.082 | Methylamino | 3,5-Dichlor-phenyl | |
| 1.083 | Methylamino | 2,3-Difluor-phenyl | |
| 1.084 | Methylamino | 2,4-Difluor-phenyl | |
| 1.085 | Methylamino | 2,5-Difluor-phenyl | |
| 1.086 | Methylamino | 2,6-Difluor-phenyl | |
| 1.087 | Methylamino | 3,4-Difluor-phenyl | |
| 1.088 | Methylamino | 3,5-Difluor-phenyl | |
| 1.089 | Methylamino | 3,5-Dimethoxy-phenyl | |
| 1.090 | Methylamino | 2,4-Dimethoxy-phenyl | |
| 1.091 | Methylamino | 2,5-Dimethoxy-phenyl | |
| 1.092 | Methylamino | 2,6-Dimethoxy-phenyl | |
| 1.093 | Methylamino | 3,4-Dimethoxy-phenyl | 223-225° (Zers.) |
| 1.094 | Dimethylamino | 3,4-Dimethoxy-phenyl | 203-207° (Zers.) |
| 1.095 | Methylamino | 3,5-Dimethoxy-phenyl | |
| 1.096 | Methylamino | 3,4-Dinitro-phenyl | |
| 1.097 | Methylamino | 3,5-Dinitro-phenyl | |
| 1.098 | Methylamino | 2-Chlor-3-nitro-phenyl | |
| 1.099 | Methylamino | 2-Chlor-4-nitro-phenyl | |
| 1.100 | Methylamino | 2-Chlor-5-nitro-phenyl | |

Tabelle 1: Fortsetzung

| Verb. Nr. | $R^1R^2N$- | A | Smp. [°C] |
|---|---|---|---|
| 1.101 | Methylamino | 3-Chlor-2-nitro-phenyl | |
| 1.102 | Methylamino | 4-Chlor-2-nitro-phenyl | |
| 1.103 | Methylamino | 3-Chlor-3-nitro-phenyl | |
| 1.104 | Methylamino | 5-Chlor-2-nitro-phenyl | |
| 1.105 | Methylamino | 2-Methyl-3-nitro-phenyl | 247-248° (Zers.) |
| 1.106 | Methylamino | 2-Methyl-5-nitro-phenyl | |
| 1.107 | Methylamino | 2-Methyl-6-nitro-phenyl | |
| 1.108 | Methylamino | 3-Methyl-2-nitro-phenyl | |
| 1.109 | Methylamino | 3-Methyl-4-nitro-phenyl | |
| 1.110 | Methylamino | 4-Methyl-3-nitro-phenyl | |
| 1.111 | Methylamino | 5-Methyl-2-nitro-phenyl | |
| 1.112 | Methylamino | 4-Fluor-3-nitro-phenyl | |
| 1.113 | Methylamino | 5-Chlor-2-methoxy-phenyl | |
| 1.114 | Methylamino | 4-Chlor-2-methoxy-phenyl | |
| 1.115 | Methylamino | 4-Chlor-6-fluor-phenyl | |
| 1.116 | Methylamino | 4-Chlor-2-fluor-phenyl | |
| 1.117 | Methylamino | 2,4,6-Trimethyl-phenyl | |
| 1.118 | Methylamino | 2,4,5-Trimethoxy-phenyl | |
| 1.119 | Methylamino | 3,4,5-Trimethoxy-phenyl | 238-239° (Zers.) |
| 1.120 | Methylamino | 1-Naphthyl | 240-242° (Zers.) |
| 1.121 | Methylamino | 2-Naphthyl | 234-235° (Zers.) |
| 1.122 | Methylamino | 2-Furyl | 245-246° (Zers.) |
| 1.123 | Dimethylamino | 2-Furyl | 246-247° (Zers.) |
| 1.124 | Methylamino | 3-Furyl | |

Tabelle 1: Fortsetzung

| Verb. Nr. | $R^1R^2N$- | A | Smp. [°C] |
|---|---|---|---|
| 1.125 | Methylamino | 2-Thienyl | 245-247° (Zers.) |
| 1.126 | Methylamino | 2-Chlor-pyridinyl | |
| 1.127 | Methylamino | 2,6-Dichlor-pyridinyl | 138-139° (Zers.) |
| 1.128 | Methylamino | 2,6-Dibrom-pyridinyl | |
| 1.129 | Methylamino | Piperonyl | 268-270° (Zers.) |
| 1.130 | Dimethylamino | 3-Tolyl | 204-207° (Zers.) |
| 1.131 | Cyclopropylamino | 3-Trifluormethyl-phenyl | 246-247° (Zers.) |
| 1.132 | i-Propylamino | 3-Tolyl | 217-220° (Zers.) |
| 1.133 | Dimethylamino | 3-Trifluormethylphenyl | 229-230° (Zers.) |
| 1.134 | Amino | 4-Chlor | 226-228° (Zers.) |
| 1.135 | Methylamino | 2-Carboxyphenyl | 181-183° (Zers.) |
| 1.136 | i-Propylamino | 2-Tolyl | 236-238° (Zers.) |
| 1.137 | Methylamino | 3-Brom-4-Methylphenyl | 244-245° (Zers.) |
| 1.138 | i-Propylamino | 1-Naphtyl | 235-237° (Zers.) |
| 1.139 | Allylamino | 1-Naphtyl | 218-219° (Zers.) |
| 1.140 | Allylamino | 2-Tolyl | 195-198° (Zers.) |
| 1.141 | 3-Tolylamino | 2-Tolyl | 197-199° (Zers.) |

Tabelle 1: Fortsetzung

| Verb. Nr. | $R^1R^2N$- | A | Smp. [°C] |
|---|---|---|---|
| 1.142 | tert.-Butylamino | 2-Tolyl | 144-145° (Zers.) |
| 1.143 | tert.-Butylamino | 2,4-Dimethylphenyl | 226-227° (Zers.) |
| 1.144 | Propargylamino | 1-Naphtyl | 149-151° (Zers.) |
| 1.145 | sec. Butylamino | 2,4-Dimethylphenyl | 230-232° (Zers.) |
| 1.146 | Propargylamino | 2,4-Dimethylphenyl | 191-192° (Zers.) |
| 1.147 | Methylamino | 2-Biphenyl | 262-264° (Zers.) |
| 1.148 | sec. Butylamino | 2,5-Dimethylphenyl | 208-210° (Zers.) |
| 1.149 | Dimethylamino | 2,4-Dimethylphenyl | 206-209° (Zers.) |
| 1.150 | Dimethylamino | 1-Naphtyl | >250° (Zers.) |
| 1.151 | Dimethylamino | 2-Tolyl | 236-240° (Zers.) |
| 1.152 | Propargylamino | 2,5-Dimethyl-phenyl | 198-200° (Zers.) |
| 1.153 | Methylamino | 4-Chlormethyl-phenyl | 228-229° (Zers.) |
| 1.154 | Methylamino | 3-(2,3-Tetramethylen)-thienyl | >250° |
| 1.155 | Methylamino | 3-(2,3-Tetramethylen)-furyl | |
| 1.156 | Methylamino | 3-(4,5-Dimethyl)-thienyl | 238-239° (Zers.) |

Tabelle 2:

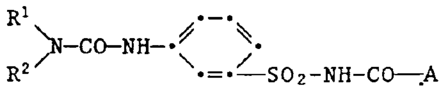

| Verb. Nr. | $R^1R^2N$- | A | Smp. [°C] |
|---|---|---|---|
| 2.001 | Amino | Phenyl | |
| 2.002 | Methylamino | Phenyl | |
| 2.003 | Dimethylamino | Phenyl | |
| 2.004 | Methylamino | 2-Tolyl | |
| 2.005 | Methylamino | 3-Tolyl | |
| 2.006 | Methylamino | 4-Tolyl | |
| 2.007 | Methylamino | 2-Chlor-phenyl | |
| 2.008 | Methylamino | 4-Chlor-phenyl | |
| 2.009 | Methylamino | 3-Trifluormethyl-phenyl | |
| 2.010 | Methylamino | 2,3-Dimethyl-phenyl | |
| 2.011 | Methylamino | 3,4-Dimethyl-phenyl | 147-152° |
| 2.012 | Methylamino | 1-Naphthyl | |
| 2.013 | Methylamino | 2-Naphthyl | |
| 2.014 | Dimethylamino | 2-Furyl | 216-217° (Zers.) |

Tabelle 3:

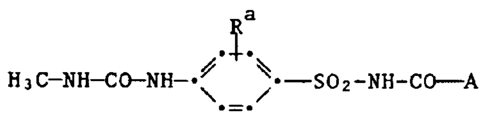

| Verb. Nr. | A | $R^a$ | Smp. [°C] |
|---|---|---|---|
| 3.001 | Phenyl | 2-Fluor | |
| 3.002 | Phenyl | 3-Fluor | |
| 3.003 | 4-Tolyl | 2-Fluor | 258-260° (Zers.) |
| 3.004 | 4-Tolyl | 3-Fluor | ca. 270° (Zers.) |
| 3.005 | 4-Tolyl | 2-Chlor | |
| 3.006 | 4-Tolyl | 3-Chlor | |
| 3.007 | 4-Tolyl | 2-Methyl | |
| 3.008 | 4-Tolyl | 3-Methyl | |
| 3.009 | 3,4-Dimethyl-phenyl | 2-Fluor | 246-248° (Zers.) |
| 3.010 | 3,4-Dimethyl-phenyl | 3-Fluor | 239-240° |
| 3.011 | 3,4-Dimethyl-phenyl | 2-Chlor | |
| 3.012 | 3,4-Dimethyl-phenyl | 3-Chlor | 223-224° (Zers.) |
| 3.013 | 3,4-Dimethyl-phenyl | 2-Methyl | |
| 3.014 | 3,4-Dimethyl-phenyl | 3-Methyl | 218-220° (Zers.) |
| 3.015 | 2-Tolyl | 3-Fluor | 236-238° (Zers.) |
| 3.016 | 1-Naphtyl | 3-Fluor | 131-133° (Zers.) |
| 3.017 | 2,4-Dimethyl-phenyl | 2-Fluor | 236-238° (Zers.) |
| 3.018 | 2,4-Dimethyl-phenyl | 3-Fluor | 233-234° (Zers.) |
| 3.019 | 1-Naphtyl | 2-Fluor | 240-241° (Zers.) |
| 3.020 | 2,3-Dimethyl-phenyl | 3-Fluor | 227-230° (Zers.) |
| 3.021 | 2,3-Dimethyl-phenyl | 2-Fluor | 244-246° (Zers.) |

Tabelle 3: Fortsetzung

| Verb. Nr. | A | $R^a$ | Smp. [°C] |
|---|---|---|---|
| 3.022 | 2-Tolyl | 2-Fluor | 236-237° (Zers.) |
| 3.023 | 3,5-Dimethyl-phenyl | 3-Fluor | 198-200° (Zers.) |
| 3.024 | 3,5-Dimethyl-phenyl | 2-Fluor | 244-246° (Zers.) |
| 3.025 | 2,5-Dimethyl-phenyl | 3-Fluor | 213-215° (Zers.) |
| 3.026 | 2,5-Dimethyl-phenyl | 2-Fluor | 227-229° (Zers.) |
| 3.027 | 2-Tolyl | 3-Methyl | 176° (Zers.) |
| 3.028 | 2-Tolyl | 3-Chlor | 218-220° (Zers.) |
| 3.029 | 1-Naphtyl | 3-Chlor | 150° (Zers.) |
| 3.030 | 1-Naphtyl | 3-Methyl | 153-155° (Zers.) |

Tabelle 4:

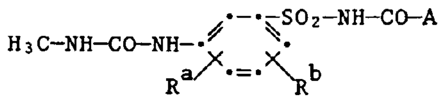

| Verb. Nr. | A | $R^a$ | $R^b$ | Smp. [°C] |
|---|---|---|---|---|
| 4.001 | 1-Naphtyl | 2-Methyl | 6-Methyl | 214-216° (Zers.) |
| 4.002 | 3,4-Dimethyl-phenyl | 2-Methyl | 6-Methyl | 206-208° (Zers.) |
| 4.003 | 2-Tolyl | 2-Methyl | 6-Methyl | 217-219° (Zers.) |
| 4.004 | 1-Naphtyl | 2-Fluor | 6-Fluor | |
| 4.005 | 3,4-Dimethyl-phenyl | 2-Fluor | 6-Fluor | |
| 4.006 | 2-Tolyl | 2-Chlor | 6-Chlor | |
| 4.007 | 4-Tolyl | 2-Fluor | 5-Fluor | |
| 4.008 | 4-Tolyl | 2-Fluor | 5-Fluor | |
| 4.009 | 4-Tolyl | 2-Methyl | 5-Methyl | |

Tabelle 5:

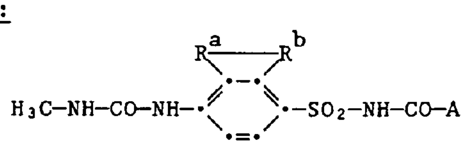

| Verb. Nr. | A | 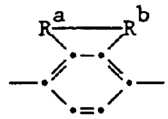 | Smp. [°C] |
|---|---|---|---|
| 5.001 | 3,4-Dimethyl-phenyl | 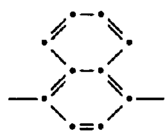 | 201-203° (Zers.) |
| 5.002 | 2-Tolyl | 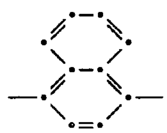 | 184-186° (Zers.) als Triäthyl-aminsalz |
| 5.003 | 1-Naphtyl | 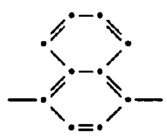 | 138-140° (Zers.) als Triäthyl-aminsalz |
| 5.004 | 2-Tolyl | 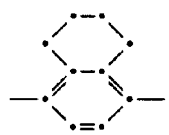 | |
| 5.005 | 4-Tolyl | 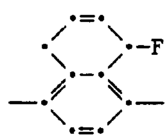 | |
| 5.006 | 3,4-Dimethyl-phenyl | 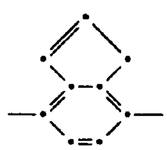 | |
| 5.007 | 2-Tolyl | 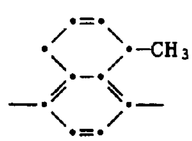 | |

Formulierungsbeispiele für Wirkstoffe der Formel I oder Mischungen dieser Wirkstoffe mit Herbiziden

Beispiel F1:

| Spritzpulver | a) | b) | c) |
|---|---|---|---|
| Wirkstoff Nr. 1.050 | 20 % | 60 % | 0,5% |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Oktylphenolpolyäthylenglykol-äther 7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Das Wirkstoffgemisch wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Beispiel F2: Emulsions-Konzentrat | a) | b) |
|---|---|---|
| Wirkstoff Nr. 2.011 | 10 % | 1 % |
| Oktylphenolpolyäthylenglykol-äther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesen Konzentraten können durch Verdünnen mit Wasser Emulsionen jeder gewünschten Konzentration hergestellt werden.

| Beispiel F3: Stäubemittel | a) | b) |
|---|---|---|
| Wirkstoff Nr. 1.050 | 0,1 % | 1 % |
| Talkum | 99,9 % | - |
| Kaolin | - | 99 % |

Man erhält anwendungsfertige Stäubemittel, indem das Wirkstoffgemisch mit dem Träger vermischt und auf einer geeigneten Mühle vermahlen wird.

| Beispiel F4: Extruder Granulat | a) | b) |
|---|---|---|
| Wirkstoff Nr. 1.006 | 10 % | 1 % |
| Na-Ligninsulfonat | 2 % | 2 % |
| Carboxymethylcellulose | 1 % | 1 % |
| Kaolin | 87 % | 96 % |

Das Wirkstoffgemisch wird mit den Zusatzstoffen vermischt, vermahlen und mit Wasser angefeuchtet. Dieses Gemisch wird extrudiert und anschliessend im Luftstrom getrocknet.

Beispiel F5: Umhüllungs-Granulat

| | |
|---|---|
| Wirkstoff Nr. 1.005 | 3 % |
| Polyäthylenglykol (MG 200) | 3 % |
| Kaolin | 94 % |

Das fein gemahlene Wirkstoffgemisch wird in einem Mischer auf das mit Polyäthylenglykol angefeuchtete Kaolin gleichmässig aufgetragen. Auf diese Weise erhält man staubfreie Umhüllungs-Granulate.

| Beispiel F6: Suspensions-Konzentrat | a) | b) |
|---|---|---|
| Wirkstoff Nr. 1.050 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

| Beispiel F7: Salzlösung | |
|---|---|
| Wirkstoff Nr. 1.026 | 5 % |
| Isopropylamin | 1 % |
| Oktylphenolpolyäthylenglykoläther (78 Mol AeO) | 3 % |
| Wasser | 91 %. |

Beispiel F8:

| Spritzpulver | a) | b) | c) |
|---|---|---|---|
| Wirkstoff Nr. 1.050 in Mischung mit N-[2-(2-Methoxyäthoxy)-phenylsulfonyl]-N'-(4,6-dimethoxy-1,3,5-triazin-2-yl)-harnstoff | 20 % | 60 % | 0,5% |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Oktylphenolpolyäthylenglykol-äther 7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 |

Das Wirkstoffgemisch wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Beispiel F9: Emulsions-Konzentrat | a) | b) |
|---|---|---|
| Wirkstoff Nr. 2.011 in Mischung mit 2-[4-(5-Trifluormethylpyridin-2-yloxy)-phenoxy]-propionsäure-n-butylester | 10 % | 1 % |
| Oktylphenolpolyäthylenglykol-äther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesen Konzentraten können durch Verdünnen mit Wasser Emulsionen jeder gewünschten Konzentration hergestellt werden.

| Beispiel F10: Stäubemittel | a) | b) |
|---|---|---|
| Wirkstoff Nr. 1.050 in Mischung mit N-[2-(2-Methoxyäthoxy)-phenylsulfonyl]-N'-(4,6-dimethoxy-1,3,5-triazin-2-yl)-harnstoff | 0,1 % | 1 % |
| Talkum | 99,9 % | - |
| Kaolin | - | 99 % |

Man erhält anwendungsfertige Stäubemittel, indem das Wirkstoffgemisch mit dem Träger vermischt und auf einer geeigneten Mühle vermahlen wird.

| Beispiel F11: Extruder Granulat | a) | b) |
|---|---|---|
| Wirkstoff Nr. 1.006 in Mischung mit N-[2-(2-Methoxyäthoxy)-phenylsulfonyl]-N'-(4,6-dimethoxy-1,3,5-triazin-2-yl)-harnstoff | 10 % | 1 % |
| Na-Ligninsulfonat | 2 % | 2 % |
| Carboxymethylcellulose | 1 % | 1 % |
| Kaolin | 87 % | 96 % |

Das Wirkstoffgemisch wird mit den Zusatzstoffen vermischt, vermahlen und mit Wasser angefeuchtet. Dieses Gemisch wird extrudiert und anschliessend im Luftstrom getrocknet.

| Beispiel F12: Umhüllungs-Granulat | |
|---|---|
| Wirkstoff Nr. 1.005 in Mischung mit N-Chloracetyl-N-(2-methoxy-1-methyläthyl)-2-äthyl-6-methyl-anilin | 3 % |
| Polyäthylenglykol (MG 200) | 3 % |
| Kaolin | 94 % |

Das fein gemahlene Wirkstoffgemisch wird in einem Mischer auf das mit Polyäthylenglykol angefeuchtete Kaolin gleichmässig aufgetragen. Auf diese Weise erhält man staubfreie Umhüllungs-Granulate.

| Beispiel F13: Suspensions-Konzentrat | a) | b) |
|---|---|---|
| Wirkstoff Nr. 1.050 in Mischung mit 2-[4-(6-Chlorbenzoxazol-2-yloxy)-phenoxy]-propionsäure-äthylester | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

| Beispiel F14: Salzlösung | |
|---|---|
| Wirkstoff Nr. 1.026 in Mischung mit 2-[4-(3,5-Dichlorpyridin-2-yloxy)-phenoxy]-propionsäure-propargylester | 5 % |
| Isopropylamin | 1 % |
| Oktylphenolpolyäthylenglykoläther (78 Mol AeO) | 3 % |
| Wasser | 91 %. |

Biologische Beispiele

Die Fähigkeit der Verbindungen der Formel I, Kulturpflanzen vor der phytotoxischen Wirkung starker Herbizide zu schützen, kann aus den folgenden Beispielen ersehen werden. In der Versuchsbeschreibung werden die Verbindungen der Formel I als Antidote (Gegenmittel oder Safener) bezeichnet.

Die Auswertung der Tests erfolgt in der Weise, dass einerseits die Wirkung des Herbizids allein in Prozent vorgenommen wird, wobei 100 % Wirkung der Abtötung der Pflanzen entspricht und 0 % Wirkung keine Wirkung wie bei den unbehandelten Kontrollpflanzen bedeutet. Andererseits wird die Wirkung der Herbizid-Antidote Kombination auf die gleiche Weise ebenfalls in Prozent Herbizidwirkung bestimmt. Die Differenz der beiden erhaltenen Prozentzahlen wird als "Schutzwirkung in %" in den Testresultaten wiedergegeben.

Die Wirkung der Herbizid-Antidote Kombination kann sowohl bei direkter gleichzeitiger Applikation der beiden Wirkstoffe in einem einzigen Spritzvorgang in Tankmischung, als auch bei getrennter und auch bei zeitlich verschobener Applikation, wie beispielsweise bei Saatbeizung mit Antidote und postemergenter Herbizidbehandlung bewertet werden.

Beispiel B1: Versuch mit Herbizid und Antidote in Kulturhirse (Sorghum). Herbizid und Antidote werden zusammen als Tankmischung im Vorauflaufverfahren oder Nachauflaufverfahren appliziert.

In Töpfen von 11 cm Durchmesser, gefüllt mit Gartenerde, werden Samen von Sorghum der Sorte "Funk G-623" ausgesät und im Gewächshaus unter angepassten Temperatur- und Lichtverhältnissen angezogen. Bewässerung und Düngung erfolgt nach Bedarf. Zur Ermittlung der preemergenten Wirkung wird eine Mischung von Antidote und Herbizid in einer Wassermenge von 550 l/ha direkt nach der Aussaat appliziert. Für die Ermittlung der postemergenten Wirkung das Wirkstoffgemisch nach dem Auflaufen der Pflanzen im 3- bis 5-Blattstadium appliziert. Jeweils 21 Tage nach der Behandlung erfolgt die Versuchsauswertung der "Schutzwirkung in Prozent".

Testresultate:

a) preemergenter Test:

| | |
|---|---|
| Testpflanze: | Sorghum "Funk G-623" |
| Behandlung: | preemergent mit Tankmischung von 250 g/ha Verbindung Nr. 1.050 und 125 g/ha N-[2-(2-Methoxyäthoxy)-phenylsulfonyl]-N′-(4,6-dimethoxy-1,3,5-triazin-2-yl)-harnstoff. |
| Wirkung: | 21 Tage nach Applikation:<br>50 Prozent Schutzwirkung. |

b) postemergenter Test:

| | |
|---|---|
| Testpflanze: | Sorghum "Funk G-623" |
| Behandlung: | postemergent mit Tankmischung von 250 g/ha Verbindung Nr. 1.050 und 125 g/ha N-[2-(2-Methoxyäthoxy)-phenylsulfonyl]-N′-(4,6-dimethoxy-1,3,5-triazin-2-yl)-harnstoff. |
| Wirkung: | 21 Tage nach Applikation:<br>45 Prozent Schutzwirkung. |

Beispiel B2: Versuch mit Herbizid und Antidote in Mais und Sorghum. Das Antidote wird auf das Saatgut gebeizt, das Herbizid wird im Vorauflaufverfahren oder Nachauflaufverfahren appliziert.

In Töpfen von 11 cm Durchmesser, gefüllt mit Gartenerde, werden die mit dem Antidote gebeizte Samen von Mais der Sorte "Blizzard" und Sorghum der Sorte "Funk G-623" ausgesät und im Gewächshaus unter angepassten Temperatur- und Lichtverhältnissen angezogen. Bewässerung und Düngung erfolgt nach Bedarf. Zur Ermittlung der preemergenten Wirkung wird das Herbizid in einer Wassermenge von 550 l/ha direkt nach der Aussaat appliziert. Für die Ermittlung der postemergenten Wirkung wird das Herbizid nach dem Auflaufen der Pflanzen im 3- bis 5-Blattstadium appliziert. Jeweils 12 bis 26 Tage nach der Behandlung erfolgt die Versuchsauswertung der "Schutzwirkung in Prozent".

Testresultate:

a) Mais-Tests:

| | |
|---|---|
| Testpflanze: | Mais "Blizzard" |
| Behandlung: | Saatbeizung mit 0,5 g, 1 oder 2 g der Verbindung Nr. 1.050 pro kg Mais-Saatgut. |

Herbizid-preemergent:

4000 g/ha N-Chloracetyl-N-(2-methoxy-1-methoxyäthyl)-äthyl-6-methylanilin, oder
240 g/ha N-[2-(3,3,3-Trifluorpropen-1-yl)-phenylsulfonyl]-N′-(4-methoxy-6-methyl-1,3,5-triazin-2-yl)-harnstoff, oder
120, 60 oder 30 g/ha N-[2-(2-Methoxyäthoxy)-phenylsulfonyl]-N′-(4,6-dimethoxy-1,3,5-triazin-2-yl)-harnstoff.

Herbizid-postemergent:

120 g/ha N-(2-Methoxycarbonylthien-3-ylsulfonyl)-N′-(4-methoxy-6-methyl-1,3,5-triazin-2-yl)-harnstoff, oder
480 g/ha N-[2-(3,3,3-Trifluorpropen-1-yl)-phenylsulfonyl]-N′-(4-methoxy-6-methyl-1,3,5-triazin-2-yl)-harnstoff, oder
120 g/ha N-(2-Methoxycarbonylphenylsulfonyl)-N′-(4-difluormethoxy-6-methoxypyrimidin-2-yl)-harnstoff, oder
120 g/ha N-(2-Methoxycarbonylphenylsulfonyl)-N′-(4-cyclopropyl-6-methoxy-1,3,5-triazin-2- yl)-harnstoff, oder
120 g/ha N-(2-Methoxycarbonylphenylsulfonyl)-N′-(4,6-bis-difluormethoxy-pyrimidin-2-yl)-harnstoff, oder
10 g/ha N-[2-(2-Chloräthoxy)-phenylsulfonyl]-N′-(4-methoxy-6-methyl-1,3,5-triazin-2-yl)-harnstoff, oder
30 g/ha 2-[4-(6-Chlorchinoxalin-2-yloxy)-phenoxy]-propionsäureäthylester, oder
60 g/ha 2-[4-(6-Chlorbenzoxazol-2-yloxy)-phenoxy]-propionsäureäthylester, oder
30 g/ha 2-[4-(3,5-Dichlorpyridin-2-yloxy)-phenoxy]-propionsäure-propargylester, oder
10 g/ha N-(2-Methoxycarbonylphenylsulfonyl)-N′-(4-äthoxy-6-methylamino-1,3,5-triazin-2- yl)-harnstoff, oder
240, 120, 60 oder 30 g/ha N-[2-(2-Methoxyäthoxy)-phenylsulfonyl]-N′-(4,6-dimethoxy,1,3,5-triazin-2-yl)-harnstoff.

Wirkung: (DAT: Tage nach Applikation)

| Safener Beizmenge in g/kg Samen | Herbizid -Dosis in g/ha | | Applikationsart des Herbizids/ Auswertung | Schutzwirkung in % |
|---|---|---|---|---|
| 2 | 4000 | N-Chloracetyl-N-(2-methoxy-1-äthyl)-2-äthyl-6-methylanilin | pre/14 DAT | 40 |
| 1 | 240 | N-[2-(3,3,3-Trifluorpropen-1-yl)-phenylsulfonyl]-N'-(4-methoxy-6-methyl-1,3,5-triazin-2-yl)-harnstoff | pre/20 DAT | 25 |
| 1 | 480 | | post/20 DAT | 30 |
| 2 | 120 | N-[2-(2-Methoxyäthoxy)-phenylsulfonyl]-N'-(4,6-dimethoxy-1,3,5-triazin-2-yl)-harnstoff | pre/12 DAT | 70 |
| 1 | 120 | | pre/12 DAT | 65 |
| 0,5 | 120 | | pre/12 DAT | 65 |
| 2 | 60 | | pre/12 DAT | 65 |
| 1 | 60 | | pre/12 DAT | 65 |
| 0,5 | 60 | | pre/12 DAT | 70 |
| 2 | 30 | | pre/12 DAT | 55 |
| 1 | 30 | | pre/12 DAT | 50 |
| 0,5 | 30 | | pre/12 DAT | 55 |
| 2 | 240 | | post/20 DAT | 65 |
| 1 | 240 | | post/20 DAT | 65 |
| 0,5 | 240 | | post/20 DAT | 50 |
| 2 | 120 | | post/20 DAT | 60 |
| 1 | 120 | | post/20 DAT | 55 |
| 0,5 | 120 | | post/20 DAT | 25 |
| 2 | 60 | | post/20 DAT | 40 |
| 1 | 60 | | post/20 DAT | 40 |
| 0,5 | 60 | | post/20 DAT | 35 |
| 2 | 30 | | post/20 DAT | 25 |
| 1 | 30 | | post/20 DAT | 30 |
| 0,5 | 30 | | post/20 DAT | 15 |

Fortsetzung

| Safener Beizmenge in g/kg Samen | Herbizid -Dosis in g/ha | | Applikationsart des Herbizids/ Auswertung | Schutzwirkung in % |
|---|---|---|---|---|
| 1 | 120 | N-(2-Methoxycarbonylthien-3-ylsulfonyl)-N'-(4-methoxy-6-methyl-1,3,5-triazin-2-yl)-harnstoff | post/16 DAT | 20 |
| 1 | 120 | N-(2-Methoxycarbonylphenyl sulfonyl)-N'-(4-difluormethoxy-6-methoxy-pyrimidin-2-yl)-harnstoff | post/16 DAT | 15 |
| 1 | 120 | N-(2-Methoxycarbonylphenyl sulfonyl)-N'-(4-cyclopropyl-6-methoxy-1,3,5-triazin-2-yl)-harnstoff | post/16 DAT | 20 |
| 1 | 120 | N-(2-Methoxycarbonylphenyl sulfonyl)-N'-(4,6-bis-difluormethoxy-pyrimidin-2-yl)-harnstoff | post/16 DAT | 20 |
| 1 | 10 | N-[2-(2-Chloräthoxy)-phenyl sulfonyl]-N'-(4-methoxy-6-methyl-1,3,5-triazin-2-yl)-harnstoff | post/16 DAT | 35 |
| 1 | 30 | 2-[4-(6-Chlorchinoxalin-2-yloxy)-phenoxy]-propionsäure-äthylester | post/16 DAT | 30 |
| 1 | 30 | 2-[4-(3,5-Dichlorpyridin-2-yloxy)-phenoxy]-propionsäure-propargylester | post/16 DAT | 45 |
| 1 | 10 | N-(2-Methoxycarbonylphenyl sulfonyl)-N'-(4-äthoxy-6-methylamino-1,3,5-triazin-2-yl)-harnstoff | post/16 DAT | 30 |
| 1 | 60 | 2-[4-(6-Chlorbenzoxazol-2-yloxy)-phenoxy]-propionsäure-äthylester | post/16 DAT | 30 |

b) Sorghum-Tests:

Testpflanze: Sorghum "Funk G-623"

Behandlung: Saatbeizung mit 0,5 g, 1 oder 2 g der Verbindung Nr. 1.050 pro kg Sorghum-Saatgut.

Herbizid-preemergent:

1000 g/ha N-Chloracetyl-N-(2-methoxy-1-methoxyäthyl)-äthyl-6-methylanilin, oder
250 g/ha N-[2-(3,3,3-Trifluorpropen-1-yl)-phenylsulfonyl]-N'-(4-methoxy-6-methyl-1,3,5-triazin-2-yl)-harnstoff, oder
120, 60 oder 30 g/ha N-[2-(2-Methoxyäthoxy)-phenylsulfonyl]-N'-(4,6-dimethoxy-1,3,5-triazin-2-yl)-harnstoff.

Herbizid-postemergent:

480 g/ha N-[2-(3,3,3-Trifluorpropen-1-yl)-phenylsulfonyl]-N'-(4-methoxy-6-methyl-1,3,5-triazin-2-yl)-harnstoff, oder
15 g/ha 2-[4-(6-Chlorchinoxalin-2-yloxy)-phenoxy]-propionsäureäthylester, oder
60 g/ha 2-[4-(6-chlorbenzoxazol-2-yloxy)-phenoxy]-propionsäure-äthylester, oder
15 g/ha 2-[4-(3,5-Dichlorpyridin-2-yloxy)-phenoxy]-propionsäure-propargylester, oder
30 g/ha 2-[4-(5-Trifluormethylpyridin-2-yloxy)-phenoxy]-propionsäure-butylester, oder
240, 120, 60 oder 30 g/ha N-[2-(2-Methoxyäthoxy)-phenylsulfonyl]-N'-(4,6-dimethoxy-1,3,5-triazin-2-yl)-harnstoff.

Wirkung: (DAT: Tage nach Applikation)

| Safener Beizmenge in g/kg Samen | Herbizid -Dosis in g/ha | | Applikationsart des Herbizids/ Auswertung | Schutzwirkung in % |
|---|---|---|---|---|
| 1 | 1000 | N-Chloracetyl-N-(2-methoxy-1-äthyl)-2-äthyl-6-methylanilin | pre/26 DAT | 35 |
| 1 | 250 | N-[2-(3,3,3-Trifluorpropen-1-yl)-phenylsulfonyl]-N'-(4-methoxy-6-methyl-1,3,5-triazin-2-yl)-harnstoff | pre/20 DAT | 45 |
| 1 | 480 | | post/20 DAT | 40 |
| 2 | 120 | N-[2-(2-Methoxyäthoxy)-phenylsulfonyl]-N'-(4,6-dimethoxy-1,3,5-triazin-2-yl)-harnstoff | pre/12 DAT | 55 |
| 1 | 120 | | pre/12 DAT | 55 |
| 0,5 | 120 | | pre/12 DAT | 60 |
| 2 | 60 | | pre/12 DAT | 55 |
| 1 | 60 | | pre/12 DAT | 50 |
| 0,5 | 60 | | pre/12 DAT | 60 |
| 2 | 30 | | pre/12 DAT | 70 |
| 1 | 30 | | pre/12 DAT | 65 |
| 0,5 | 30 | | pre/12 DAT | 70 |
| 2 | 240 | | post/20 DAT | 50 |
| 1 | 240 | | post/20 DAT | 60 |
| 0,5 | 240 | | post/20 DAT | 60 |
| 2 | 120 | | post/20 DAT | 50 |
| 1 | 120 | | post/20 DAT | 65 |
| 0,5 | 120 | | post/20 DAT | 60 |
| 2 | 60 | | post/20 DAT | 45 |
| 1 | 60 | | post/20 DAT | 65 |
| 0,5 | 60 | | post/20 DAT | 60 |
| 2 | 30 | | post/20 DAT | 30 |
| 1 | 30 | | post/20 DAT | 45 |
| 0,5 | 30 | | post/20 DAT | 50 |

Fortsetzung

| Safener Beizmenge in g/kg Samen | Herbizid -Dosis in g/ha | | Applikationsart des Herbizids/ Auswertung | Schutzwirkung in % |
|---|---|---|---|---|
| 1 | 60 | 2-[4-(6-Chlorbenzoxazol-2-yloxy)-phenoxy]-propionsäure-äthylester | post/16 DAT | 33 |
| 1 | 15 | 2-[4-(6-Chlorchinoxalin-2-yloxy)-phenoxy]-propionsäure-äthylester | post/16 DAT | 40 |
| 1 | 15 | 2-[4-(3,5-Dichlorpyridin-2-yloxy)-phenoxy]-propionsäure-propargylester | post/16 DAT | 50 |
| 1 | 30 | 2-[4-(5-Trifluormethylpyridin-2-yloxy)-phenoxy]-propionsäure-butylester | post/16 DAT | 20 |

Testresultate:

Beispiel B3: Versuch mit Herbizid und Antidote in Reis. Das Saatgut wird in Antidote-Dispersion vorgequollen, das Herbizid im Vorauflaufverfahren appliziert.

Reissamen der Sorte "S-201" werden für 48 Stunden in einer wässrigen Dispersion des Antidotes vorgequollen (seed soaking), dann für 24 Stunden trocken aufbewahrt und schliesslich in Töpfe (9 x 9 cm), gefüllt mit sumpfig-feuchter Gartenerde, oberflächlich ausgesät. Das Herbizid wird in einer Wassermenge von 550 l/ha direkt nach der Aussaat auf die Bodenoberfläche gesprüht. Die Pflanzen werden im Gewächshaus unter angepassten Temperatur- und Lichtverhältnissen angezogen. Bewässerung und Düngung erfolgt nach Bedarf. 26 Tage nach der Behandlung erfolgt die Versuchsauswertung der "Schutzwirkung in Prozent".

Testresultate:

| | |
|---|---|
| Testpflanze: | Reis "S-201" |
| Behandlung: | 48 Stunden Saatgutquellung in einer 300 ppm-Lösung von Verbindung Nr. 1.050, gefolgt von preemergenter Applikation mit 30 g/ha N-[2-(2-Methoxyäthoxy)-phenylsulfonyl]-N'-(4,6-dimethoxy-1,3,5-triazin-2-yl)-harnstoff. |
| Wirkung: | 26 Tage nach Applikation:<br>55 Prozent Schutzwirkung. |

**Patentansprüche**

**Patentansprüche für folgende Vertragsstaaten: AT, BE, CH, DE, FR, GB, GR, IT, LI, LU, NL, SE**

1. N-Acylsulfamoylphenylharnstoffe der Formel I

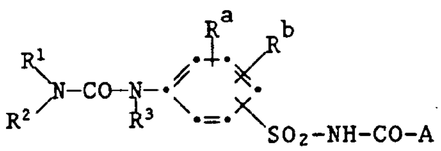

(I)

worin

A für einen Rest aus der Gruppe

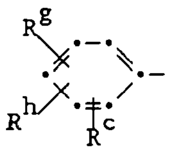 ,

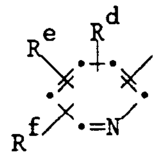 ,

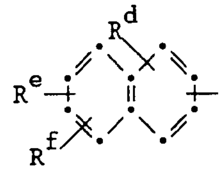 ,

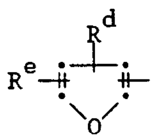

oder

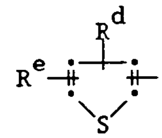 ,

$R^1$ und $R^2$ unabhängig voneinander für Wasserstoff, $C_1$-$C_8$-Alkyl, $C_3$-$C_8$-Cycloalkyl, $C_3$-$C_6$-Alkenyl, $C_3$-$C_6$-Alkinyl,

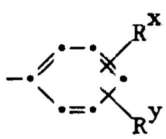

oder durch $C_1$-$C_4$-Alkoxy oder

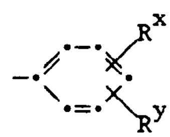

substituiertes $C_1$-$C_4$-Alkyl, oder

$R^1$ und $R^2$ gemeinsam für eine $C_4$-$C_6$-Alkylenbrücke oder eine durch Sauerstoff, Schwefel, SO, $SO_2$, NH oder -N($C_1$-$C_4$-Alkyl)- unterbrochene $C_4$-$C_6$-Alkylenbrücke,

$R^3$ für Wasserstoff oder $C_1$-$C_4$-Alkyl,

$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$-Alkyl-sulfinyl, $C_1$-$C_4$-Alkylsulfonyl, $-COOR^j$, $-CONR^kR^m$, $-COR^n$, $-SO_2NR^kR^m$ oder $-OSO_2$-$C_1$-$C_4$-Alkyl, oder $R^a$ und $R^b$ gemeinsam für eine $C_3$-$C_4$-Alkylenbrücke, die durch Halogen oder $C_1$-$C_4$-Alkyl substituiert sein kann, oder eine $C_3$-$C_4$-Alkenylenbrücke, die durch Halogen oder $C_3$-$C_4$-Alkyl substituiert sein kann, oder eine $C_4$-Alkadienylenbrücke, die durch Halogen oder $C_1$-$C_4$-Alkyl substituiert sein kann, und

$R^g$ und $R^h$ unabhängig voneinander für Wasserstoff, Halogen, $C_1$-$C_4$-Alkyl, Trifluormethyl, Methoxy, Methylthio oder $-COOR^j$ stehen, wobei

$R^c$ Wasserstoff, Halogen, $C_1$-$C_4$-Alkyl oder Methoxy,

$R^d$ Wasserstoff, Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $-COOR^j$ oder $-CONR^k R^m$,

$R^e$ Wasserstoff, Halogen, $C_1$-$C_4$-Alkyl, $-COOR^j$, Trifluormethyl oder Methoxy, oder $R^d$ und $R^e$ gemeinsam für eine $C_3$-$C_4$-Alkylenbrücke,

$R^f$ Wasserstoff, Halogen oder $C_1$-$C_4$-Alkyl,

$R^x$ und $R^Y$ unabhängig voneinander Wasserstoff, Halogen, $C_1$-$C_4$-Alkyl, $C_1$-$C_4$-Alkoxy, $C_1$-$C_4$-Alkylthio, -$COOR^4$, Trifluormethyl, Nitro oder Cyan,
$R^j$, $R^k$ und $R^m$ unabhängig voneinander Wasserstoff oder $C_1$-$C_4$-Alkyl,
$R^k$ und $R^m$ gemeinsam eine $C_4$-$C_6$-Alkylenbrücke oder eine durch Sauerstoff, NH oder -N($C_1$-$C_4$-Alkyl)- unterbrochene $C_4$-$C_6$-Alkylenbrücke, und
$R^n$ $C_1$-$C_4$-Alkyl, Phenyl oder durch Halogen, $C_1$-$C_4$-Alkyl, Methoxy, Nitro oder Trifluormethyl substituiertes Phenyl bedeuten,
sowie die Verbindungen
1-[4-(N-3-Chlormethylbenzoyl-sulfamoyl)-phenyl]-3-methyl-harnstoff,
1-[4-(N-2-Äthoxybenzoyl-sulfamoyl)-phenyl]-3-methyl-harnstoff,
1-[4-(N-3-Äthoxybenzoyl-sulfamoyl)-phenyl]-3-methyl-harnstoff,
1-[4-(N-4-Äthoxybenzoyl-sulfamoyl)-phenyl]-3-methyl-harnstoff,
1-[4-(N-2-Methylsulfonylbenzoyl-sulfamoyl)-phenyl]-3-methyl-harnstoff,
1-[4-(N-2-Nitrobenzoyl-sulfamoyl)-phenyl]-3-methyl-harnstoff,
1-[4-(N-3-Nitrobenzoyl-sulfamoyl)-phenyl]-3-methyl-harnstoff,
1-[4-(N-4-Nitrobenzoyl-sulfamoyl)-phenyl]-3-methyl-harnstoff,
1-[4-(N-2-Acetylbenzoyl-sulfamoyl)-phenyl]-3-methyl-harnstoff,
1-[4-(N-4-Acetylbenzoyl-sulfamoyl)-phenyl]-3-methyl-harnstoff,
1-[4-(N-3-Cyanobenzoyl-sulfamoyl)-phenyl]-3-methyl-harnstoff,
1-[4-(N-4-Cyanobenzoyl-sulfamoyl)-phenyl]-3-methyl-harnstoff,
1-[4-(N-3,4-Dinitrobenzoyl-sulfamoyl)-phenyl]-3-methyl-harnstoff,
1-[4-(N-3,5-Dinitrobenzoyl-sulfamo yl)-phenyl]-3-methyl-harnstoff,
1-[4-(N-2-Chlor-3-nitrobenzoyl-sulfamoyl)-phenyl]-3-methyl-harnstoff,
1-[4-(N-2-Chlor-4-nitrobenzoyl-sulfamoyl)-phenyl]-3-methyl-harnstoff,
1-[4-(N-2-Chlor-5-nitrobenzoyl-sulfamoyl)-phenyl]-3-methyl-harnstoff,
1-[4-(N-3-Chlor-2-nitrobenzoyl-sulfamoyl)-phenyl]-3-methyl-harnstoff,
1-[4-(N-4-Chlor-2-nitrobenzoyl-sulfamoyl)-phenyl]-3-methyl-harnstoff,
1-[4-(N-3-Chlor-3-nitrobenzoyl-sulfamoyl)-phenyl]-3-methyl-harnstoff,
1-[4-(N-5-Chlor-2-nitrobenzoyl-sulfamoyl)-phenyl]-3-methyl-harnstoff,
1-[4-(N-2-Methyl-3-nitrobenzoyl-su lfamoyl)-phenyl]-3-methyl-harnstoff,
1-[4-(N-2-Methyl-5-nitrobenzoyl-sulfamoyl)-phenyl]-3-methyl-harnstoff,
1-[4-(N-2-Methyl-6-nitrobenzoyl-sulfamoyl)-phenyl]-3-methyl-harnstoff,
1-[4-(N-3-Methyl-2-nitrobenzoyl-sulfamoyl)-phenyl]-3-methyl-harnstoff,
1-[4-(N-3-Methyl-4-nitrobenzoyl-sulfamoyl)-phenyl]-3-methyl-harnstoff,
1-[4-(N-4-Methyl-3-nitrobenzoyl-sulfamoyl)-phenyl]-3-methyl-harnstoff,
1-[4-(N-5-Methyl-2-nitrobenzoyl-sulfamoyl)-phenyl]-3-methyl-harnstoff und
1-[4-(N-4-Fluor-3-nitrobenzoyl-sulfamoyl)-phenyl]-3-methyl-harnstoff.

**2.** Verbindungen der Formel I gemäss Anspruch 1, dadurch gekennzeichnet, dass
A für einen Rest aus der Gruppe

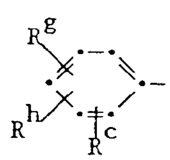 , 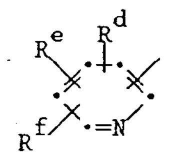 , 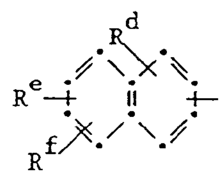 ,

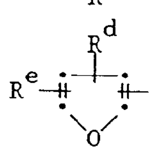 oder 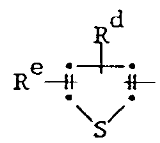 ,

$R^1$ und $R^2$ unabhängig voneinander für Wasserstoff, $C_1$-$C_8$-Alkyl, $C_3$-$C_8$-Cycloalkyl, $C_3$-$C_6$-Alkenyl, $C_3$-$C_6$-Alkinyl,

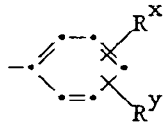

oder durch $C_1$-$C_4$-Alkoxy oder

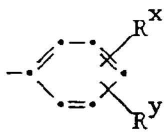

substituiertes $C_1$-$C_4$-Alkyl, oder
$R^1$ und $R^2$ gemeinsam für eine $C_4$-$C_6$-Alkylenbrücke oder eine durch Sauerstoff, Schwefel, SO, $SO_2$, NH oder -N($C_1$-$C_4$-Alkyl)- unterbrochene $C_4$-$C_6$-Alkylenbrücke,
$R^3$ für Wasserstoff oder $C_1$-$C_4$-Alkyl,
$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, -$COOR^j$, -$CONR^kR^m$, -$COR^n$, -$SO_2NR^kR^m$ oder -$OSO_2$-$C_1$-$C_4$-Alkyl, und
$R^g$ und $R^h$ unabhängig voneinander für Wasserstoff, Halogen, $C_1$-$C_4$-Alkyl, Trifluormethyl, Methoxy, Methylthio oder -$COOR^j$ stehen, wobei
$R^c$ Wasserstoff, Halogen, $C_1$-$C_4$-Alkyl oder Methoxy,
$R^d$ Wasserstoff, Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, -$COOR^j$ oder -$CONR^k R^m$,
$R^e$ Wasserstoff, Halogen, $C_1$-$C_4$-Alkyl, -$COOR^j$, Trifluormethyl oder Methoxy, oder $R^d$ und $R^e$ gemeinsam für eine $C_3$-$C_4$-Alkylenbrücke
$R^f$ Wasserstoff, Halogen oder $C_1$-$C_4$-Alkyl,
$R^x$ und $R^y$ unabhängig voneinander Wasserstoff, Halogen, $C_1$-$C_4$-Alkyl, $C_1$-$C_4$-Alkoxy, $C_1$-$C_4$-Alkylthio, -$COOR^4$, Trifluormethyl, Nitro oder Cyan,
$R^j$, $R^k$ und $R^m$ unabhängig voneinander Wasserstoff oder $C_1$-$C_4$-Alkyl,
$R^k$ und $R^m$ gemeinsam eine $C_4$-$C_6$-Alkylenbrücke oder eine durch Sauerstoff, NH oder -N($C_1$-$C_4$-Alkyl)- unterbrochene $C_4$-$C_6$-Alkylenbrücke, und
$R^n$ $C_1$-$C_4$-Alkyl, Phenyl oder durch Halogen, $C_1$-$C_4$-Alkyl, Methoxy, Nitro oder Trifluormethyl substituiertes Phenyl bedeuten.

3. Verbindungen gemäss Anspruch 1, dadurch gekennzeichnet, dass $R^b$ Wasserstoff bedeutet.

4. Verbindungen gemäss Anspruch 1, dadurch gekennzeichnet, dass die Sulfamoylgruppe die 4-Stellung am Phenylring besetzt.

5. Verbindungen gemäss Anspruch 1, dadurch gekennzeichnet, dass $R^2$ und $R^3$ Wasserstoff bedeuten.

6. Verbindungen gemäss Anspruch 1, dadurch gekennzeichnet, dass $R^b$ Wasserstoff bedeutet und die Sulfamoylgruppe die 4-Stellung am Phenylring einnimmt.

7. Verbindungen gemäss Anspruch 1, dadurch gekennzeichnet, dass $R^2$, $R^3$ und $R^b$ Wasserstoff bedeuten und die Sulfamoylgruppe die 4-Stellung am Phenylring einnimmt.

8. Verbindungen gemäss Anspruch 1, dadurch gekennzeichnet, dass A für die Gruppe

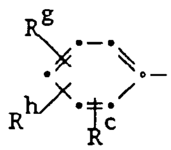

steht.

**9.** Verbindungen gemäss Anspruch 8, dadurch gekennzeichnet, dass $R^2$, $R^3$ und $R^b$ für Wasserstoff stehen und die Sulfamoylgruppe die 4-Stellung am Phenylring besetzt.

**10.** Verbindungen gemäss Anspruch 1, dadurch gekennzeichnet, dass sie der Unterformel Ia

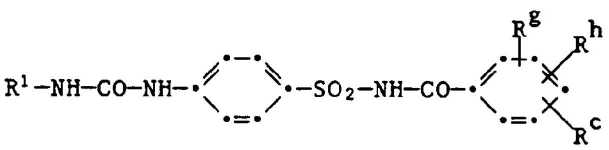

(Ia)

angehören.

**11.** Verbindungen gemäss Anspruch 10, dadurch gekennzeichnet, dass $R^c$ Wasserstoff bedeutet.

**12.** Verbindungen gemäss Anspruch 11, dadurch gekennzeichnet, dass $R^1$, $R^g$ und $R^h$ $C_1$-$C_4$-Alkyl bedeuten.

**13.** 1-[4-(N-4-Methylbenzoyl-sulfamoyl)-phenyl]-3-methylharnstoff gemäss Anspruch 1.

**14.** 1-[4-(N-3-Methylbenzoyl-sulfamoyl)-phenyl]-3-methylharnstoff gemäss Anspruch 1.

**15.** 1-[4-(N-4-tert.Butylbenzoyl-sulfamoyl)-phenyl]-3-methylharnstoff gemäss Anspruch 1.

**16.** 1-[4-(N-3-Trifluormethylbenzoyl-sulfamoyl)-phenyl]-3-methylharnstoff gemäss Anspruch 1.

**17.** 1-[4-(N-4-Nitrobenzoyl-sulfamoyl)-phenyl]-3-methylharnstoff gemäss Anspruch 1.

**18.** 1-[4-(N-2,3-Dimethylbenzoyl-sulfamoyl)-phenyl]-3-methylharnstoff gemäss Anspruch 1.

**19.** 1-[4-(N-3,4-Dimethylbenzoyl-sulfamoyl)-phenyl]-3-methylharnstoff gemäss Anspruch 1.

**20.** 1-[4-(N-3,4-Dimethylbenzoyl-sulfamoyl)-phenyl]-3,3-dimethylharnstoff gemäss Anspruch 1.

**21.** 1-[4-(N-3,4-Dimethylbenzoyl-sulfamoyl)-phenyl]-3-äthylharnstoff gemäss Anspruch 1.

**22.** 1-[4-(N-3,4-Dimethylbenzoyl-sulfamoyl)-phenyl]-3-allylharnstoff gemäss Anspruch 1.

**23.** 1-[4-(N-3,4-Dimethylbenzoyl-sulfamoyl)-phenyl]-3-phenylharnstoff gemäss Anspruch 1.

**24.** 1-[4-(N-3,5-Dimethylbenzoyl-sulfamoyl)-phenyl]-3-methylharnstoff gemäss Anspruch 1.

**25.** 1-[4-(N-3,4-Dichlorbenzoyl-sulfamoyl)-phenyl]-3-methylharnstoff gemäss Anspruch 1.

**26.** 1-[4-(N-3,4-Dimethoxybenzoyl-sulfamoyl)-phenyl]-3-methylharnstoff gemäss Anspruch 1.

**27.** 1-[4-(N-3,4-Dimethoxybenzoyl-sulfamoyl)-phenyl]-3,3-dimethylharnstoff gemäss Anspruch 1.

**28.** 1-[4-(N-2,4,5-Trimethoxybenzoyl-sulfamoyl)-phenyl]-3-methylharnstoff gemäss Anspruch 1.

**29.** 1-[4-(N-1-Naphthylcarbonyl-sulfamoyl)-phenyl]-3-methylharnstoff gemäss Anspruch 1.

**30.** 1-[4-(N-2-Furylcarbonyl-sulfamoyl)-phenyl]-3-methylharnstoff gemäss Anspruch 1.

**31.** 1-[4-(N-2-Furylcarbonyl-sulfamoyl)-phenyl]-3,3-dimethylharnstoff gemäss Anspruch 1.

**32.** 1-[4-(N-2-Thienylcarbonyl-sulfamoyl)-phenyl]-3-methylharnstoff gemäss Anspruch 1.

**33.** 1-[4-(N-Piperonyloyl-sulfamoyl)-phenyl]-3-methylharnstoff gemäss Anspruch 1.

**34.** 1-[4-(N-3-Methylbenzoyl-sulfamoyl)-phenyl]-3,3-dimethylharnstoff gemäss Anspruch 1.

**35.** 1-[4-(N-3-Trifluormethylbenzoyl-sulfamoyl)-phenyl]-3-cyclopropylharnstoff gemäss Anspruch 1.

**36.** 1-[3-(N-3,4-Dimethylbenzoyl-sulfamoyl)-phenyl]-3-methylharnstoff gemäss Anspruch 1.

**37.** 1-[3-(N-2-Furylcarbonyl-sulfamoyl)-phenyl]-3,3-dimethylharnstoff gemäss Anspruch 1.

**38.** Mittel zum Schützen von Kulturpflanzen vor der phytotoxischen Wirkung von Sulfonylharnstoffherbiziden, Chloracetanilidherbiziden, Acylcyclohexandionherbiziden oder Aryloxyphenoxypropionsäureherbiziden, dadurch gekennzeichnet, dass es einen Wirkstoff der Formel I gemäss Anspruch 1 enthält.

**39.** Mittel zum Schützen von Kulturpflanzen vor der phytotoxischen Wirkung von Sulfonylharnstoffherbiziden, Chloracetanilidherbiziden oder Aryloxyphenoxypropionsäureherbiziden, dadurch gekennzeichnet, dass es einen Wirkstoff der Formel I gemäss Anspruch 1 enthält.

**40.** Mittel gemäss Anspruch 39, dadurch gekennzeichnet, dass es sich bei den Kulturpflanzen um Reis, Mais, Sorghum, Getreide oder Soja handelt.

**41.** Mittel gemäss Anspruch 40, dadurch gekennzeichnet, dass es sich bei den Kulturpflanzen um Reis, Mais oder Sorghum handelt.

**42.** Mittel gemäss Anspruch 39, dadurch gekennzeichnet, dass es sich bei den Kulturpflanzen um Sorghum und bei den Herbiziden um Sulfonylharnstoffe handelt.

**43.** Mittel gemäss Anspruch 39, dadurch gekennzeichnet, dass es zum Schützen von Kulturpflanzen vor der phytotoxischen Wirkung von Sulfonylharnstoffherbiziden der Formel II

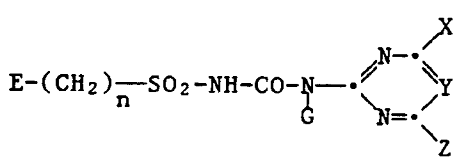

(II)

worin
E eine Gruppe

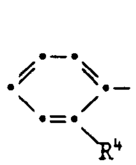

,

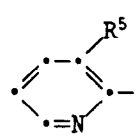

,

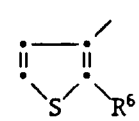

,

oder

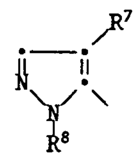,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten, eingesetzt wird.

**44.** Mittel gemäss Anspruch 43, dadurch gekennzeichnet, dass die Sulfonylharnstoffe der Formel II aus der Gruppe
N-(3-Trifluormethylpyridin-2-ylsulfonyl)-N′-(4,6-dimethoxypyrimidin-2-yl)-harnstoff,
N-(3-Dimethylcarbamoylpyridin-2-ylsulfonyl)-N′-(4,6-dimethoxypyrimidin-2-yl)-harnstoff,
N-(1-Methyl-4-äthoxycarbonylpyrazol-2-ylsulfonyl)-N′-(4,6-dimethoxypyrimidin-2-yl)-harnstoff,
N-(2-Methoxycarbonylthien-3-ylsulfonyl)-N′-(4-methoxy-6-methyl-1,3,5-triazin-2-yl)-harnstoff,
N-(2-Methoxycarbonylbenzylsulfonyl)-N′-(4,6-dimethoxypyrimidin-2-yl)-harnstoff,
N-(2-Methoxycarbonylphenylsulfonyl)-N′-(4,6-bis-difluormethoxypyrimidin-2-yl)-harnstoff,
N-(2-Methoxycarbonylphenylsulfonyl)-N′-(4-äthoxy-6-methylamino-1,3,5-triazin-2-yl)-harnstoff,
N-(2-Methoxycarbonylphenylsulfonyl)-N′-(4-methoxy-6-methyl-1,3,5-triazin-2-yl)-harnstoff,
N-(2-Aethoxycarbonylphenylsulfonyl)-N′-(4-chlor-6-methoxypyrimidin-2-yl)-harnstoff,
N-(2-Methoxycarbonylphenylsulfonyl)-N′-(4-methoxy-6-methyl-1,3,5-triazin-2-yl)-N′-methylharnstoff,
N-(2-Methoxycarbonylphenylsulfonyl)-N′-(4,6-dimethoxypyrimidin-2-yl)-harnstoff,
N-(2-Chlorphenylsulfonyl)-N′-(4-methoxy-6-methyl-1,3,5-triazin-2-yl)-harnstoff,
N-[2-(2-Chloräthoxy)-phenylsulfonyl]-N′-(4-methoxy-6-methyl-1,3,5-triazin-2-yl)-harnstoff, und
N-[2-(2-Methoxyäthoxy)-phenylsulfonyl]-N′-(4,6-dimethoxy-1,3,5-triazin-2-yl)-harnstoff ausgewählt werden.

**45.** Mittel gemäss Anspruch 39, dadurch gekennzeichnet, dass es zum Schützen von Kulturpflanzen vor der phytotoxischen Wirkung von Chloracetanilidherbiziden der Formel III

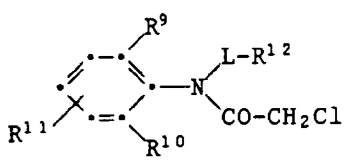

(III)

worin
L eine $C_1$-$C_4$-Alkylenbrücke,
$R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R^{12}$ $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,

gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet.

**46.** Mittel gemäss Anspruch 45, dadurch gekennzeichnet, dass die Chloracetanilide der Formel III aus der Gruppe
N-Aethoxymethyl-N-chloracetyl-2-äthyl-6-methylanilin,
N-Chloracetyl-N-methoxymethyl-2,6-diäthylanilin,
N-Chloracetyl-N-(2-methoxyäthyl)-2,6-dimethylanilin,
N-Chloracetyl-N-(2-n-propoxyäthyl)-2,6-dimethylanilin,
N-Chloracetyl-N-(2-isopropoxyäthyl)-2,6-dimethylanilin,
N-Chloracetyl-N-(2-methoxyäthyl)-2-äthyl-6-methylanilin,
N-Chloracetyl-N-(methoxyäthyl)-2,6-diäthylanilin,
N-(2-Aethoxyäthyl)-N-chloracetyl-2-äthyl-6-methylanilin,
N-Chloracetyl-N-(2-methoxy-1-methyläthyl)-2-methylanilin,
N-Chloracetyl-N-(2-methoxy-1-methyläthyl)-2,6-dimethylanilin,
N-Chloracetyl-N-(2-methoxy-1-methyläthyl)-2,6-diäthylanilin,
N-Chloracetyl-N-(2-methoxy-1-methyläthyl)-2-äthyl-6-methylanilin,
N-(2-Aethoxyäthyl)-N-chloracetyl-2,6-diäthylanilin,
N-Chloracetyl-N-(2-n-propoxyäthyl)-2-äthyl-6-methylanilin,
N-Chloracetyl-N-(2-n-propoxyäthyl)-2,6-diäthylanilin,
N-Chloracetyl-N-(2-isopropoxyäthyl)-2-äthyl-6-methylanilin,
N-Aethoxycarbonylmethyl-N-chloracetyl-2,6-dimethylanilin
N-Aethoxycarbonylmethyl-N-chloracetyl-2,6-diäthylanilin
N-Chloracetyl-N-methoxycarbonylmethyl-2,6-dimethylanilin,
N-Chloracetyl-N-(2,2-diäthoxyäthyl)-2,6-dimethylanilin,
N-Chloracetyl-N-(2-methoxy-1-methyläthyl)-2,3-dimethylanilin,
N-(2-Aethoxyäthyl)-N-chloracetyl-2-methylanilin,
N-Chloracetyl-N-(2-methoxyäthyl)-2-methylanilin,
N-Chloracetyl-N-(2-methoxy-2-methyläthyl)-2,6-dimethylanilin,
N-(2-Aethoxy-2-methyläthyl)-N-chloracetyl-2-äthyl-6-methylanilin,
N-Chloracetyl-N-(1-äthyl-1-methoxyäthyl)-2,6-dimethylanilin,
N-Chloracetyl-N-(2-methoxyäthyl)-2-methoxy-6-methylanilin,
N-n-Butoxymethyl-N-chloracetyl-2-tert.-butylanilin,
N-(2-Aethoxyäthyl-2-methyläthyl)-2,6-dimethylanilin,
N-Chloracetyl-N-(-2-methoxyäthyl)-2-chlor-6-methylanilin,
N-(2-Aethoxyäthyl)-N-chloracetyl-2-chlor-6-methylanilin,
N-(2-Aethoxyäthyl)-N-chloracetyl-2,3,6-trimethylanilin,
N-Chloracetyl-1-(2-methoxyäthyl)-2,3,6-trimethylanilin,
N-Chloracetyl-N-cyanomethyl-2,6-dimethylanilin,
N-Chloracetyl-N-(1,3-dioxolan-2-ylmethyl-2,6-dimethylanilin,
N-Chloracetyl-N-(1,3-dioxolan-2-ylmethyl)-2-äthyl-6-methylanilin,
N-Chloracetyl-N-(1,3-dioxan-2-ylmethyl)-2-äthyl-6-methylanilin,
N-Chloracetyl-N-(2-furylmethyl)-2,6-dimethylanilin,
N-Chloracetyl-N-(2-furylmethyl)-2-äthyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diäthylanilin,
N-(2-n-Butoxyäthyl)-N-chloracetyl-2,6-diäthylanilin,
N-Chloracetyl-N-(2-methoxy-1,2-dimethyläthyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,

N-Chloracetyl-N-(1H-pyrazol-ylmethyl)-2-äthyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diäthylanilin,
N-Benzoylmethyl-N-chloracetyl-2,6-diäthylanilin,
N-Benzoylmethyl-N-chloracetyl-2-äthyl-6-methylanilin,
N-Chloracetyl-N-(5-methyl-1,3,4-oxadiazol-2-yl)-2,6-diäthylanilin,
N-Chloracetyl-N-(5-methyl-1,3,4-oxadiazol-2-yl)-2-äthyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diäthylanilin ausgewählt werden.

**47.** Mittel gemäss Anspruch 39, dadurch gekennzeichnet, dass es zum Schützen von Kulturpflanzen vor der phytotoxischen Wirkung von Aryloxyphenoxypropionsäureherbiziden der Formel IV

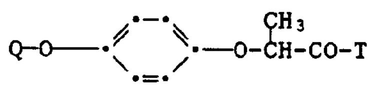 (IV)

worin
Q für den Rest

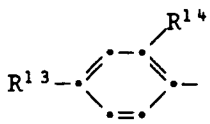 , 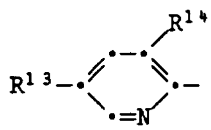 , 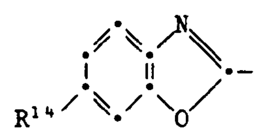 ,

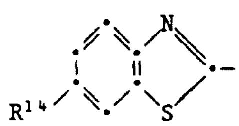 oder 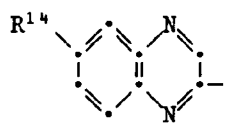

und T für
$-NR^{15}R^{16}$, $-N(CN)R^{17}$, $-OR^{18}$, $SR^{18}$ oder $-O-N=CR^{19}R^{20}$ stehen, wobei

$R^{13}$ Halogen oder Trifluormethyl,

$R^{14}$ Wasserstoff oder Halogen,

$R^{15}$ und $R^{16}$ unabhängig voneinander Wasserstoff, $C_1$-$C_8$-Alkoxy, $C_1$-$C_8$-Alkyl, Phenyl oder Benzyl, $R^{15}$ und $R^{16}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R^{17}$ $C_1$-$C_4$-Alkyl, $C_3$-$C_4$-Alkenyl, $C_3$-$C_4$-Alkinyl oder $C_2$-$C_4$-Alkoxyalkyl

$R^{18}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$, $-CON(C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{21}$, $COSR^{21}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3- bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten, eingesetzt wird.

**48.** Mittel gemäss Anspruch 47, dadurch gekennzeichnet, dass die Aryloxyphenoxypropionsäurederivate der

Formel IV aus der Gruppe
2-[4-(3,5-Dichlorpyridin-2-yloxy)-phenoxy]-propionsäure-propargylester,
2-[4-(3,5-Dichlorpyridin-2-yloxy)-phenoxy]-thiopropionsäure-propargylester,
2-[4-(5-Chlor-3-fluorpyridin-2-yloxy)-phenoxy]-propionsäure-methylester,
2-[4-(5-Chlor-3-fluorpyridin-2-yloxy)-phenoxy]-propionsäure-butylester,
2-[4-(5-Trifluormethylpyridin-2-yloxy)-phenoxy]-propionsäure-butylester,
2-[4-(5-Trifluormethylpyridin-2-yloxy)-phenoxy]-propionsäure-methylester,
2-[4-(6-Chlorchinoxalin-2-yloxy)-phenoxy]-propionsäure-äthylester und
2-[4-(6-Chlorbenzoxazolin-2-yloxy)-phenoxy]-propionsäure-äthylester ausgewählt werden.

**49.** Mittel gemäss Anspruch 38, dadurch gekennzeichnet, dass es zum Schützen von Kulturpflanzen vor der phytotoxischen Wirkung von Acylcyclohexandionherbiziden der Formel IX

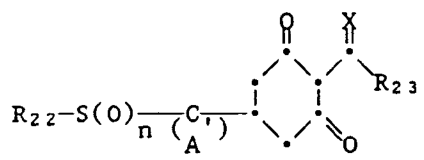

(IX)

worin A′ eine 2-7-gliedrige Alkylenbrücke, eine 3-7-gliedrige Alkenylenbrücke, welche ein- oder mehrfach ungesättigt sein kann,
n′ Null, eins oder zwei
$R_{22}$ $C_1$-$C_4$-Alkyl oder Benzyl
$R_{23}$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl wobei der Phenylring durch Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,
X′ Sauerstoff oder ein Rest -$NOR_{24}$ und
$R_{24}$ $C_1$-$C_4$-Alkyl, $C_1$-$C_6$-Halogenalkyl, $C_3$-$C_6$-Alkenyl, $C_3$-$C_6$-Halogenalkenyl oder $C_3$-$C_6$-Alkinyl bedeuten.

**50.** Mittel gemäss Anspruch 49, dadurch gekennzeichnet, dass die Acylcyclohexandionherbizide aus der Gruppe
5-(1-Methylthiocyclobutan-1-yl)-2-(2,4-dichlorobenzoyl)-cyclohexan-1,3-dion,
5-(1-Methylthiocyclobutan-1-yl)-2-n-butyryl-cyclohexan-1,3-dion,
5-(1-Methylthiocyclobutan-1-yl)-2-cyclopropylcarbonyl-cyclohexan-1,3-dion,
5-(1-Methylthiocyclobutan-1-yl)-2-(2,3-dichlorobenzoyl)-cyclohexan-1,3-dion,
5-(1-Methylsulfonylcyclobutan-1-yl)-2-n-butyryl-cyclohexan-1,3-dion,
5-(1-Methylthiocyclopropan-1-yl)-2-propionyl-cyclohexan-1,3-dion,
5-(1-Aethylthiocyclopropan-1-yl)-2-propionyl-cyclohexan-1,3-dion und
5-(1-Methylthiocyclopropan-1-yl)-2-n-butyryl-cyclohexan-1,3-dion.
5-(1-Methylthiocyclobutan-1-yl)-2-(1-äthoximino-n-butyryl)-cyclohexan-1,3-dion,
5-(1-Methylthiocyclopropan-1-yl)-2-(1-äthoximino-n-butyryl)-cyclohexan-1,3-dion,
5-(1-Methylthiocyclohexan-1-yl)-2-(1-äthoximino-n-butyryl)- cyclohexan-1,3-dion,
5-(1-Methylthiocyclobutan-1-yl)-2-(1-allyloxyimino-n-butyryl)-cyclohexan-1,3-dion und
5-(1-Methylthiocyclopentan-1-yl)-2-(1-äthoximino-n-butyryl)-cyclohexan-1,3-dion,
5-(1-Methylthiocyclopropan-1-yl)-2-[1-(trans-3-chlorallyloximino)-n-butyryl]-cyclohexan-1,3-dion,
5-(1-Methylthiocyclopropan-1-yl)-2-[1-(trans-3-chlorallyloximino)-propionyl]-cyclohexan-1,3-dion,
5-(1-Methylthiocyclopropan-1-yl)-2-[1-(cis-3-chlorallyloximino)-propionyl]-cyclohexan-1,3-dion und
5-(1-Aethylthiocyclopropan-1-yl)-2-[1-(trans-3-chlorallyloximino)-propionyl]-cyclohexan-1,3-dion.
ausgewählt werden.

**51.** Verwendung der Sulfamoylphenylharnstoffe der Formel I gemäss Anspruch 1 zum Schützen von Kulturpflanzen gegen die schädigende Wirkung von Sulfonylharnstoffherbiziden, Chloracetanilidenherbiziden, Acylcyclohexandionherbiziden oder Aryloxyphenoxypropionsäureherbiziden.

**52.** Verwendung der Sulfamoylphenylharnstoffe der Formel I gemäss Anspruch 1 zum Schützen von Kulturpflanzen gegen die schädigende Wirkung von Sulfonylharnstoffherbiziden, Chloracetanilidenherbiziden oder Aryloxyphenoxypropionsäureherbiziden.

**53.** Verwendung gemäss Anspruch 52, dadurch gekennzeichnet, dass es sich bei den Sulfonylharnstoffherbiziden um Verbindungen der Formel II

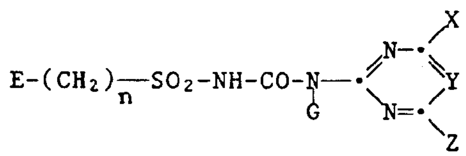

(II)

worin
E eine Gruppe

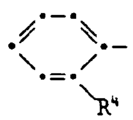 , 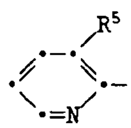 , 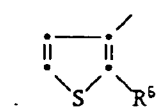 ,

oder

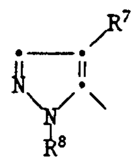 ,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten, handelt.

**54.** Verwendung gemäss Anspruch 52, dadurch gekennzeichnet, dass es sich bei den Chloracetanilidherbiziden um Verbindungen der Formel III

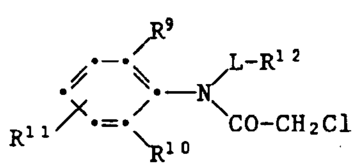

(III)

worin
L eine $C_1$-$C_4$-Alkylenbrücke,
$R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und

$R^{12}$ $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet, handelt.

**55.** Verwendung gemäss Anspruch 52, dadurch gekennzeichnet, dass es sich bei den Aryloxyphenoxypropionsäureherbiziden um Verbindungen der Formel IV

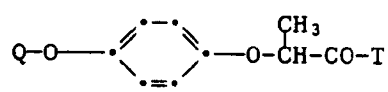

(IV)

worin
Q für den Rest

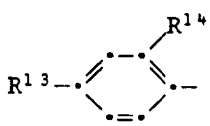

,

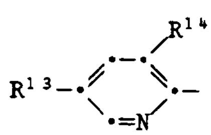

,

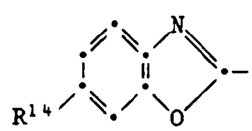

,

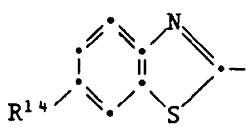

oder

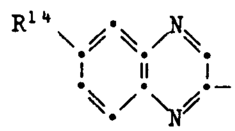

und T für
-$NR^{15}R^{16}$, -$N(CN)R^{17}$, -$OR^{18}$, $SR^{18}$ oder -O-N=$CR^{19}R^{20}$ stehen, wobei

$R^{13}$ Halogen oder Trifluormethyl,

$R^{14}$ Wasserstoff oder Halogen,

$R^{15}$ und $R^{16}$ unabhängig voneinander Wasserstoff, $C_1$-$C_8$-Alkoxy, $C_1$-$C_8$-Alkyl, Phenyl oder Benzyl, $R^{15}$ und $R^{16}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R^{17}$ $C_1$-$C_4$-Alkyl, $C_3$-$C_4$-Alkenyl, $C_3$-$C_4$-Alkinyl oder $C_2$-$C_4$-Alkoxyalkyl

$R^{18}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, -$COOR^{21}$, -$COSR^{21}$, -$CONH_2$, -CON($C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, -$COOR^{21}$, $COSR^{21}$, -$CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3- bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halo-

genalkyl, $C_3$-$C_6$-Alkinyl oder $C_3$-$C_6$-Halogenalkinyl bedeuten, handelt.

**56.** Verwendung gemäss Anspruch 51, dadurch gekennzeichnet, dass es sich bei den Acylcyclohexandionherbizidien um Verbindungen der Formel IX

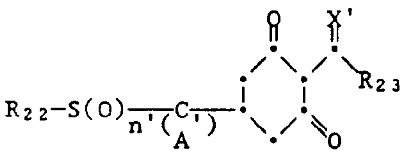

(IX)

worin A' eine 2-7-gliedrige Alkylenbrücke, eine 3-7-gliedrige Alkenylenbrücke, welche ein- oder mehrfach ungesättigt sein kann,

n' Null, eins oder zwei

$R_{22}$ $C_1$-$C_4$-Alkyl oder Benzyl

$R_{23}$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl wobei der Phenylring durch Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,

X' Sauerstoff oder ein Rest -$NOR_{24}$ und

$R_{24}$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl bedeuten.

**57.** Herbizides Mittel zur selektiven Bekämpfung von Unkräutern in Nutzpflanzenkulturen, dadurch gekennzeichnet, dass es als Wirkstoffe sowohl ein Sulfonylharnstoffherbizid, ein Acylcyclohexandionherbizid, ein Chloracetanilidherbizid oder ein Aryloxyphenoxypropionsäureherbizid als auch ein Gegenmittel der Formel I gemäss Anspruch 1 enthält.

**58.** Herbizides Mittel zur selektiven Bekämpfung von Unkräutern in Nutzpflanzenkulturen, dadurch gekennzeichnet, dass es als Wirkstoffe sowohl ein Sulfonylharnstoffherbizid, ein Chloracetanilidherbizid oder ein Aryloxyphenoxypropionsäureherbizid als auch ein Gegenmittel der Formel I gemäss Anspruch 1 enthält.

**59.** Mittel gemäss Anspruch 57, dadurch gekennzeichnet, dass es sich bei dem Acylcyclohexandionheribizid um eine Verbindung der Formel IX

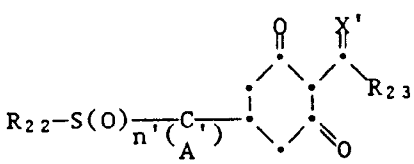

(IX)

worin A' eine 2-7-gliedrige Alkylenbrücke, eine 3-7-gliedrige Alkenylenbrücke, welche ein- oder mehrfach ungesättigt sein kann,

n' Null, eins oder zwei

$R_{22}$ $C_1$-$C_4$-Alkyl oder Benzyl

$R_{23}$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl wobei der Phenylring durch Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,

X' Sauerstoff oder ein Rest -$NOR_{24}$ und

$R_{24}$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl bedeuten.

**60.** Mittel gemäss Anspruch 58, dadurch gekennzeichnet, dass es sich bei dem Sulofonylharnstoffherbizid um eine Verbindung der Formel II

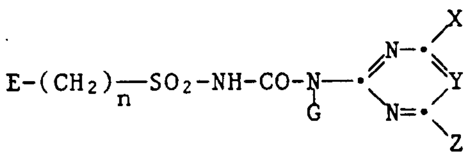

(II)

worin
E eine Gruppe

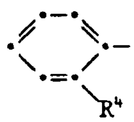 , 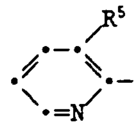 , 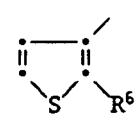 ,

oder

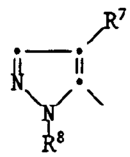 ,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten, handelt.

**61.** Mittel gemäss Anspruch 58, dadurch gekennzeichnet, dass es sich bei dem Chloracetanilidherbizid um eine Verbindung der Formel III

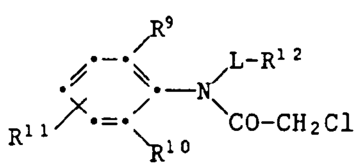

(III)

worin
L eine $C_1$-$C_4$-Alkylenbrücke,
$R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R^{12}$ $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,

gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet, handelt.

**62.** Mittel gemäss Anspruch 58, dadurch gekennzeichnet, dass es sich bei dem Aryloxyphenoxypropionsäuredherbizid um eine Verbindung der Formel IV

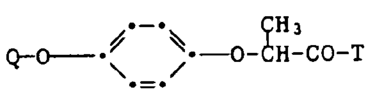

(IV)

worin
Q für den Rest

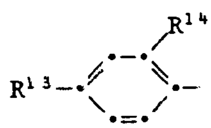

,

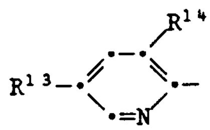

,

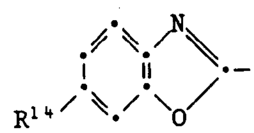

,

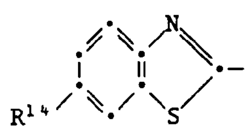

oder

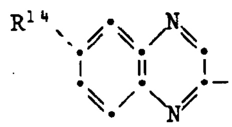

und T für
$-NR^{15}R^{16}$, $-N(CN)R^{17}$, $-OR^{18}$, $SR^{18}$ oder $-O-N=CR^{19}R^{20}$ stehen, wobei

$R^{13}$ Halogen oder Trifluormethyl,

$R^{14}$ Wasserstoff oder Halogen,

$R^{15}$ und $R^{16}$ unabhängig voneinander Wasserstoff, $C_1$-$C_8$-Alkoxy, $C_1$-$C_8$-Alkyl, Phenyl oder Benzyl, $R^{15}$ und $R^{16}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R^{17}$ $C_1$-$C_4$-Alkyl, $C_3$-$C_4$-Alkenyl, $C_3$-$C_4$-Alkinyl oder $C_2$-$C_4$-Alkoxyalkyl

$R^{18}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$, $-CON(C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{21}$, $COSR^{21}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3- bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten, handelt.

**63.** Verfahren zur selektiven Bekämpfung von Unkräutern in Nutzpflanzenkulturen, dadurch gekennzeichnet, dass man die Kulturpflanzen oder deren Anbaufläche sowohl mit einem Sulfonylharnstoffherbizid, einem Acylcyclohexandionherbizid, einem Chloracetanilidherbizid oder einem Aryloxyphenoxyropionsäureher-

bizid als auch einer wirksamen Menge eines N-Acylsulfamoylphenylharnstoffes der Formel I gemäss Anspruch 1 als Gegenmittel behandelt.

**64.** Verfahren zum Schützen von Kulturpflanzen vor Schäden, die bei der Applikation von Sulfonylharnstoffherbiziden, Acylcyclohexandionherbiziden, Chloracetanilidherbiziden oder Aryloxyphenoxypropionsäureherbiziden auftreten, dadurch gekennzeichnet, dass man entweder die Anbaufläche für die Pflanze vor oder während der Applikation des Herbizids oder den Samen oder die Stecklinge der Pflanzen oder die Pflanze selbst mit einer wirksamen Menge eines N-Acylsulfamoylphenylharnstoffs der Formel I gemäss Anspruch 1 behandelt.

**65.** Verfahren zur selektiven Bekämpfung von Unkräutern in Nutzpflanzenkulturen, dadurch gekennzeichnet, dass man die Kulturpflanzen oder deren Anbaufläche sowohl mit einem Sulfonylharnstoffherbizid, einem Chloracetanilidherbizid oder einem Aryloxyphenoxypropionsäureherbizid als auch einer wirksamen Menge eines N-Acylsulfamoylphenylharnstoffes der Formel I gemäss Anspruch 1 als Gegenmittel behandelt.

**66.** Verfahren zum Schützen von Kulturpflanzen vor Schäden, die bei der Applikation von Sulfonylharnstoffherbiziden, Chloracetanilidherbiziden oder Aryloxyphenoxypropionsäureherbiziden auftreten, dadurch gekennzeichnet, dass man entweder die Anbaufläche für die Pflanze vor oder während der Applikation des Herbizids oder den Samen oder die Stecklinge der Pflanzen oder die Pflanze selbst mit einer wirksamen Menge eines N-Acylsulfamoylphenylharnstoffs der Formel I gemäss Anspruch 1 behandelt.

**67.** Saatgut von Nutzpflanzen, das mit einer antagonistisch wirksamen Menge eines N-Acylsulfamoylphenylharnstoffs der Formel I gemäss Anspruch 1 behandelt worden ist.

**68.** Saatgut gemäss Anspruch 67, dadurch gekennzeichnet, dass es sich um Saatgut von Getreide, Soja, Reis, Mais oder Sorghum handelt.

**69.** Saatgut gemäss Anspruch 67, dadurch gekennzeichnet, dass es sich um Saatgut von Sorghum, Mais oder Reis handelt.

**70.** Verfahren zur Herstellung der Sulfamoylphenylharnstoffe der Formel I, worin $R^1$ die unter Formel I in Anspruch 1 gegebenen Bedeutungen mit Ausnahme von Wasserstoff hat und $R^2$ für Wasserstoff steht, dadurch gekennzeichnet, dass man ein Sulfamoylanilin der Formel V

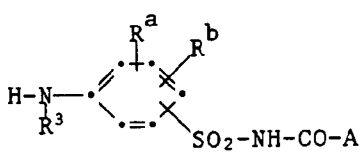

(V)

worin A, $R^3$, $R^a$ und $R^b$ die unter Formel I gegebenen Bedeutungen haben, mit einem Isocyanat der Formel VI

$R^1$-N=C=O (VI)

worin $R^1$ die unter Formel I gegebenen Bedeutungen mit Ausnahme von Wasserstoff hat, umsetzt.

**71.** Verfahren zur Herstellung der Verbindungen der Formel I gemäss Anspruch 1, dadurch gekennzeichnet, dass man einen Sulfamoylphenylharnstoff der Formel VII

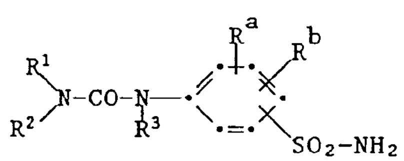

(VII)

worin $R^1$, $R^2$, $R^3$, $R^a$ und $R^b$ die unter Formel I gegebenen Bedeutungen haben, mit einem Carbonsäurehalogenid der Formel VIII

Hal-CO-A (VIII)

worin A die unter Formel I gegebenen Bedeutungen hat und Hal Chlor oder Brom bedeutet, acyliert.

**72.** 1-[4-(N-2-Äthoxybenzoyl-sulfamoyl)-phenyl]-3-methyl-harnstoff.

**Patentansprüche für folgenden Vertragsstaat: ES**

**1.** Verfahren zur Herstellung der Verbindungen der Formel I

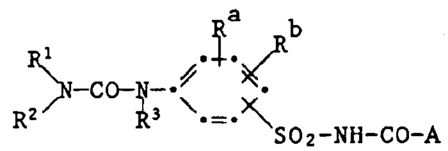

(I)

worin
A für einen Rest aus der Gruppe

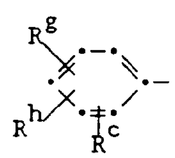

,

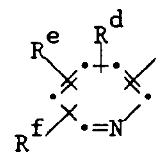

,

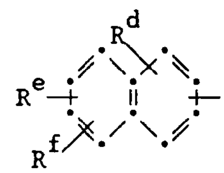

,

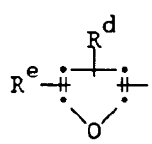

oder

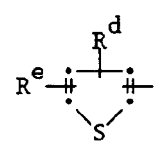

,

$R^1$ und $R^2$ unabhängig voneinander für Wasserstoff, $C_1$-$C_8$-Alkyl, $C_3$-$C_8$-Cycloalkyl, $C_3$-$C_6$-Alkenyl, $C_3$-$C_6$-Alkinyl,

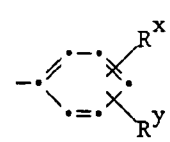

oder durch $C_1$-$C_4$-Alkoxy oder

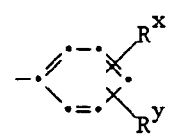

substituiertes $C_1$-$C_4$-Alkyl, oder
$R^1$ und $R^2$ gemeinsam für eine $C_4$-$C_6$-Alkylenbrücke oder eine durch Sauerstoff, Schwefel, SO, $SO_2$, NH oder -N($C_1$-$C_4$-Alkyl)- unterbrochene $C_4$-$C_6$-Alkylenbrücke,
$R^3$ für Wasserstoff oder $C_1$-$C_4$-Alkyl,
$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $-COOR^j$, $-CONR^kR^m$, $-COR^n$, $-SO_2NR^kR^m$ oder $-OSO_2$-$C_1$-$C_4$-Alkyl, oder $R^a$ und $R^b$ gemeinsam für eine $C_3$-$C_4$-Alkylenbrücke, die durch Halogen oder $C_1$-$C_4$-Alkyl substituiert sein kann, oder eine $C_3$-$C_4$-Alkenylenbrücke, die durch Halogen oder $C_3$-$C_4$-Alkyl substituiert sein kann, oder eine $C_4$-Alkadienylenbrücke, die durch Halogen oder $C_1$-$C_4$-Alkyl substituiert sein kann, und

$R^g$ und $R^h$ unabhängig voneinander für Wasserstoff, Halogen, $C_1$-$C_4$-Alkyl, Trifluormethyl, Methoxy, Methylthio oder -COOR$^j$ stehen, wobei
$R^c$ Wasserstoff, Halogen, $C_1$-$C_4$-Alkyl oder Methoxy,
$R^d$ Wasserstoff, Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, -COOR$^j$ oder -CONR$^k$ $R^m$,
$R^e$ Wasserstoff, Halogen, $C_1$-$C_4$-Alkyl, -COOR$^j$, Trifluormethyl oder Methoxy, oder $R^d$ und $R^e$ gemeinsam für eine $C_3$-$C_4$-Alkylenbrücke,
$R^f$ Wasserstoff, Halogen oder $C_1$-$C_4$-Alkyl,
$R^x$ und $R^y$ unabhängig voneinander Wasserstoff, Halogen, $C_1$-$C_4$-Alkyl, $C_1$-$C_4$-Alkoxy, $C_1$-$C_4$-Alkylthio, -COOR$^4$, Trifluormethyl, Nitro oder Cyan,
$R^j$, $R^k$ und $R^m$ unabhängig voneinander Wasserstoff oder $C_1$-$C_4$-Alkyl,
$R^k$ und $R^m$ gemeinsam eine $C_4$-$C_6$-Alkylenbrücke oder eine durch Sauerstoff, NH oder -N($C_1$-$C_4$-Alkyl)-unterbrochene $C_4$-$C_6$-Alkylenbrücke, und
$R^n$ $C_1$-$C_4$-Alkyl, Phenyl oder durch Halogen, $C_1$-$C_4$-Alkyl, Methoxy, Nitro oder Trifluormethyl substituiertes Phenyl bedeuten, dadurch gekennzeichnet, dass man einen Sulfamoylphenylharnstoff der Formel VII

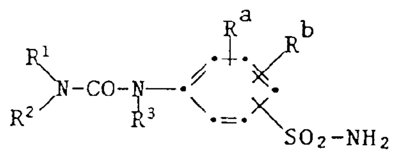

(VII)

worin $R^1$, $R^2$, $R^3$, $R^a$ und $R^b$ die unter Formel I gegebenen Bedeutungen haben, mit einem Carbonsäurehalogenid der Formel VIII

Hal-CO-A (VIII)

worin A die unter Formel I gegebenen Bedeutungen hat und Hal Chlor oder Brom bedeutet, acyliert.

2. Verfahren nach Anspruch 1 zur Herstellung der Verbindungen der Formel I, in denen
A für einen Rest aus der Gruppe

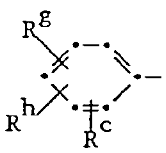

,

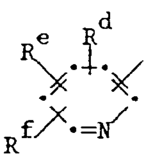

,

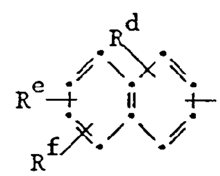

,

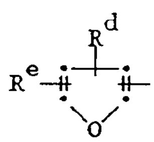

oder

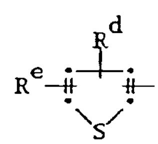

,

$R^1$ und $R^2$ unabhängig voneinander für Wasserstoff, $C_1$-$C_8$-Alkyl, $C_3$-$C_8$-Cycloalkyl, $C_3$-$C_6$-Alkenyl, $C_3$-$C_6$-Alkinyl,

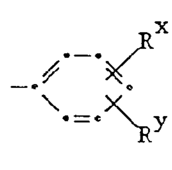

oder durch $C_1$-$C_4$-Alkoxy oder

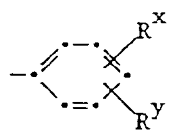

substituiertes $C_1$-$C_4$-Alkyl, oder
$R^1$ und $R^2$ gemeinsam für eine $C_4$-$C_6$-Alkylenbrücke oder eine durch Sauerstoff, Schwefel, SO, $SO_2$, NH oder -N($C_1$-$C_4$-Alkyl)- unterbrochene $C_4$-$C_6$-Alkylenbrücke,
$R^3$ für Wasserstoff oder $C_1$-$C_4$-Alkyl,
$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, -COOR$^j$, -CONR$^k$R$^m$, -COR$^n$, -$SO_2$ NR$^k$R$^m$ oder -OSO$_2$-$C_1$-$C_4$-Alkyl, und
$R^g$ und $R^h$ unabhängig voneinander für Wasserstoff, Halogen, $C_1$-$C_4$-Alkyl, Trifluormethyl, Methoxy, Methylthio oder -COOR$^j$ stehen, wobei
$R^c$ Wasserstoff, Halogen, $C_1$-$C_4$-Alkyl oder Methoxy,
$R^d$ Wasserstoff, Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, -COOR$^j$ oder -CONR$^k$ R$^m$,
$R^e$ Wasserstoff, Halogen, $C_1$-$C_4$-Alkyl, -COOR$^j$, Trifluormethyl oder Methoxy, oder $R^d$ und $R^e$ gemeinsam für eine $C_3$-$C_4$-Alkylenbrücke
$R^f$ Wasserstoff, Halogen oder $C_1$-$C_4$-Alkyl,
$R^x$ und $R^y$ unabhängig voneinander Wasserstoff, Halogen, $C_1$-$C_4$-Alkyl, $C_1$-$C_4$-Alkoxy, $C_1$-$C_4$-Alkylthio, -COOR$^4$, Trifluormethyl, Nitro oder Cyan,
$R^j$, $R^k$ und $R^m$ unabhängig voneinander Wasserstoff oder $C_1$-$C_4$-Alkyl,
$R^k$ und $R^m$ gemeinsam eine $C_4$-$C_6$-Alkylenbrücke oder eine durch Sauerstoff, NH oder -N($C_1$-$C_4$-Alkyl)- unterbrochene $C_4$-$C_6$-Alkylenbrücke, und
$R^n$ $C_1$-$C_4$-Alkyl, Phenyl oder durch Halogen, $C_1$-$C_4$-Alkyl, Methoxy, Nitro oder Trifluormethyl substituiertes Phenyl bedeuten.

3. Verfahren nach Anspruch 1 zur Herstellung der Verbindungen der Formel I, in denen $R^b$ Wasserstoff bedeutet.

4. Verfahren nach Anspruch 1 zur Herstellung der Verbindungen der Formel I, in denen die Sulfamoylgruppe die 4-Stellung am Phenylring besetzt.

5. Verfahren nach Anspruch 1 zur Herstellung der Verbindungen der Formel I, in denen $R^2$ und $R^3$ Wasserstoff bedeuten.

6. Verfahren nach Anspruch 1 zur Herstellung der Verbindungen der Formel I, in denen $R^b$ Wasserstoff bedeutet und die Sulfamoylgruppe die 4-Stellung am Phenylring einnimmt.

7. Verfahren nach Anspruch 1 zur Herstellung der Verbindungen der Formel I, in denen $R^2$, $R^3$ und $R^b$ Wasserstoff bedeuten und die Sulfamoylgruppe die 4-Stellung am Phenylring einnimmt.

8. Verfahren nach Anspruch 1 zur Herstellung der Verbindungen der Formel I, in denen A für die Gruppe

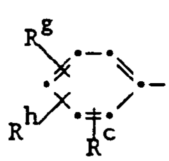

steht.

9. Verfahren nach Anspruch 8 zur Herstellung der Verbindungen der Formel I, in denen $R^2$, $R^3$ und $R^b$ für Wasserstoff stehen und die Sulfamoylgruppe die 4-Stellung am Phenylring besetzt.

10. Verfahren nach Anspruch 1 zur Herstellung der Verbindungen der Formel I, in denen sie der Unterformel Ia

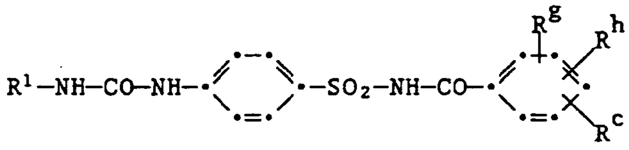

(Ia)

.

11. Verfahren nach Anspruch 10 zur Herstellung der Verbindungen der Unterformel Ia, in denen $R^c$ Wasserstoff bedeutet.

12. Verfahren nach Anspruch 11 zur Herstellung der Verbindungen der Unterformel I, in denen $R^1$, $R^g$ und $R^h$ $C_1$-$C_4$-Alkyl bedeuten.

13. Verfahren nach Anspruch 1 zur Herstellung von 1-[4-(N-4-Methylbenzoyl-sulfamoyl)-phenyl]-3-methylharnstoff.

14. Verfahren nach Anspruch 1 zur Herstellung von 1-[4-(N-3-Methylbenzoyl-sulfamoyl)-phenyl]-3-methylharnstoff.

15. Verfahren nach Anspruch 1 zur Herstellung von 1-[4-(N-4-tert.Butylbenzoyl-sulfamoyl)-phenyl]-3-methylharnstoff.

16. Verfahren nach Anspruch 1 zur Herstellung von 1-[4-(N-3-Trifluormethylbenzoyl-sulfamoyl)-phenyl]-3-methylharnstoff.

17. Verfahren nach Anspruch 1 zur Herstellung von 1-[4-(N-4-Nitrobenzoyl-sulfamoyl)-phenyl]-3-methylharnstoff.

18. Verfahren nach Anspruch 1 zur Herstellung von 1-[4-(N-2,3-Dimethylbenzoyl-sulfamoyl)-phenyl]-3-methylharnstoff.

19. Verfahren nach Anspruch 1 zur Herstellung von 1-[4-(N-3,4-Dimethylbenzoyl-sulfamoyl)-phenyl]-3-methylharnstoff.

20. Verfahren nach Anspruch 1 zur Herstellung von 1-[4-(N-3,4-Dimethylbenzoyl-sulfamoyl)-phenyl]-3,3-dimethylharnstoff.

21. Verfahren nach Anspruch 1 zur Herstellung von 1-[4-(N-3,4-Dimethylbenzoyl-sulfamoyl)-phenyl]-3-äthylharnstoff.

22. Verfahren nach Anspruch 1 zur Herstellung von 1-[4-(N-3,4-Dimethylbenzoyl-sulfamoyl)-phenyl]-3-allylharnstoff.

23. Verfahren nach Anspruch 1 zur Herstellung von 1-[4-(N-3,4-Dimethylbenzoyl-sulfamoyl)-phenyl]-3-phenylharnstoff.

24. Verfahren nach Anspruch 1 zur Herstellung von 1-[4-(N-3,5-Dimethylbenzoyl-sulfamoyl)-phenyl]-3-methylharnstoff.

25. Verfahren nach Anspruch 1 zur Herstellung von 1-[4-(N-3,4-Dichlorbenzoyl-sulfamoyl)-phenyl]-3-methylharnstoff.

26. Verfahren nach Anspruch 1 zur Herstellung von 1-[4-(N-3,4-Dimethoxybenzoyl-sulfamoyl)-phenyl]-3-methylharnstoff.

27. Verfahren nach Anspruch 1 zur Herstellung von 1-[4-(N-3,4-Dimethoxybenzoyl-sulfamoyl)-phenyl]-3,3-dimethylharnstoff.

28. Verfahren nach Anspruch 1 zur Herstellung von 1-[4-(N-2,4,5-Trimethoxybenzoyl-sulfamoyl)-phenyl]-3-methylharnstoff.

**29.** Verfahren nach Anspruch 1 zur Herstellung von 1-[4-(N-1-Naphthylcarbonyl-sulfamoyl)-phenyl]-3-methylharnstoff.

**30.** Verfahren nach Anspruch 1 zur Herstellung von 1-[4-(N-2-Furylcarbonyl-sulfamoyl)-phenyl]-3-methylharnstoff.

**31.** Verfahren nach Anspruch 1 zur Herstellung von 1-[4-(N-2-Furylcarbonyl-sulfamoyl)-phenyl]-3,3-dimethylharnstoff.

**32.** Verfahren nach Anspruch 1 zur Herstellung von 1-[4-(N-2-Thienylcarbonyl-sulfamoyl)-phenyl]-3-methylharnstoff.

**33.** Verfahren nach Anspruch 1 zur Herstellung von 1-[4-(N-Piperonyloyl-sulfamoyl)-phenyl]-3-methylharnstoff.

**34.** Verfahren nach Anspruch 1 zur Herstellung von 1-[4-(N-3-Methylbenzoyl-sulfamoyl)-phenyl]-3,3-dimethylharnstoff.

**35.** Verfahren nach Anspruch 1 zur Herstellung von 1-[4-(N-3-Trifluormethylbenzoyl-sulfamoyl)-phenyl]-3-cyclopropylharnstoff.

**36.** Verfahren nach Anspruch 1 zur Herstellung von 1-[3-(N-3,4-Dimethylbenzoyl-sulfamoyl)-phenyl]-3-methylharnstoff.

**37.** Verfahren nach Anspruch 1 zur Herstellung von 1-[3-(N-2-Furylcarbonyl-sulfamoyl)-phenyl]-3,3-dimethylharnstoff.

**38.** Verwendung der Sulfamoylphenylharnstoffe der Formel I gemäss Anspruch 1 zum Schützen von Kulturpflanzen gegen die schädigende Wirkung von Sulfonylharnstoffherbiziden, Chloracetanilidherbiziden, Acylcyclohexandionherbiziden oder Aryloxyphenoxypropionsäureherbiziden.

**39.** Verwendung der Sulfamoylphenylharnstoffe der Formel I gemäss Anspruch 1 zum Schützen von Kulturpflanzen gegen die schädigende Wirkung von Sulfonylharnstoffherbiziden, Chloracetanilidherbiziden oder Aryloxyphenoxypropionsäureherbiziden.

**40.** Verwendung gemäss Anspruch 39, dadurch gekennzeichnet, dass es sich bei den Sulfonylharnstoffherbiziden um Verbindungen der Formel II

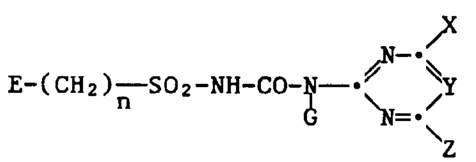

(II)

worin
E eine Gruppe

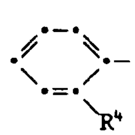 , 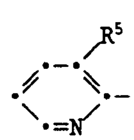 , 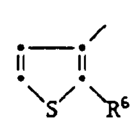 ,

oder

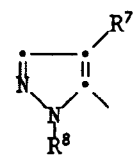,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten, handelt.

41. Verwendung gemäss Anspruch 39, dadurch gekennzeichnet, dass es sich bei den Chloracetanilidherbiziden um Verbindungen der Formel III

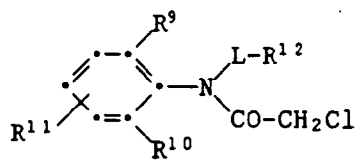

(III)

worin
L eine $C_1$-$C_4$-Alkylenbrücke,
$R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R^{12}$ $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet, handelt.

42. Verwendung gemäss Anspruch 39, dadurch gekennzeichnet, dass es sich bei den Aryloxyphenoxypropionsäureherbiziden um Verbindungen der Formel IV

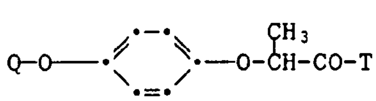

(IV)

worin

Q für den Rest

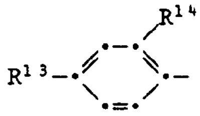, 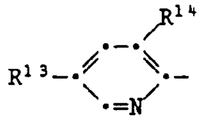, 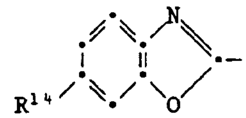,

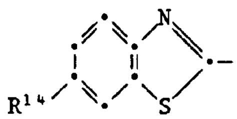 oder 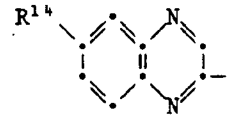

und T für
$-NR^{15}R^{16}$, $-N(CN)R^{17}$, $-OR^{18}$, $SR^{18}$ oder $-O-N=CR^{19}R^{20}$ stehen, wobei

$R^{13}$ Halogen oder Trifluormethyl,

$R^{14}$ Wasserstoff oder Halogen,

$R^{15}$ und $R^{16}$ unabhängig voneinander Wasserstoff, $C_1$-$C_8$-Alkoxy, $C_1$-$C_8$-Alkyl, Phenyl oder Benzyl, $R^{15}$ und $R^{16}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R^{17}$ $C_1$-$C_4$-Alkyl, $C_3$-$C_4$-Alkenyl, $C_3$-$C_4$-Alkinyl oder $C_2$-$C_4$-Alkoxyalkyl

$R^{18}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$, $-CON(C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{21}$, $COSR^{21}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3- bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten, handelt.

**43.** Verwendung gemäss Anspruch 38, dadurch gekennzeichnet, dass es sich bei den Acylcyclohexandionherbizidien um Verbindungen der Formel IX

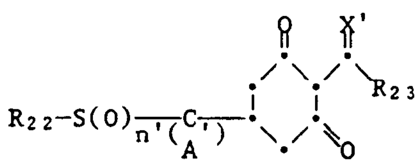 (IX)

worin A′ eine 2-7-gliedrige Alkylenbrücke, eine 3-7-gliedrige Alkenylenbrücke, welche ein- oder mehrfach ungesättigt sein kann,

n′ Null, eins oder zwei

$R_{22}$ $C_1$-$C_4$-Alkyl oder Benzyl

$R_{23}$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl wobei der Phenylring durch Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,

X′ Sauerstoff oder ein Rest $-NOR_{24}$ und

$R_{24}$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl bedeuten.

44. Verfahren nach Anspruch 1 zur Herstellung von 1-[4-(N-2-Äthoxybenzoyl-sulfamoyl)-phenyl]-3-methylharnstoff.

## Claims

**Claims for the following Contracting States: AT, BE, CH, DE, FR, GB, GR, IT, LI, LU, NL, SE**

1. An N-acylsulfamoylphenylurea of formula I

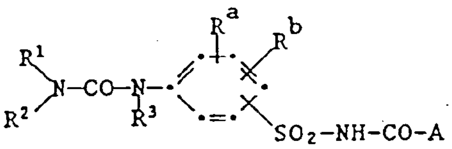 (I),

wherein A is a radical selected from the group

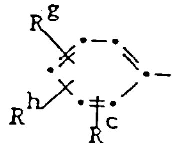 , 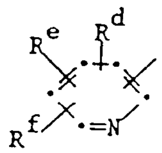 , 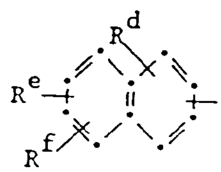 ,

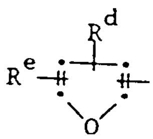 . or 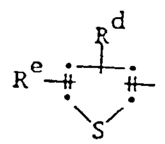 ,

each of $R^1$ and $R^2$, independently of the other, is hydrogen, $C_1$-$C_8$alkyl, $C_3$-$C_8$cycloalkyl, $C_3$-$C_6$alkenyl, $C_3$-$C_6$alkynyl,

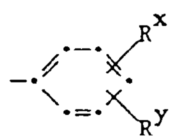

or $C_1$-$C_4$alkyl substituted by $C_1$-$C_4$-alkoxy or by

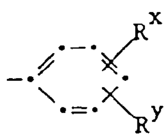 ,

or $R^1$ and $R^2$ together form a $C_4$-$C_6$alkylene bridge, or a $C_4$-$C_6$alkylene bridge interrupted by oxygen, sulfur, SO, $SO_2$, NH or by -N($C_1$-$C_4$alkyl)-, $R^3$ is hydrogen or $C_1$-$C_4$alkyl, each of $R^a$ and $R^b$, independently of the other, is hydrogen, halogen, cyano, nitro, trifluoromethyl, $C_1$-$C_4$alkyl, $C_1$-$C_4$alkoxy, $C_1$-$C_4$alkylthio, $C_1$-$C_4$alkylsulfinyl, $C_1$-$C_4$alkylsulfonyl, $-COOR^j$, $-CONR^kR^m$, $-COR^n$, $-SO_2NR^kR^m$ or $-OSO_2$-$C_1$-$C_4$alkyl, or $R^a$ and $R^b$ together form a $C_3$-$C_4$-alkylene bridge, which can be substituted by halogen or by $C_1$-$C_4$alkyl, or a $C_3$-$C_4$alkenylene bridge, which can be substituted by halogen or by $C_3$-$C_4$alkyl, or a $C_4$alkadienylene bridge which can be substituted by halogen or by $C_1$-$C_4$alkyl, and each of $R^g$ and $R^h$, independently of the other, is hydrogen, halogen, $C_1$-$C_4$alkyl, trifluoromethyl, methoxy, methylthio or $-COOR^j$, wherein $R^c$ is hydrogen, halogen, $C_1$-$C_4$alkyl or methoxy, $R^d$ is hydrogen, halogen, nitro, $C_1$-$C_4$alkyl, $C_1$-$C_4$alkoxy, $C_1$-$C_4$alkylthio, $C_1$-$C_4$alkylsulfinyl, $C_1$-$C_4$alkylsulfonyl, $-COOR^j$ or $-CONR^kR^m$, $R^e$ is hydrogen, halogen, $C_1$-$C_4$alkyl, $-COOR^j$, trifluoromethyl or methoxy, or $R^d$ and $R^e$ together form a $C_3$-$C_4$alkylene bridge, $R^f$ is hydrogen,

halogen or $C_1$-$C_4$alkyl, each of $R^x$ and $R^y$, independently of the other, is hydrogen, halogen, $C_1$-$C_4$alkyl, $C_1$-$C_4$alkoxy, $C_1$-$C_4$alkylthio, -$COOR^4$, trifluoromethyl, nitro or cyano, each of $R^j$, $R^k$ and $R^m$, independently of the others, is hydrogen or $C_1$-$C_4$alkyl, $R^k$ and $R^m$ together form a $C_4$-$C_6$alkylene bridge, or a $C_4$-$C_6$alkylene bridge interrupted by oxygen, NH or by -N($C_1$-$C_4$alkyl)-, and $R^n$ is $C_1$-$C_4$alkyl, phenyl, or phenyl substituted by halogen, $C_1$-$C_4$alkyl, methoxy, nitro or by trifluoromethyl, or a compound 1-[4-(N-3-chloromethylbenzoyl-sulfamoyl)-phenyl]-3-methylurea,
1-[4-(N-2-ethoxybenzoyl-sulfamoyl)-phenyl]-3-methylurea,
1-[4-(N-3-ethoxybenzoyl-sulfamoyl)-phenyl]-3-methylurea,
1-[4-(N-4-ethoxybenzoyl-sulfamoyl)-phenyl]-3-methylurea,
1-[4-(N-2-methylsulfonylbenzoyl-sulfamoyl)-phenyl]-3-methylurea, 1-[4-(N-2-nitrobenzoyl-sulfamoyl)-phenyl]-3-methylurea, 1-[4-(N-3-nitrobenzoyl-sulfamoyl)-phenyl]-3-methylurea, 1-[4-(N-4-nitrobenzoyl-sulfamoyl)-phenyl]-3-methylurea, 1-[4-(N-2-acetylbenzoyl-sulfamoyl)-phenyl]-3-methylurea, 1-[4-(N-4-acetylbenzoyl-sulfamoyl)-phenyl]-3-methylurea, 1-[4-(N-3-cyanobenzoyl-sulfamoyl)-phenyl]-3-methylurea, 1-[4-(N-4-cyanobenzoyl-sulfamoyl)-phenyl]-3-methylurea, 1-[4-(N-3,4-dinitrobenzoyl-sulfamoyl)-phenyl]-3-methylurea, 1-[4-(N-3,5-dinitrobenzoyl-sulfamoyl)-phenyl]-3-methylurea, 1-[4-(N-2-chloro-3-nitrobenzoyl-sulfamoyl)-phenyl]-3-methylurea, 1-[4-(N-2-chloro-4-nitrobenzoyl-sulfamoyl)-phenyl]-3-methylurea, 1-[4-(N-2-chloro-5-nitrobenzoyl-sulfamoyl)-phenyl]-3-methylurea, 1-[4-(N-3-chloro-2-nitrobenzoyl-sulfamoyl)-phenyl]-3-methylurea, 1-[4-(N-4-chloro-2-nitrobenzoyl-sulfamoyl)-phenyl]-3-methylurea, 1-[4-(N-3-chloro-3-nitrobenzoyl-sulfamoyl)-phenyl]-3-methylurea, 1-[4-(N-5-chloro-2-nitrobenzoyl-sulfamoyl)-phenyl]-3-methylurea, 1-[4-(N-2-methyl-3-nitrobenzoyl-sulfamoyl)-phenyl]-3-methylurea, 1-[4-(N-2-methyl-5-nitrobenzoyl-sulfamoyl)-phenyl]-3-methylurea, 1-[4-(N-2-methyl-6-nitrobenzoyl-sulfamoyl)-phenyl]-3-methylurea, 1-[4-(N-3-methyl-2-nitrobenzoyl-sulfamoyl)-phenyl]-3-methylurea, 1-[4-(N-3-methyl-4-nitrobenzoyl-sulfamoyl)-phenyl]-3-methylurea, 1-[4-(N-4-methyl-3-nitrobenzoyl-sulfamoyl)-phenyl]-3-methylurea, 1-[4-(N-5-methyl-2-nitrobenzoyl-sulfamoyl)-phenyl]-3-methylurea and 1-[4-(N-4-fluoro-3-nitrobenzoyl-sulfamoyl)-phenyl]-3-methylurea.

2. A compound of formula I according to claim 1 wherein A is a radical selected from the group

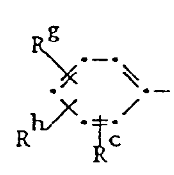 , 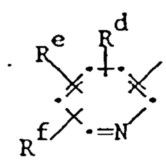 , 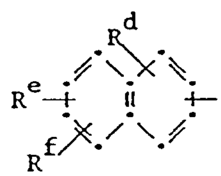 ,

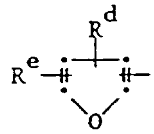 or 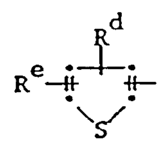 ,

each of $R^1$ and $R^2$, independently of the other, is hydrogen, $C_1$-$C_8$alkyl, $C_3$-$C_8$cycloalkyl, $C_3$-$C_6$alkenyl, $C_3$-$C_6$alkynyl,

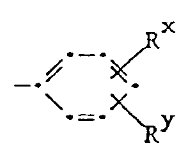

or $C_1$-$C_4$alkyl substituted by $C_1$-$C_4$alkoxy or by

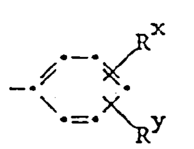 ,

or $R^1$ and $R^2$ together form a $C_4$-$C_6$alkylene bridge, or a $C_4$-$C_6$alkylene bridge interrupted by oxygen, sulfur,

SO, $SO_2$, NH or by -N($C_1$-$C_4$alkyl)-, $R^3$ is hydrogen or $C_1$-$C_4$alkyl, each of $R^a$ and $R^b$, independently of the other, is hydrogen, halogen, cyano, nitro, trifluoromethyl, $C_1$-$C_4$alkyl, $C_1$-$C_4$alkoxy, $C_1$-$C_4$alkylthio, $C_1$-$C_4$alkylsulfinyl, $C_1$-$C_4$alkylsulfonyl, -COOR$^j$, -CONR$^k$R$^m$, -COR$^n$, -$SO_2$NR$^k$R$^m$ or -$OSO_2$-$C_1$-$C_4$alkyl, and each of $R^g$ and $R^h$, independently of the other, is hydrogen, halogen, $C_1$-$C_4$alkyl, trifluoromethyl, methoxy, methylthio or -COOR$^j$, wherein $R^c$ is hydrogen, halogen, $C_1$-$C_4$alkyl or methoxy, $R^d$ is hydrogen, halogen, nitro, $C_1$-$C_4$alkyl, $C_1$-$C_4$alkoxy, $C_1$-$C_4$alkylthio, $C_1$-$C_4$alkylsulfinyl, $C_1$-$C_4$-alkylsulfonyl, -COOR$^j$ or -CONR$^k$R$^m$, $R^e$ is hydrogen, halogen, $C_1$-$C_4$alkyl, -COOR$^j$, trifluoromethyl or methoxy, or $R^d$ and $R^e$ together form a $C_3$-$C_4$alkylene bridge, $R^f$ is hydrogen, halogen or $C_1$-$C_4$alkyl, each of $R^x$ and $R^y$, independently of the other, is hydrogen, halogen, $C_1$-$C_4$alkyl, $C_1$-$C_4$alkoxy, $C_1$-$C_4$alkylthio, -COOR$^4$, trifluoromethyl, nitro or cyano, each of $R^j$, $R^k$ and $R^m$, independently of the others, is hydrogen or $C_1$-$C_4$alkyl, $R^k$ and $R^m$ together form a $C_4$-$C_6$alkylene bridge, or a $C_4$-$C_6$alkylene bridge interrupted by oxygen, NH or by -N($C_1$-$C_4$alkyl)-, and $R^n$ is $C_1$-$C_4$alkyl, phenyl, or phenyl substituted by halogen, $C_1$-$C_4$alkyl, methoxy, nitro or by trifluoromethyl.

**3.** A compound according to claim 1 wherein $R^b$ is hydrogen.

**4.** A compound according to claim 1 wherein the sulfamoyl group occupies the 4-position of the phenyl ring.

**5.** A compound according to claim 1 wherein $R^2$ and $R^3$ are hydrogen.

**6.** A compound according to claim 1 wherein $R^b$ is hydrogen and the sulfamoyl group occupies the 4-position of the phenyl ring.

**7.** A compound according to claim 1 wherein $R^2$, $R^3$ and $R^b$ are hydrogen and the sulfamoyl group occupies the 4-position of the phenyl ring.

**8.** A compound according to claim 1 wherein A is the group

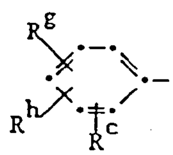

.

**9.** A compound according to claim 8 wherein $R^2$, $R^3$ and $R^b$ are hydrogen and the sulfamoyl group occupies the 4-position of the phenyl ring.

**10.** A compound according to claim 1 falling within the scope of sub-formula Ia

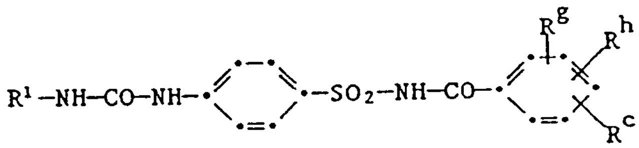

(Ia).

**11.** A compound according to claim 10 wherein $R^c$ is hydrogen.

**12.** A compound according to claim 11 wherein $R^1$, $R^g$ and $R^h$ are $C_1$-$C_4$alkyl.

**13.** 1-[4-(N-4-methylbenzoyl-sulfamoyl)-phenyl]-3-methylurea according to claim 1.

**14.** 1-[4-(N-3-methylbenzoyl-sulfamoyl)-phenyl]-3-methylurea according to claim 1.

**15.** 1-[4-(N-4-tert-butylbenzoyl-sulfamoyl)-phenyl]-3-methylurea according to claim 1.

**16.** 1-[4-(N-3-trifluoromethylbenzoyl-sulfamoyl)-phenyl]-3-methylurea according to claim 1.

**17.** 1-[4-(N-4-nitrobenzoyl-sulfamoyl)-phenyl]-3-methylurea according to claim 1.

**18.** 1-[4-(N-2,3-dimethylbenzoyl-sulfamoyl)-phenyl]-3-methylurea according to claim 1.

19. 1-[4-(N-3,4-dimethylbenzoyl-sulfamoyl)-phenyl]-3-methylurea according to claim 1.

20. 1-[4-(N-3,4-dimethylbenzoyl-sulfamoyl)-phenyl]-3,3-dimethylurea according to claim 1.

21. 1-[4-(N-3,4-dimethylbenzoyl-sulfamoyl)-phenyl]-3-ethylurea according to claim 1.

22. 1-[4-(N-3,4-dimethylbenzoyl-sulfamoyl)-phenyl]-3-allylurea according to claim 1.

23. 1-[4-(N-3,4-dimethylbenzoyl-sulfamoyl)-phenyl]-3-phenylurea according to claim 1.

24. 1-[4-(N-3,5-dimethylbenzoyl-sulfamoyl)-phenyl]-3-methylurea according to claim 1.

25. 1-[4-(N-3,4-dichlorobenzoyl-sulfamoyl)-phenyl]-3-methylurea according to claim 1.

26. 1-[4-(N-3,4-dimethoxybenzoyl-sulfamoyl)-phenyl]-3-methylurea according to claim 1.

27. 1-[4-(N-3,4-dimethoxybenzoyl-sulfamoyl)-phenyl]-3,3-dimethylurea according to claim 1.

28. 1-[4-(N-2,4,5-trimethoxybenzoyl-sulfamoyl)-phenyl]-3-methylurea according to claim 1.

29. 1-[4-(N-1-naphthylcarbonyl-sulfamoyl)-phenyl]-3-methylurea according to claim 1.

30. 1-[4-(N-2-furylcarbonyl-sulfamoyl)-phenyl]-3-methylurea according to claim 1.

31. 1-[4-(N-2-furylcarbonyl-sulfamoyl)-phenyl]-3,3-dimethylurea according to claim 1.

32. 1-[4-(N-2-thienylcarbonyl-sulfamoyl)-phenyl]-3-methylurea according to claim 1.

33. 1-[4-(N-piperonyloyl-sulfamoyl)-phenyl]-3-methylurea according to claim 1.

34. 1-[4-(N-3-methylbenzoyl-sulfamoyl)-phenyl]-3,3-dimethylurea according to claim 1.

35. 1-[4-(N-3-trifluoromethylbenzoyl-sulfamoyl)-phenyl]-3-cyclopropylurea according to claim 1.

36. 1-[3-(N-3,4-dimethylbenzoyl-sulfamoyl)-phenyl]-3-methylurea according to claim 1.

37. 1-[3-(N-2-furylcarbonyl-sulfamoyl)-phenyl]-3,3-dimethylurea according to claim 1.

38. A composition for protecting cultivated plants from the phytotoxic action of sulfonylurea herbicides, chloroacetanilide herbicides, acylcyclohexanedione herbicides or aryloxyphenoxypropionic acid herbicides, which composition comprises a compound of formula I according to claim 1.

39. A composition for protecting cultivated plants from the phytotoxic action of sulfonylurea herbicides, chloroacetanilide herbicides or aryloxyphenoxypropionic acid herbicides, which composition comprises a compound of formula I according to claim 1.

40. A composition according to claim 39 wherein the cultivated plants are rice, maize, sorghum, cereals or soybeans.

41. A composition according to claim 40 wherein the cultivated plants are rice, maize or sorghum.

42. A composition according to claim 39 wherein the cultivated plants are sorghum and the herbicides are sulfonylureas.

43. A composition according to claim 39, wherein it is used for protecting cultivated plants from the phytotoxic action of sulfonylurea herbicides of formula II

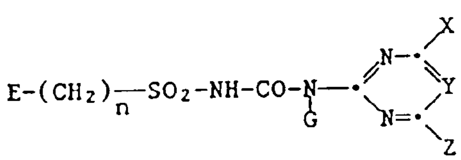

(II)

wherein E is a group

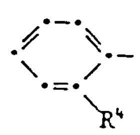 , 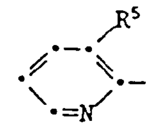 , 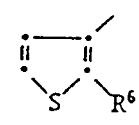 ,

or

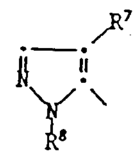 ,

n is 0 or 1, G is hydrogen or methyl, X is methoxy, ethoxy, difluoromethoxy, methyl or chlorine, Y is CH or N, Z is methoxy, methyl, difluoromethoxy, cyclopropyl or methylamino, $R^4$ is $C_2$-$C_5$alkoxyalkoxy, $C_1$-$C_4$haloalkoxy, $C_1$-$C_4$haloalkylthio, $C_2$-$C_4$haloalkenyl, chlorine or $C_1$-$C_4$-alkoxycarbonyl, $R^5$ is trifluoromethyl or di($C_1$-$C_4$alkyl)-carbamoyl, $R^6$ is $C_1$-$C_4$alkoxycarbonyl, $R^7$ is $C_1$-$C_4$alkoxycarbonyl, and $R^8$ is $C_1$-$C_4$alkyl.

**44.** A composition according to claim 43 wherein the sulfonylureas of formula II are selected from the group:
N-(3-trifluoromethylpyridin-2-ylsulfonyl)-N′-(4,6-dimethoxypyrimidin-2-yl)-urea, N-(3-dimethylcarbamoylpyridin-2-ylsulfonyl)-N′-(4,6-dimethoxypyrimidin-2-yl)-urea, N-(1-methyl-4-ethoxycarbonylpyrazol-2-ylsulfonyl)-N′-(4,6-dimethoxypyrimidin-2-yl)-urea, N-(2-methoxycarbonylthien-3-ylsulfonyl)-N′-(4-methoxy-6-methyl-1,3,5-triazin-2-yl)-urea, N-(2-methoxycarbonylbenzylsulfonyl)-N′-(4,6-dimethoxypyrimidin-2-yl)-urea, N-(2-methoxycarbonylphenylsulfonyl)-N′-(4,6-bis-difluoromethoxy-pyrimidin-2-yl)-urea, N-(2-methoxycarbonylphenylsulfonyl)-N′-(4-ethoxy-6-methylamino-1,3,5-triazin-2-yl)-urea, N-(2-methoxycarbonylphenylsulfonyl)-N′-(4-methoxy-6-methyl-1,3,5-triazin-2-yl)-urea, N-(2-ethoxycarbonylphenylsulfonyl)-N′-(4-chloro-6-methoxypyrimidin-2-yl)-urea, N-(2-methoxycarbonylphenylsulfonyl)-N′-(4-methoxy-6-methyl-1,3,5-triazin-2-yl)-N′-methylurea, N-(2-methoxycarbonylphenylsulfonyl)-N′-(4,6-dimethoxypyrimidin-2-yl)-urea, N-(2-chlorophenylsulfonyl)-N′-(4-methoxy-6-methyl-1,3,5-triazin-2-yl)-urea, N-[2-(2-chloroethoxy)-phenylsulfonyl]-N′-(4-methoxy-6-methyl-1,3,5-triazin-2-yl)-urea and N-[2-(2-methoxyethoxy)-phenylsulfonyl]-N′-(4,6-dimethoxy-1,3,5-triazin-2-yl)-urea.

**45.** A composition according to claim 39, wherein it is used for protecting cultivated plants from the phytotoxic action of chloroacetanilide herbicides of formula III

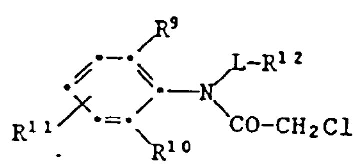 (III)

wherein L is a $C_1$-$C_4$alkylene bridge, each of $R^9$, $R^{10}$ and $R^{11}$, independently of the others, is hydrogen, halogen, $C_1$-$C_4$alkyl, $C_1$-$C_4$alkoxy, $C_1$-$C_4$haloalkyl, $C_2$-$C_5$alkoxyalkyl or $C_2$-$C_5$alkylthioalkyl, and $R^{12}$ is $C_1$-$C_4$alkoxy, -COOH, $C_1$-$C_4$alkoxycarbonyl, -$CONH_2$, $C_1$-$C_4$alkylcarbamoyl, di-$C_1$-$C_4$alkylcarbamoyl, cyano, $C_1$-$C_4$alkylcarbonyl, unsubstituted or substituted benzoyl, unsubstituted or substituted furyl, unsubstituted or substituted thienyl, unsubstituted or substituted pyrrolyl, unsubstituted or substituted pyrazolyl, unsubstituted or substituted 1,3,4-oxadiazol-2-yl, unsubstituted or substituted 1,3,4-thiadiazol-2-yl, unsubstituted or substituted 1,2,4-triazol-3-yl, unsubstituted or substituted dioxolanyl, unsubstituted or substituted dioxanyl, unsubstituted or substituted 1,3,4-triazol-2-yl or unsubstituted or substituted tetrahydrofuryl.

**46.** A composition according to claim 45 wherein the chloroacetanilides of formula III are selected from the group:
N-ethoxymethyl-N-chloroacetyl-2-ethyl-6-methylaniline, N-chloroacetyl-N-methoxymethyl-2,6-diethylaniline, N-chloroacetyl-N-(2-methoxyethyl)-2,6-dimethylaniline, N-chloroacetyl-N-(2-n-propoxyethyl)-2,6-dimethylaniline, N-chloroacetyl-N-(2-isopropoxyethyl)-2,6-dimethylaniline, N-chloroacetyl-N-(2-methoxyethyl)-2-ethyl-6-methylaniline, N-chloroacetyl-N-(methoxyethyl)-2,6-diethylaniline, N-(2-ethoxyethyl)-N-chloroacetyl-2-ethyl-6-methylaniline, N-chloroacetyl-N-(2-methoxy-1-methylethyl)-2-methylaniline, N-chloroacetyl-N-(2-methoxy-1-methylethyl)-2,6-dimethylaniline, N-chloroacetyl-N-(2-methoxy-1-methylethyl)-2,6-diethylaniline, N-chloroacetyl-N-(2-methoxy-1-methylethyl)-2-ethyl-6-methylaniline, N-(2-ethoxyethyl)-N-chloroacetyl-2,6-diethylaniline, N-chloroacetyl-N-(2-n-propoxyethyl)-2-ethyl-6-methylaniline, N-chloroacetyl-N-(2-n-propoxyethyl)-2,6-diethylaniline, N-chloroacetyl-N-(2-isopropoxyethyl)-2-ethyl-6-methylaniline, N-ethoxycarbonylmethyl-N-chloroacetyl-2,6-dimethylaniline, N-ethoxycarbonylmethyl-N-chloroacetyl-2,6-diethylaniline, N-chloroacetyl-N-methoxycarbonylmethyl-2,6-dimethylaniline, N-chloroacetyl-N-(2,2-diethoxyethyl)-2,6-dimethylaniline, N-chloroacetyl-N-(2-methoxy-1-methylethyl)-2,3-dimethylaniline, N-(2-ethoxyethyl)-N-chloroacetyl-2-methylaniline, N-chloroacetyl-N-(2-methoxyethyl)-2-methylaniline, N-chloroacetyl-N-(2-methoxy-2-methylethyl)-2,6-dimethylaniline, N-(2-ethoxy-2-methylethyl)-N-chloroacetyl-2-ethyl-6-methylaniline, N-chloroacetyl-N-(1-ethyl-1-methoxyethyl)-2,6-dimethylaniline, N-chloroacetyl-N-(2-methoxyethyl)-2-methoxy-6-methylaniline, N-n-butoxymethyl-N-chloroacetyl-2-tert-butylaniline, N-(2-ethoxyethyl-2-methylethyl)-2,6-dimethylaniline, N-chloroacetyl-N-(2-methoxyethyl)-2-chloro-6-methylaniline, N-(2-ethoxyethyl)-N-chloroacetyl-2-chloro-6-methylaniline, N-(2-ethoxyethyl)-N-chloroacetyl-2,3,6-trimethylaniline, N-chloroacetyl-1-(2-methoxyethyl)-2,3,6-trimethylaniline, N-chloroacetyl-N-cyanomethyl-2,6-dimethylaniline, N-chloroacetyl-N-(1,3-dioxolan-2-ylmethyl)-2,6-dimethylaniline, N-chloroacetyl-N-(1,3-dioxolan-2-ylmethyl)-2-ethyl-6-methylaniline, N-chloroacetyl-N-(1,3-dioxan-2-ylmethyl)-2-ethyl-6-methylaniline, N-chloroacetyl-N-(2-furylmethyl)-2,6-dimethylaniline, N-chloroacetyl-N-(2-furylmethyl)-2-ethyl-6-methylaniline, N-chloroacetyl-N-(2-tetrahydrofurylmethyl)-2,6-dimethylaniline, N-chloroacetyl-N-(N,N-dimethylcarbamoylmethyl)-2,6-dimethylaniline, N-(n-butoxymethyl)-N-chloroacetyl-2,6-diethylaniline, N-(2-n-butoxyethyl)-N-chloroacetyl-2,6-diethylaniline, N-chloroacetyl-N-(2-methoxy-1,2-dimethylethyl)-2,6-dimethylaniline, N-chloroacetyl-N-isopropyl-2,3-dimethylaniline, N-chloroacetyl-N-isopropyl-2-chloroaniline, N-chloroacetyl-N-(1H-pyrazol-1-ylmethyl)-2,6-dimethylaniline, N-chloroacetyl-N-(1H-pyrazol-1-ylmethyl)-2-ethyl-6-methylaniline, N-chloroacetyl-N-(1H-1,2,4-triazol-1-ylmethyl)-2,6-dimethylaniline, N-chloroacetyl-N-(1H-1,2,4-triazol-1-ylmethyl)-2,6-diethylaniline, N-benzoylmethyl-N-chloroacetyl-2,6-diethylaniline, N-benzoylmethyl-N-chloroacetyl-2-ethyl-6-methylaniline, N-chloroacetyl-N-(5-methyl-1,3,4-oxadiazol-2-yl)-2,6-diethylaniline, N-chloroacetyl-N-(5-methyl-1,3,4-oxadiazol-2-yl)-2-ethyl-6-methylaniline, N-chloroacetyl-N-(5-methyl-1,3,4-oxadiazol-2-yl)-2-tert-butylaniline, N-chloroacetyl-N-(4-chlorobenzoylmethyl)2,6-dimethylaniline and N-chloroacetyl-N-(1-methyl-5-methylthio-1,3,4-triazol-2-ylmethyl)-2,6-diethylaniline.

**47.** A composition according to claim 39, wherein it is used to protect cultivated plants from the phytotoxic action of aryloxyphenoxypropionic acid herbicides of formula IV

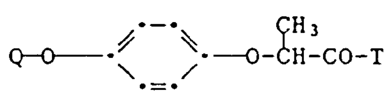

(IV)

wherein Q is a radical

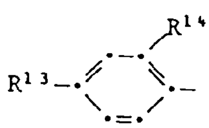 , 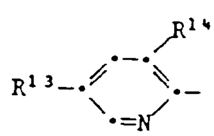 , 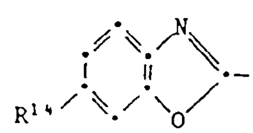 ,

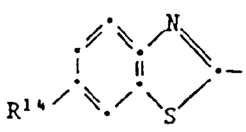 or 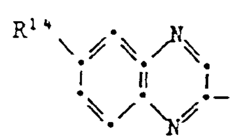

and T is $-NR^{15}R^{16}$, $-N(CN)R^{17}$, $-OR^{18}$, $SR^{18}$ or $-O-N=CR^{19}R^{20}$, wherein $R^{13}$ is halogen or trifluoromethyl, $R^{14}$ is hydrogen or halogen, each of $R^{15}$ and $R^{16}$, independently of the other, is hydrogen, $C_1$-$C_8$alkoxy, $C_1$-$C_8$-alkyl, phenyl or benzyl, $R^{15}$ and $R^{16}$ together with the nitrogen atom carrying them form a 5- or 6-membered saturated nitrogen heterocycle that may be interrupted by an oxygen or a sulfur atom, $R^{17}$ is $C_1$-$C_4$alkyl, $C_3$-$C_4$-alkenyl, $C_3$-$C_4$alkynyl or $C_2$-$C_4$alkoxyalkyl, $R^{18}$ is hydrogen or the equivalent of an alkali metal, alkaline earth metal, copper or iron ion; a quaternary $C_1$-$C_4$alkylammonium or $C_1$-$C_4$hydroxyalkylammonium radical; a $C_1$-$C_9$-alkyl radical that is unsubstituted or mono- or poly-substituted by amino, halogen, hydroxy, cyano, nitro, phenyl, $C_1$-$C_4$alkoxy, polyethoxy having from 2 to 6 ethylene oxide units, -COOR$^{21}$, -COSR$^{21}$, -CONH$_2$, -CON-($C_1$-$C_4$alkoxy)-$C_1$-$C_4$alkyl, -CO-N-di-$C_1$-$C_4$alkyl, CONH-$C_1$-$C_4$alkyl, -N($C_1$-$C_4$alkoxy)-$C_1$-$C_4$alkyl or by di-$C_1$-$C_4$-alkylamino; a $C_3$-$C_9$alkenyl radical that is unsubstituted or substituted by halogen or by $C_1$-$C_4$alkoxy; a $C_3$-$C_9$alkynyl radical that is unsubstituted or substituted by halogen or by $C_1$-$C_4$alkoxy; $C_3$-$C_9$cycloalkyl; or phenyl that is unsubstituted or substituted by cyano, $C_1$-$C_4$alkyl, $C_1$-$C_4$alkoxy, acetyl, -COOR$^{21}$, COSR$^{21}$, -CONH$_2$, -CON-($C_1$-$C_4$alkoxy)-$C_1$-$C_4$alkyl, -CO-N-di-$C_1$-$C_4$alkyl or by -CONH-$C_1$-$C_4$alkyl, each of $R^{19}$ and $R^{20}$, independently of the other, is $C_1$-$C_4$alkyl, or together they form a 3- to 6-membered alkylene chain, and $R^{21}$ is hydrogen, $C_1$-$C_6$-alkyl, $C_1$-$C_6$haloalkyl, $C_2$-$C_6$alkoxyalkyl, $C_3$-$C_6$alkenyl, $C_3$-$C_6$haloalkyl, $C_3$-$C_6$alkynyl or $C_3$-$C_6$haloalkynyl.

**48.** A composition according to claim 47 wherein the aryloxyphenoxypropionic acid derivatives of formula IV are selected from the group:
2-[4-(3,5-dichloropyridin-2-yloxy)-phenoxy]-propionic acid propargyl ester, 2-[4-(3,5-dichloropyridin-2-yloxy)-phenoxy]-thiopropionic acid propargyl ester, 2-[4-(5-chloro-3-fluoropyridin-2-yloxy)-phenoxy]-propionic acid methyl ester, 2-[4-(5-chloro-3-fluoropyridin-2-yloxy)-phenoxy]-propionic acid butyl ester, 2-[4-(5-trifluoromethylpyridin-2-yloxy)-phenoxy]-propionic acid butyl ester, 2-[4-(5-trifluoromethylpyridin-2-yloxy)-phenoxy]-propionic acid methyl ester, 2-[4-(6-chloroquinoxalin-2-yloxy)-phenoxy]-propionic acid ethyl ester and 2-[4-(6-chlorobenzoxazolin-2-yloxy)-phenoxy]-propionic acid ethyl ester.

**49.** A composition according to claim 38, wherein it is used for protecting cultivated plants from the phytotoxic action of acylcyclohexanedione herbicides of formula IX

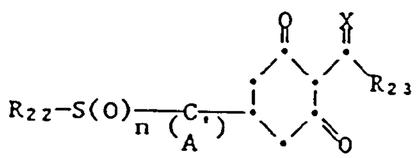

(IX)

wherein A′ is a 2- to 7-membered alkylene bridge, a 3- to 7-membered alkenylene bridge which can be mono- or poly-unsaturated, n′ is 0, 1 or 2, $R_{22}$ is $C_1$-$C_4$alkyl or benzyl, $R_{23}$ is $C_1$-$C_6$alkyl unsubstituted or substituted by halogen, $C_1$-$C_4$alkoxy or by $C_1$-$C_4$alkylthio; $C_3$-$C_6$cycloalkyl; phenyl, benzyl or phenylethyl wherein the phenyl ring can be substituted by halogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, cyano or by nitro, X′ is oxygen or a radical -NOR$_{24}$, and $R_{24}$ is $C_1$-$C_4$alkyl, $C_1$-$C_6$haloalkyl, $C_3$-$C_6$alkenyl, $C_3$-$C_6$haloalkenyl or $C_3$-$C_6$alkynyl.

**50.** A composition according to claim 49 wherein the acylcyclohexanedione herbicides are selected from the group:
5-(1-methylthiocyclobutan-1-yl)-2-(2,4-dichlorobenzoyl)-cyclohexane-1,3-dione, 5-(1-methylthiocyclobutan-1-yl)-2-n-butyryl-cyclohexane-1,3-dione, 5-(1-methylthiocyclobutan-1-yl)-2-cyclopropylcarbonyl-cyclohexane-1,3-dione, 5-(1-methylthiocyclobutan-1-yl)-2-(2,3-dichlorobenzoyl)-cyclohexane-1,3-dione, 5-(1-methylsulfonylcyclobutan-1-yl)-2-n-butyryl-cyclohexane-1,3-dione, 5-(1-methylthiocyclopropan-1-yl)-2-propionyl-cyclohexane-1,3-dione, 5-(1-ethylthiocyclopropan-1-yl)-2-propionyl-cyclohexane-1,3-dione and 5-(1-methylthiocyclopropan-1-yl)-2-n-butyril-cyclohexane-1,3-dione, 5-(1-methylthiocyclobutan-1-yl)-2-(1-ethoxyimino-n-butyryl)-cyclohexane-1,3-dione, 5-(1-methylthiocyclopropan-1-yl)-2-(1-ethoxyimino-n-butyryl)-cyclohexane-1,3-dione, 5-(1-methylthiocyclohexan-1-yl)-2-(1-ethoxyimino-n-butyryl)-cyclohexane-1,3-dione, 5-(1-methylthiocyclobutan-1-yl)-2-(1-allyloxyimino-n-butyryl)-cyclohexane-1,3-dione and 5-(1-methylthiocyclopentan-1-yl)-2-(1-ethoxyimino-n-butyryl)-cyclohexane-1,3-dione, 5-(1-methylthiocyclopropan-1-yl)-2-[1-(trans-3-chloroallyloxyimino)-n-butyryl]-cyclohexane-1,3-dione, 5-(1-methylthiocyclopropan-1-yl)-2-[1-trans-3-chloroallyloxyimino)-propionyl]-cyclohexane-1,3-dione, 5-(1-methylthiocyclopropan-1-yl)-2-[1-(cis-3-chlorollyloxyimino)-propionyl]-cyclohexane-1,3-dione and 5-(1-ethylthiocyclopropan-1-yl)-2-[1-(trans-3-chloroallyloxyimino)-propionyl]-cyclohexane-1,3-dione.

**51.** The use of sulfamoylphenylureas of formula I according to claim 1 for protecting cultivated plants against

the harmful effect of sulfonylurea herbicides, chloroacetanilide herbicides, acylcyclohexanedione herbicides or aryloxyphenoxypropionic acid herbicides.

**52.** The use of sulfamoylphenylureas of formula I according to claim 1 for protecting cultivated plants against the harmful effect of sulfonylurea herbicides, chloroacetanilide herbicides or aryloxyphenoxypropionic acid herbicides.

**53.** The use according to claim 52 wherein the sulfonylurea herbicides are compounds of formula II

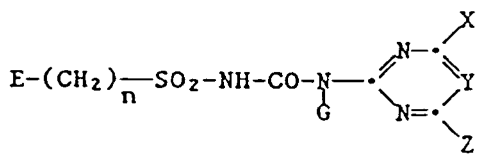

(II)

wherein E is a group

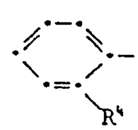

,

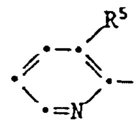

,

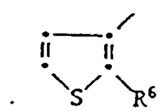

,

or

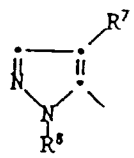

,

n is 0 or 1, G is hydrogen or methyl, X is methoxy, ethoxy, difluoromethoxy, methyl or chlorine, Y is CH or N, Z is methoxy, methyl, difluoromethoxy, cyclopropyl or methylamino, $R^4$ is $C_2$-$C_5$alkoxyalkoxy, $C_1$-$C_4$haloalkoxy, $C_1$-$C_4$haloalkylthio, $C_2$-$C_4$haloalkenyl, chlorine or $C_1$-$C_4$- alkoxycarbonyl, $R^5$ is trifluoromethyl or di($C_1$-$C_4$alkyl)-carbamoyl, $R^6$ is $C_1$-$C_4$alkoxycarbonyl, $R^7$ is $C_1$-$C_4$alkoxycarbonyl, and $R^8$ is $C_1$-$C_4$alkyl.

**54.** The use according to claim 52 wherein the chloroacetanilide herbicides are compounds of formula III

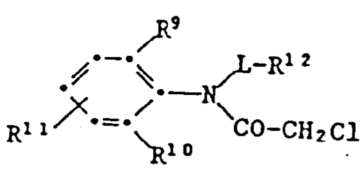

(III)

wherein L is a $C_1$-$C_4$alkylene bridge, each of $R^9$, $R^{10}$ and $R^{11}$, independently of the others, is hydrogen, halogen, $C_1$-$C_4$alkyl, $C_1$-$C_4$alkoxy, $C_1$-$C_4$haloalkyl, $C_2$-$C_5$alkoxyalkyl or $C_2$-$C_5$alkylthioalkyl, and $R^{12}$ is $C_1$-$C_4$alkoxy, -COOH, $C_1$-$C_4$alkoxycarbonyl, $-CONH_2$, $C_1$-$C_4$alkylcarbamoyl, di-$C_1$-$C_4$alkylcarbamoyl, cyano, $C_1$-$C_4$alkylcarbonyl, unsubstituted or substituted benzoyl, unsubstituted or substituted furyl, unsubstituted or substituted thienyl, unsubstituted or substituted pyrrolyl, unsubstituted or substituted pyrazolyl, unsubstituted or substituted 1,3,4-oxadiazol-2-yl, unsubstituted or substituted 1,3,4-thiadiazol-2-yl, unsubstituted or substituted 1,2,4-triazol-3-yl, unsubstituted or substituted dioxolanyl, unsubstituted or substituted dioxanyl, unsubstituted or substituted 1,3,4-triazol-2-yl or unsubstituted or substituted tetrahydrofuryl.

**55.** The use according to claim 52 wherein the aryloxyphenoxypropionic acid herbicides are compounds of formula IV

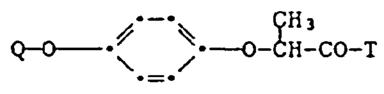

(IV)

wherein Q is a radical

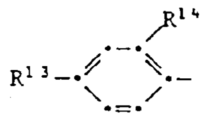 , 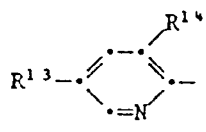 , 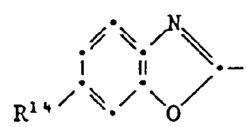 ,

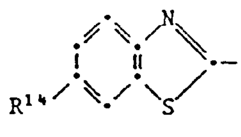 or 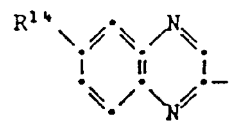

and T is $-NR^{15}R^{16}$, $-N(CN)R^{17}$, $-OR^{18}$, $SR^{18}$ or $-O-N=CR^{19}R^{20}$, wherein $R^{13}$ is halogen or trifluoromethyl, $R^{14}$ is hydrogen or halogen, each of $R^{15}$ and $R^{16}$, independently of the other, is hydrogen, $C_1$-$C_8$alkoxy, $C_1$-$C_8$alkyl, phenyl or benzyl, $R^{15}$ and $R^{16}$ together with the nitrogen atom carrying them form a 5- or 6-membered saturated nitrogen heterocycle that may be interrupted by an oxygen or a sulfur atom, $R^{17}$ is $C_1$-$C_4$alkyl, $C_3$-$C_4$alkenyl, $C_3$-$C_4$alkynyl or $C_2$-$C_4$alkoxyalkyl, $R^{18}$ is hydrogen or the equivalent of an alkali metal, alkaline earth metal, copper or iron ion; a quaternary $C_1$-$C_4$alkylammonium or $C_1$-$C_4$hydroxyalkylammonium radical; a $C_1$-$C_9$alkyl radical that is unsubstituted or mono- or poly-substituted by amino, halogen, hydroxy, cyano, nitro, phenyl, $C_1$-$C_4$alkoxy, polyethoxy having from 2 to 6 ethylene oxide units, $-COOR^{21}$, $-COSR^{21}$, $-CONH_2$, -CON-($C_1$-$C_4$alkoxy)-$C_1$-$C_4$alkyl, -CO-N-di-$C_1$-$C_4$alkyl, CONH-$C_1$-$C_4$alkyl, -N($C_1$-$C_4$alkoxy)-$C_1$-$C_4$alkyl or by di-$C_1$-$C_4$-alkylamino; a $C_3$-$C_9$alkenyl radical that is unsubstituted or substituted by halogen or by $C_1$-$C_4$alkoxy; a $C_3$-$C_9$alkynyl radical that is unsubstituted or substituted by halogen or by $C_1$-$C_4$alkoxy; $C_3$-$C_9$cycloalkyl; or phenyl that is unsubstituted or substituted by cyano, $C_1$-$C_4$alkyl, $C_1$-$C_4$alkoxy, acetyl, $-COOR^{21}$, $COSR^{21}$, $-CONH_2$, -CON($C_1$-$C_4$alkoxy)-$C_1$-$C_4$alkyl, -CO-N-di-$C_1$-$C_4$alkyl or by -CONH-$C_1$-$C_4$alkyl, each of $R^{19}$ and $R^{20}$, independently of the other, is $C_1$-$C_4$alkyl, or together they form a 3- to 6-membered alkylene chain, and $R^{21}$ is hydrogen, $C_1$-$C_6$-alkyl, $C_1$-$C_6$haloalkyl, $C_2$-$C_6$alkoxyalkyl, $C_3$-$C_6$alkenyl, $C_3$-$C_6$haloalkyl, $C_3$-$C_6$alkynyl or $C_3$-$C_6$haloalkynyl.

**56.** The use according to claim 51 wherein the acylcyclohexanedione herbicides are compounds of formula IX

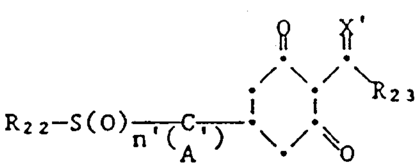

(IX)

wherein A′ is a 2- to 7-membered alkylene bridge, a 3- to 7-membered alkenylene bridge which can be mono- or polyunsaturated, <u>n</u>′ is 0, 1 or 2, $R_{22}$ is $C_1$-$C_4$alkyl or benzyl, $R_{23}$ is $C_1$-$C_6$alkyl unsubstituted or substituted by halogen, $C_1$-$C_4$alkoxy or by $C_1$-$C_4$alkylthio; $C_3$-$C_6$cycloalkyl; phenyl, benzyl or phenylethyl wherein the phenyl ring can be substituted by halogen, $C_1$-$C_4$alkyl, $C_1$-$C_4$-alkoxy, $C_1$-$C_4$alkylthio, $C_1$-$C_4$haloalkyl, $C_1$-$C_4$haloalkoxy, cyano or by nitro, X′ is oxygen or a radical $-NOR_{24}$, and $R_{24}$ is $C_1$-$C_6$alkyl, $C_1$-$C_6$haloalkyl, $C_3$-$C_6$alkenyl, $C_3$-$C_6$-haloalkenyl or $C_3$-$C_6$alkynyl.

**57.** A herbicidal composition for selectively controlling weeds in crops of useful plants, which comprises as active ingredients both a sulfonylurea herbicide, an acylcyclohexanedione herbicide, a chloroacetanilide herbicide or an aryloxyphenoxypropionic acid herbicide and a counter-agent of formula I according to claim 1.

**58.** A herbicidal composition for selectively controlling weeds in crops of useful plants, which comprises as active ingredients both a sulfonylurea herbicide, a chloroacetanilide herbicide or an aryloxyphenoxypropionic acid herbicide and a counter-agent of formula I according to claim 1.

**59.** A composition according to claim 57 wherein the acylcyclohexanedione herbicide is a compound of formula IX

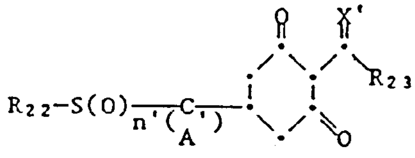

(IX)

wherein A′ is a 2- to 7-membered alkylene bridge, a 3- to 7-membered alkenylene bridge which can be mono- or polyunsaturated, n′ is 0, 1 or 2, $R_{22}$ is $C_1$-$C_4$alkyl or benzyl, $R_{23}$ is $C_1$-$C_6$alkyl unsubstituted or substituted by halogen, $C_1$-$C_4$alkoxy or by $C_1$-$C_4$alkylthio; $C_3$-$C_6$cycloalkyl; phenyl, benzyl or phenylethyl wherein the phenyl ring can be substituted by halogen, $C_1$-$C_4$alkyl, $C_1$-$C_4$-alkoxy, $C_1$-$C_4$alkylthio, $C_1$-$C_4$haloalkyl, $C_1$-$C_4$haloalkoxy, cyano or by nitro, X′ is oxygen or a radical $-NOR_{24}$, and $R_{24}$ is $C_1$-$C_6$alkyl, $C_1$-$C_6$haloalkyl, $C_3$-$C_6$alkenyl, $C_3$-$C_6$-haloalkenyl or $C_3$-$C_6$alkynyl.

**60.** A composition according to claim 58 wherein the sulfonylurea herbicide is a compound of formula II

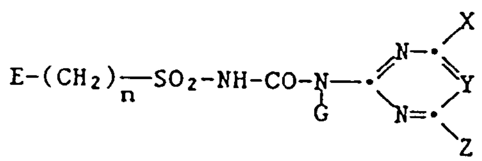

(II)

wherein E is a group

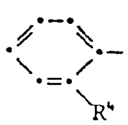 , 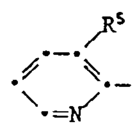 , 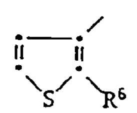 ,

or

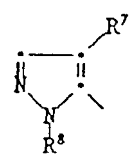 ,

n is 0 or 1, G is hydrogen or methyl, X is methoxy, ethoxy, difluoromethoxy, methyl or chlorine, Y is CH or N, Z is methoxy, methyl, difluoromethoxy, cyclopropyl or methylamino, $R^4$ is $C_2$-$C_5$alkoxyalkoxy, $C_1$-$C_4$haloalkoxy, $C_1$-$C_4$haloalkylthio, $C_2$-$C_4$haloalkenyl, chlorine or $C_1$-$C_4$-alkoxycarbonyl, $R^5$ is trifluoromethyl or di($C_1$-$C_4$alkyl)-carbamoyl, $R^6$ is $C_1$-$C_4$alkoxycarbonyl, $R^7$ is $C_1$-$C_4$alkoxy-carbonyl, and $R^8$ is $C_1$-$C_4$alkyl.

**61.** A composition according to claim 58 wherein the chloroacetanilide herbicide is a compound of formula III

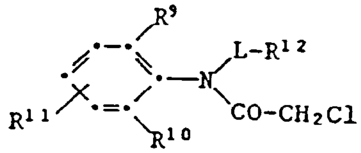

(III)

wherein L is a $C_1$-$C_4$alkylene bridge, each of $R^9$, $R^{10}$ and $R^{11}$, independently of the others, is hydrogen, halogen, $C_1$-$C_4$alkyl, $C_1$-$C_4$alkoxy, $C_1$-$C_4$haloalkyl, $C_2$-$C_5$alkoxyalkyl or $C_2$-$C_5$alkylthioalkyl, and $R^{12}$ is $C_1$-$C_4$alkoxy, -COOH, $C_1$-$C_4$alkoxycarbonyl, -$CONH_2$, $C_1$-$C_4$alkylcarbamoyl, di-$C_1$-$C_4$alkylcarbamoyl, cyano, $C_1$-$C_4$alkylcarbonyl, unsubstituted or substituted benzoyl, unsubstituted or substituted furyl, unsubstituted or substituted thienyl, unsubstituted or substituted pyrrolyl, unsubstituted or substituted pyrazolyl, unsubstituted or substituted 1,3,4-oxadiazol-2-yl, unsubstituted or substituted 1,3,4-thiadiazol-2-yl, unsubstituted or substituted 1,2,4-triazol-3-yl, unsubstituted or substituted dioxolanyl, unsubstituted or substituted dioxanyl, unsubstituted or substituted 1,3,4-triazol-2-yl or unsubstituted or substituted tetrahydrofuryl.

62. A composition according to claim 58 wherein the aryloxyphenoxypropionic acid herbicide is a compound of formula IV

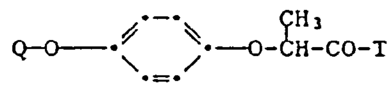

(IV)

wherein Q is a radical

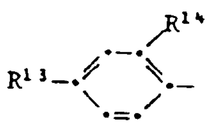

,

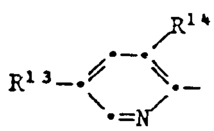

,

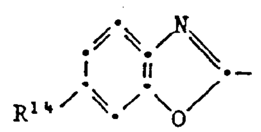

,

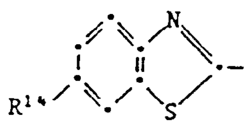

or

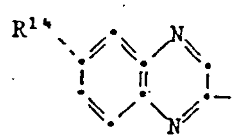

and T is -$NR^{15}R^{16}$, -N(CN)$R^{17}$, -$OR^{18}$, $SR^{18}$ or -O-N=$CR^{19}R^{20}$, wherein $R^{13}$ is halogen or trifluoromethyl, $R^{14}$ is hydrogen or halogen, each of $R^{15}$ and $R^{16}$, independently of the other, is hydrogen, $C_1$-$C_8$alkoxy, $C_1$-$C_8$-alkyl, phenyl or benzyl, $R^{15}$ and $R^{16}$ together with the nitrogen atom carrying them form a 5- or 6-membered saturated nitrogen heterocycle that may be interrupted by an oxygen or a sulfur atom, $R^{17}$ is $C_1$-$C_4$alkyl, $C_3$-$C_4$-alkenyl, $C_3$-$C_4$alkynyl or $C_2$-$C_4$alkoxyalkyl, $R^{18}$ is hydrogen or the equivalent of an alkali metal, alkaline earth metal, copper or iron ion; a quaternary $C_1$-$C_4$alkyl-ammonium or $C_1$-$C_4$hydroxyalkylammonium radical; a $C_1$-$C_9$-alkyl radical that is unsubstituted or mono- or poly-substituted by amino, halogen, hydroxy, cyano, nitro, phenyl, $C_1$-$C_4$alkoxy, polyethoxy having from 2 to 6 ethylene oxide units, -$COOR^{21}$, -$COSR^{21}$, -$CONH_2$, -CON-($C_1$-$C_4$alkoxy)-$C_1$-$C_4$alkyl, -CO-N-di-$C_1$-$C_4$alkyl, CONH-$C_1$-$C_4$alkyl, -N($C_1$-$C_4$alkoxy)-$C_1$-$C_4$alkyl or by di-$C_1$-$C_4$-alkylamino; a $C_3$-$C_9$alkenyl radical that is unsubstituted or substituted by halogen or by $C_1$-$C_4$alkoxy; a $C_3$-$C_9$-alkynyl radical that is unsubstituted or substituted by halogen or by $C_1$-$C_4$alkoxy; $C_3$-$C_9$cycloalkyl; or phenyl that is unsubstituted or substituted by cyano, $C_1$-$C_4$alkyl, $C_1$-$C_4$alkoxy, acetyl, -$COOR^{21}$, $COSR^{21}$, -$CONH_2$, CON-($C_1$-$C_4$alkoxy)-$C_1$-$C_4$alkyl, -CO-N-di-$C_1$-$C_4$alkyl or by -CONH-$C_1$-$C_4$alkyl, each of $R^{19}$ and $R^{20}$, independently of the other, is $C_1$-$C_4$alkyl, or together they form a 3- to 6-membered alkylene chain, and $R^{21}$ is hydrogen, $C_1$-$C_6$-alkyl, $C_1$-$C_6$haloalkyl, $C_2$-$C_6$alkoxyalkyl, $C_3$-$C_6$alkenyl, $C_3$-$C_6$haloalkyl, $C_3$-$C_6$alkynyl or $C_3$-$C_6$haloalkynyl.

63. A method of selectively controlling weeds in crops of useful plants wherein the cultivated plants or the cultivation area thereof is treated both with a sulfonylurea herbicide, an acylcyclohexanedione herbicide, a chloroacetanilide herbicide or an aryloxyphenoxypropionic acid herbicide and with an effective amount

of an N-acylsulfamoylphenylurea of formula I according to claim 1 as counter-agent.

**64.** A method of protecting cultivated plants from damage that occurs when sulfonylurea herbicides, acylcyclohexanedione herbicides, chloroacetanilide herbicides or aryloxyphenoxypropionic acid herbicides are applied, which method comprises treating either the cultivation area of the plants before or during application of the herbicide or treating the seeds or the cuttings of the plants or the plants themselves with an effective amount of an N-acylsulfamoylphenylurea of formula I according to claim 1.

**65.** A method of selectively controlling weeds in crops of useful plants wherein the cultivated plants or the cultivation area thereof is treated both with a sulfonylurea herbicide, a chloroacetanilide herbicide or an aryloxyphenoxypropionic acid herbicide and with an effective amount of an N-acylsulfamoylphenylurea of formula I according to claim 1 as counter-agent.

**66.** A method of protecting cultivated plants from damage that occurs when sulfonylurea herbicides, chloroacetanilide herbicides or aryloxyphenoxypropionic acid herbicides are applied, which method comprises treating either the cultivation area of the plants before or during application of the herbicide or treating the seeds or the cuttings of the plants or the plants themselves with an effective amount of an N-acylsulfamoylphenylurea of formula I according to claim 1.

**67.** Seeds of useful plants, which have been treated with an antagonistically effective amount of an N-acylsulfamoylphenylurea of formula I according to claim 1.

**68.** Seeds according to claim 67 wherein the seeds are seeds of cereals, soybeans, rice, maize or sorghum.

**69.** Seeds according to claim 67 wherein the seeds are seeds of sorghum, maize or rice.

**70.** A process for the preparation of a sulfamoylphenylurea of formula I wherein $R^1$ is as defined under formula I in claim 1 with the exception of hydrogen and $R^2$ is hydrogen, in which process a sulfamoylaniline of formula V

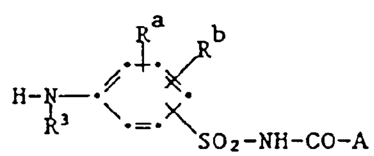

(V),

wherein A, $R^3$, $R^a$ and $R^b$ are as defined under formula I, is reacted with an isocyanate of formula VI

$R^1$-N=C=O (VI)

wherein $R^1$ is as defined under formula I with the exception of hydrogen.

**71.** A process for the preparation of a compound of formula I according to claim 1, in which process a sulfamoylphenylurea of formula VII

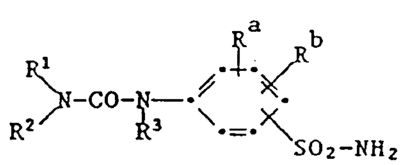

(VII),

wherein $R^1$, $R^2$, $R^3$, $R^a$ and $R^b$ are as defined under formula I, is acylated with a carboxylic acid halide of formula VIII

Hal-CO-A (VIII)

wherein A is as defined under formula I and Hal is chlorine or bromine.

**72.** 1-[4-(N-2-ethoxybenzoyl-sulfamoyl)-phenyl]-3-methylurea.

**Claims for the following Contracting State: ES**

**1.** A process for the preparation of a compound of formula I

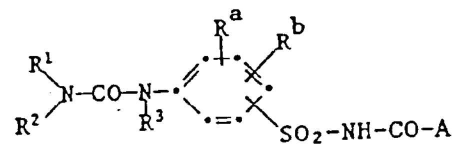 (I),

wherein A is a radical selected from the group

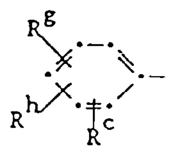 , 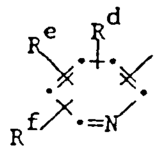 , 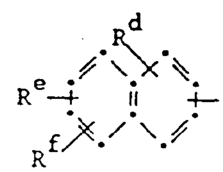 ,

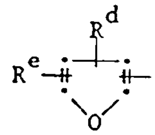 . or 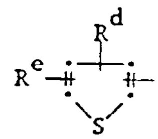 ,

each of $R^1$ and $R^2$, independently of the other, is hydrogen, $C_1$-$C_8$alkyl, $C_3$-$C_8$cycloalkyl, $C_3$-$C_6$alkenyl, $C_3$-$C_6$-alkynyl,

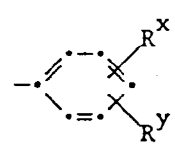

or $C_1$-$C_4$alkyl substituted by $C_1$-$C_4$-alkoxy or by

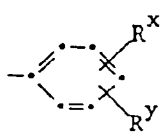 ,

or $R^1$ and $R^2$ together form a $C_4$-$C_6$alkylene bridge, or a $C_4$-$C_6$alkylene bridge interrupted by oxygen, sulfur, SO, $SO_2$, NH or by -N($C_1$-$C_4$alkyl)-, $R^3$ is hydrogen or $C_1$-$C_4$-alkyl, each of $R^a$ and $R^b$, independently of the other, is hydrogen, halogen, cyano, nitro, trifluoromethyl, $C_1$-$C_4$-alkyl, $C_1$-$C_4$alkoxy, $C_1$-$C_4$alkylthio, $C_1$-$C_4$alkylsulfinyl, $C_1$-$C_4$alkylsulfonyl, -COOR$^j$, -CONR$^k$R$^m$, -COR$^n$, -SO$_2$NR$^k$R$^m$ or -OSO$_2$-$C_1$-$C_4$alkyl, or $R^a$ and $R^b$ together form a $C_3$-$C_4$-alkylene bridge, which can be substituted by halogen or by $C_1$-$C_4$alkyl, or a $C_3$-$C_4$alkenylene bridge, which can be substituted by halogen or by $C_3$-$C_4$alkyl, or a $C_4$alkadienylene bridge which can be substituted by halogen or by $C_1$-$C_4$alkyl, and each of $R^g$ and $R^h$, independently of the other, is hydrogen, halogen, $C_1$-$C_4$alkyl, trifluoromethyl, methoxy, methylthio or -COOR$^j$, wherein $R^c$ is hydrogen, halogen, $C_1$-$C_4$alkyl or methoxy, $R^d$ is hydrogen, halogen, nitro, $C_1$-$C_4$alkyl, $C_1$-$C_4$alkoxy, $C_1$-$C_4$alkylthio, $C_1$-$C_4$alkylsulfinyl, $C_1$-$C_4$alkylsulfonyl, -COOR$^j$ or -CONR$^k$R$^m$, $R^e$ is hydrogen, halogen, $C_1$-$C_4$alkyl, -COOR$^j$, trifluoromethyl or methoxy, or $R^d$ and $R^e$ together form a $C_3$-$C_4$alkylene bridge, $R^f$ is hydrogen, halogen or $C_1$-$C_4$-alkyl, each of $R^x$ and $R^y$, independently of the other, is hydrogen, halogen, $C_1$-$C_4$alkyl, $C_1$-$C_4$alkoxy, $C_1$-$C_4$alkylthio, -COOR$^4$, trifluoromethyl, nitro or cyano, each of $R^j$, $R^k$ and $R^m$, independently of the others, is hydrogen or $C_1$-$C_4$alkyl, $R^k$ and $R^m$ together form a $C_4$-$C_6$alkylene bridge, or a $C_4$-$C_6$alkylene bridge interrupted by oxygen, NH or by -N($C_1$-$C_4$alkyl)-, and $R^n$ is $C_1$-$C_4$alkyl, phenyl, or phenyl substituted by halogen, $C_1$-$C_4$alkyl, methoxy, nitro or by trifluoromethyl, in which process a sulfamoylphenylurea of formula VII

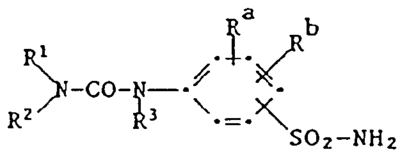

(VII),

wherein $R^1$, $R^2$, $R^3$, $R^a$ and $R^b$ are as defined under formula 1, is acylated with a carboxylic acid halide of formula VIII

Hal-CO-A (VIII)

wherein A is as defined under formula I and Hal is chlorine or bromine.

2. A process according to claim 1 for the preparation of a compound of formula I wherein A is a radical selected from the group

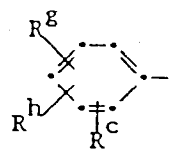 , 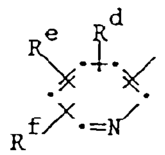 , 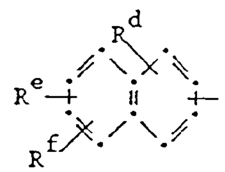 ,

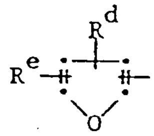 or 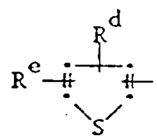 ,

each of $R^1$ and $R^2$, independently of the other, is hydrogen, $C_1$-$C_8$alkyl, $C_3$-$C_8$cycloalkyl, $C_3$-$C_6$alkenyl, $C_3$-$C_6$alkynyl,

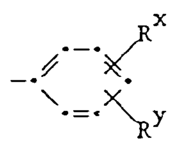

or $C_1$-$C_4$alkyl substituted by

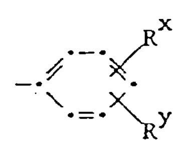 ,

or $R^1$ and $R^2$ together form a $C_4$-$C_6$alkylene bridge, or a $C_4$-$C_6$alkylene bridge interrupted by oxygen, sulfur, SO, $SO_2$, NH or by -N($C_1$-$C_4$alkyl)-, $R^3$ is hydrogen or $C_1$-$C_4$-alkyl, each of $R^a$ and $R^b$, independently of the other, is hydrogen, halogen, cyano, nitro, trifluoromethyl, $C_1$-$C_4$-alkyl, $C_1$-$C_4$alkoxy, $C_1$-$C_4$alkylthio, $C_1$-$C_4$alkylsulfinyl, $C_1$-$C_4$alkylsulfonyl, $-COOR^j$, $-CONR^kR^m$, $-COR^n$, $-SO_2NR^kR^m$ or $-OSO_2$-$C_1$-$C_4$alkyl, and each of $R^g$ and $R^h$, independently of the other, is hydrogen, halogen, $C_1$-$C_4$alkyl, trifluoromethyl, methoxy, methylthio or $-COOR^j$, wherein $R^c$ is hydrogen, halogen, $C_1$-$C_4$alkyl or methoxy, $R^d$ is hydrogen, halogen, nitro, $C_1$-$C_4$alkyl, $C_1$-$C_4$alkoxy, $C_1$-$C_4$alkylthio, $C_1$-$C_4$alkylsulfinyl, $C_1$-$C_4$-alkylsulfonyl, $-COOR^j$ or - $CONR^k R^m$, $R^e$ is hydrogen, halogen, $C_1$-$C_4$alkyl, $-COOR^j$, trifluoromethyl or methoxy, or $R^d$ and $R^e$ together form a $C_3$-$C_4$alkylene bridge, $R^f$ is hydrogen, halogen or $C_1$-$C_4$-alkyl, each of $R^x$ and $R^y$, independently of the other, is hydrogen, halogen, $C_1$-$C_4$alkyl, $C_1$-$C_4$alkoxy, $C_1$-$C_4$alkylthio, $-COOR^4$, trifluoromethyl, nitro or cyano, each of $R^j$, $R^k$ and $R^m$, independently of the others, is hydrogen or $C_1$-$C_4$alkyl, $R^k$ and $R^m$ together form a $C_4$-$C_6$alkylene bridge, or a $C_4$-$C_6$alkylene bridge interrupted by oxygen, NH or by -N($C_1$-$C_4$alkyl)-, and $R^n$ is $C_1$-$C_4$alkyl, phenyl, or phenyl substituted by halogen, $C_1$-$C_4$alkyl, methoxy, nitro or by trifluoromethyl.

3. A process according to claim 1 for the preparation of a compound of formula I wherein $R^b$ is hydrogen.

4. A process according to claim 1 for the preparation of a compound of formula I wherein the sulfamoyl group occupies the 4-position of the phenyl ring.

5. A process according to claim 1 for the preparation of a compound of formula I wherein $R^2$ and $R^3$ are hydrogen.

6. A process according to claim 1 for the preparation of a compound of formula I wherein $R^b$ is hydrogen and the sulfamoyl group occupies the 4-position of the phenyl ring.

7. A process according to claim 1 for the preparation of a compound of formula I wherein $R^2$, $R^3$ and $R^b$ are hydrogen and the sulfamoyl group occupies the 4-position of the phenyl ring.

8. A process according to claim 1 for the preparation of a compound of formula I wherein A is the group

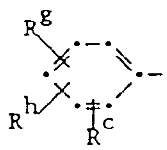

.

9. A process according to claim 8 for the preparation of a compound of formula I wherein $R^2$, $R^3$ and $R^b$ are hydrogen and the sulfamoyl group occupies the 4-position of the phenyl ring.

10. A process according to claim 1 for the preparation of a compound of formula I falling within the scope of sub-formula Ia

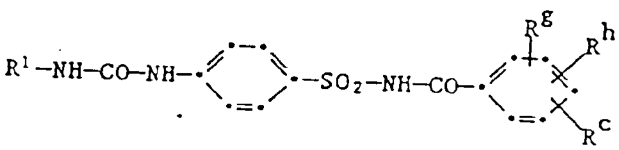

(Ia).

11. A process according to claim 10 for the preparation of a compound of sub-formula Ia wherein $R^c$ is hydrogen.

12. A process according to claim 11 for the preparation of a compound of sub-formula I wherein $R^1$, $R^g$ and $R^h$ are $C_1$-$C_4$alkyl.

13. A process according to claim 1 for the preparation of 1-[4-(N-4-methylbenzoyl-sulfamoyl)-phenyl]-3-methylurea.

14. A process according to claim 1 for the preparation of 1-[4-(N-3-methylbenzoyl-sulfamoyl)-phenyl]-3-methylurea.

15. A process according to claim 1 for the preparation of 1-[4-(N-4-tert-butylbenzoyl-sulfamoyl)-phenyl]-3-methylurea.

16. A process according to claim 1 for the preparation of 1-[4-(N-3-trifluoromethylbenzoyl-sulfamoyl)-phenyl]-3-methylurea.

17. A process according to claim 1 for the preparation of 1-[4-(N-4-nitrobenzoyl-sulfamoyl)-phenyl]-3-methylurea.

18. A process according to claim 1 for the preparation of 1-[4-(N-2,3-dimethylbenzoyl-sulfamoyl)-phenyl]-3-methylurea.

**19.** A process according to claim 1 for the preparation of 1-[4-(N-3,4-dimethylbenzoyl-sulfamoyl)-phenyl]-3-methylurea.

**20.** A process according to claim 1 for the preparation of 1-[4-(N-3,4-dimethylbenzoyl-sulfamoyl)-phenyl]-3,3-dimethylurea.

**21.** A process according to claim 1 for the preparation of 1-[4-(N-3,4-dimethylbenzoyl-sulfamoyl)-phenyl]-3-ethylurea.

**22.** A process according to claim 1 for the preparation of 1-[4-(N-3,4-dimethylbenzoyl-sulfamoyl)-phenyl]-3-allylurea.

**23.** A process according to claim 1 for the preparation of 1-[4-(N-3,4-dimethylbenzoyl-sulfamoyl)-phenyl]-3-phenylurea.

**24.** A process according to claim 1 for the preparation of 1-[4-(N-3,5-dimethylbenzoyl-sulfamoyl)-phenyl]-3-methylurea.

**25.** A process according to claim 1 for the preparation of 1-[4-(N-3,4-dichlorobenzoyl-sulfamoyl)-phenyl]-3-methylurea.

**26.** A process according to claim 1 for the preparation of 1-[4-(N-3,4-dimethoxybenzoyl-sulfamoyl)-phenyl]-3-methylurea.

**27.** A process according to claim 1 for the preparation of 1-[4-(N-3,4-dimethoxybenzoyl-sulfamoyl)-phenyl]-3,3-dimethylurea.

**28.** A process according to claim 1 for the preparation of 1-[4-(N-2,4,5-trimethoxybenzoyl-sulfamoyl)-phenyl]-3-methylurea.

**29.** A process according to claim 1 for the preparation of 1-[4-(N-1-naphthylcarbonyl-sulfamoyl)-phenyl]-3-methylurea.

**30.** A process according to claim 1 for the preparation of 1-[4-(N-2-furylcarbonyl-sulfamoyl)-phenyl]-3-methylurea.

**31.** A process according to claim 1 for the preparation of 1-[4-(N-2-furylcarbonyl-sulfamoyl)-phenyl]-3,3-dimethylurea.

**32.** A process according to claim 1 for the preparation of 1-[4-(N-2-thienylcarbonyl-sulfamoyl)-phenyl]-3-methylurea.

**33.** A process according to claim 1 for the preparation of 1-[4-(N-piperonyloyl-sulfamoyl)-phenyl]-3-methylurea.

**34.** A process according to claim 1 for the preparation of 1-[4-(N-3-methylbenzoyl-sulfamoyl)-phenyl]-3,3-dimethylurea.

**35.** A process according to claim 1 for the preparation of 1-[4-(N-3-trifluoromethylbenzoyl-sulfamoyl)-phenyl]-3-cyclopropylurea.

**36.** A process according to claim 1 for the preparation of 1-[3-(N-3,4-dimethylbenzoyl-sulfamoyl)-phenyl]-3-methylurea.

**37.** A process according to claim 1 for the preparation of 1-[3-(N-2-furylcarbonyl-sulfamoyl)-phenyl]-3,3-dimethylurea.

**38.** The use of sulfamoylphenylureas of formula I according to claim 1 for protecting cultivated plants against the damaging action of sulfonylurea herbicides, chloroacetanilide herbicides, acylcyclohexanedione herbicides or aryloxyphenoxypropionic acid herbicides.

**39.** Use of sulfamoylphenylureas of formula I according to claim 1 for protecting cultivated plants against the damaging action of sulfonylurea herbicides, chloroacetanilide herbicides or aryloxyphenoxypropionic acid herbicides.

**40.** Use according to claim 39, wherein the sulfonylurea herbicides are compounds of formula II

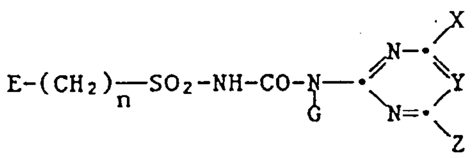

(II)

wherein E is a group

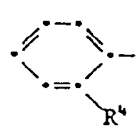
,
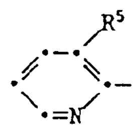
,
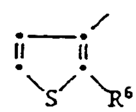
,

or

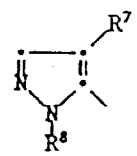
,

$\underline{n}$ is 0 or 1, G is hydrogen or methyl, X is methoxy, ethoxy, difluoromethoxy, methyl or chlorine, Y is CH or N, $\underline{Z}$ is methoxy, methyl, difluoromethoxy, cyclopropyl or methylamino, $R^4$ is $C_2$-$C_5$alkoxyalkoxy, $C_1$-$C_4$haloalkoxy, $C_1$-$C_4$haloalkylthio, $C_2$-$C_4$haloalkenyl, chlorine or $C_1$-$C_4$-alkoxycarbonyl, $R^5$ is trifluoromethyl or di($C_1$-$C_4$alkyl)-carbamoyl, $R^6$ is $C_1$-$C_4$alkoxycarbonyl, $R^7$ is $C_1$-$C_4$alkoxycarbonyl, and $R^8$ is $C_1$-$C_4$alkyl.

**41.** Use according to claim 39, wherein the chloroacetanilide herbicides are compounds of formula III

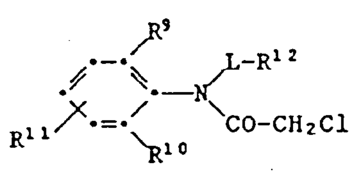

(III)

wherein L is a $C_1$-$C_4$alkylene bridge, each of $R^9$, $R^{10}$ and $R^{11}$, independently of the others, is hydrogen, halogen, $C_1$-$C_4$alkyl, $C_1$-$C_4$alkoxy, $C_1$-$C_4$haloalkyl, $C_2$-$C_5$alkoxyalkyl or $C_2$-$C_5$alkylthioalkyl, and $R^{12}$ is $C_1$-$C_4$alkoxy, -COOH, $C_1$-$C_4$alkoxycarbonyl, $-CONH_2$, $C_1$-$C_4$alkylcarbamoyl, di-$C_1$-$C_4$alkylcarbamoyl, cyano, $C_1$-$C_4$alkylcarbonyl, unsubstituted or substituted benzoyl, unsubstituted or substituted furyl, unsubstituted or substituted thienyl, unsubstituted or substituted pyrrolyl, unsubstituted or substituted pyrazolyl, unsubstituted or substituted 1,3,4-oxadiazol-2-yl, unsubstituted or substituted 1,3,4-thiadiazol-2-yl, unsubstituted or substituted 1,2,4-triazol-3-yl, unsubstituted or substituted dioxolanyl, unsubstituted or substituted dioxanyl, unsubstituted or substituted 1,3,4-triazol-2-yl or unsubstituted or substituted tetrahydrofuryl.

**42.** Use according to claim 39, wherein the aryloxyphenoxypropionic acid herbicides are compounds of formula IV

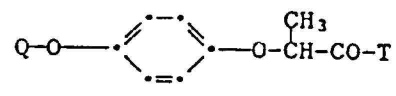

(IV)

wherein Q is a radical

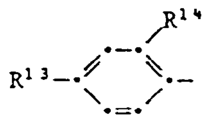 , 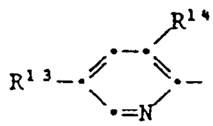 , 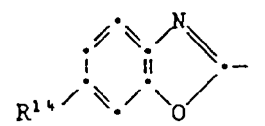 , 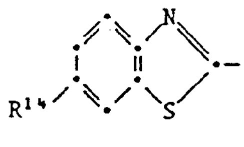 or 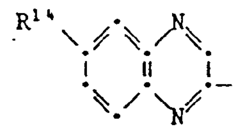

and T is $-NR^{15}R^{16}$, $-N(CN)R^{17}$, $-OR^{18}$, $SR^{18}$ or $-O-N=CR^{19}R^{20}$, wherein $R^{13}$ is halogen or trifluoromethyl, $R^{14}$ is hydrogen or halogen, each of $R^{15}$ and $R^{16}$, independently of the other, is hydrogen, $C_1$-$C_8$alkoxy, $C_1$-$C_8$-alkyl, phenyl or benzyl, $R^{15}$ and $R^{16}$ together with the nitrogen atom carrying them form a 5- or 6-membered saturated nitrogen heterocycle that may be interrupted by an oxygen or a sulfur atom, $R^{17}$ is $C_1$-$C_4$alkyl, $C_3$-$C_4$-alkenyl, $C_3$-$C_4$alkynyl or $C_2$-$C_4$alkoxyalkyl, $R^{18}$ is hydrogen or the equivalent of an alkali metal, alkaline earth metal, copper or iron ion; a quaternary $C_1$-$C_4$alkylammonium or $C_1$-$C_4$hydroxyalkylammonium radical; a $C_1$-$C_9$-alkyl radical that is unsubstituted or mono- or poly-substituted by amino, halogen, hydroxy, cyano, nitro, phenyl, $C_1$-$C_4$alkoxy, polyethoxy having from 2 to 6 ethylene oxide units, $-COOR^{21}$, $-COSR^{21}$, $-CONH_2$, -CON-($C_1$-$C_4$alkoxy)-$C_1$-$C_4$alkyl, -CO-N-di-$C_1$-$C_4$alkyl, CONH-$C_1$-$C_4$alkyl, -N($C_1$-$C_4$alkoxy)-$C_1$-$C_4$alkyl or by di-$C_1$-$C_4$-alkylamino; a $C_3$-$C_9$alkenyl radical that is unsubstituted or substituted by halogen or by $C_1$-$C_4$alkoxy; a $C_3$-$C_9$-alkynyl radical that is unsubstituted or substituted by halogen or by $C_1$-$C_4$alkoxy; $C_3$-$C_9$cycloalkyl; or phenyl that is unsubstituted or substituted by cyano, $C_1$-$C_4$-alkyl, $C_1$-$C_4$alkoxy, acetyl, $-COOR^{21}$, $COSR^{21}$, $-CONH_2$, -CON($C_1$-$C_4$alkoxy)-$C_1$-$C_4$alkyl, -CO-N-di-$C_1$-$C_4$alkyl or by -CONH-$C_1$-$C_4$alkyl, each of $R^{19}$ and $R^{20}$, independently of the other, is $C_1$-$C_4$alkyl, or together they form a 3- to 6-membered alkylene chain, and $R^{21}$ is hydrogen, $C_1$-$C_6$-alkyl, $C_1$-$C_6$haloalkyl, $C_2$-$C_6$alkoxyalkyl, $C_3$-$C_6$alkenyl, $C_3$-$C_6$haloalkyl, $C_3$-$C_6$alkynyl or $C_3$-$C_6$haloalkynyl.

**43.** Use according to claim 38, wherein the acylcyclohexanedione herbicides are compounds of formula IX

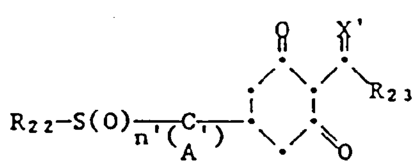

(IX)

wherein A′ is a 2- to 7-membered alkylene bridge, a 3- to 7-membered alkenylene bridge which can be mono- or polyunsaturated, n′ is 0, 1 or 2, $R_{22}$ is $C_1$-$C_4$alkyl or benzyl, $R_{23}$ is $C_1$-$C_6$alkyl unsubstituted or substituted by halogen, $C_1$-$C_4$alkoxy or by $C_1$-$C_4$alkylthio; $C_3$-$C_6$cycloalkyl; phenyl, benzyl or phenylethyl wherein the phenyl ring can be substituted by halogen, $C_1$-$C_4$alkyl, $C_1$-$C_4$-alkoxy, $C_1$-$C_4$alkylthio, $C_1$-$C_4$haloalkyl, $C_1$-$C_4$haloalkoxy, cyano or by nitro, X′ is oxygen or a radical $-NOR_{24}$, and $R_{24}$ is $C_1$-$C_6$alkyl, $C_1$-$C_6$haloalkyl, $C_3$-$C_6$alkenyl, $C_3$-$C_6$- haloalkenyl or $C_3$-$C_6$alkynyl.

**44.** A process according to claim 1 for the preparation of 1-[4-(N-2-ethoxybenzoyl-sulfamoyl)-phenyl]-3-methylurea.

## Revendications

### Revendications pour les Etats contractants suivants: AT, BE, CH, DE, FR, GB, GR, IT, LI, LU, NL, SE

**1.** N-acylsulfamoylphénylurées de formule I

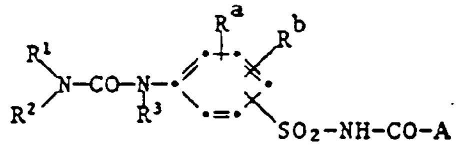

(I)

où
A représente un reste provenant du groupe

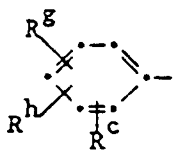 , 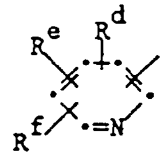 , 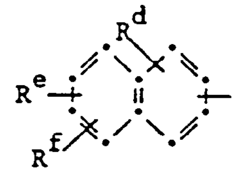 ,

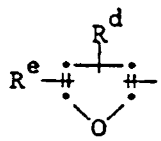 ou 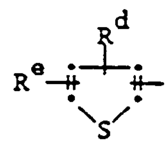 ,

$R^1$ et $R^2$ représentent, indépendamment l'un de l'autre, l'hydrogène, un alkyle en $C_1$-$C_8$, un cycloalkyle en $C_3$-$C_8$, un alcényle en $C_3$-$C_8$, un alcynyle en $C_3$-$C_6$,

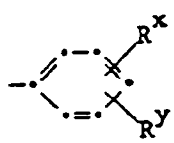

ou un alkyle en $C_1$-$C_4$ substitué par un alcoxy en $C_1$-$C_4$ ou par

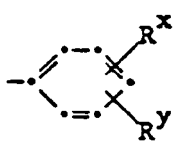

ou
$R^1$ et $R^2$ représentent ensemble un pont alkylène en $C_4$-$C_6$ ou un pont alkylène en $C_4$-$C_6$ interrompu par l'oxygène, le soufre, SO, $SO_2$, NH ou N-(alkyle en $C_1$-$C_4$),
$R^3$ représente l'hydrogène ou un alkyle en $C_1$-$C_4$,
$R^a$ et $R^b$ représentent, indépendamment l'un de l'autre, l'hydrogène, un halogène, un cyano, un nitro, le trifluorométhyle, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, --$COOR^j$, -$CONR^kR^m$, -$COR^m$, -$SO_2NR^kR^m$ ou -$OSO_2$ alkyle en $C_1$-$C_4$, ou $R^a$ et $R^b$ représentent ensemble un pont alkylène en $C_3$-$C_4$, pouvant être substitué par un halogène ou par un alkyle en $C_1$-$C_4$ ou un pont alcénylène en $C_3$-$C_4$ pouvant être substitué par un halogène ou par un alkyle en $C_1$-$C_4$ ou un pont alcadiénylène en $C_4$ pouvant être substitué par un halogène ou par un alkyle en $C_1$-$C_4$ et
$R^g$ et $R^h$ représentent, indépendamment l'un de l'autre, l'hydrogène, un halogène, un alkyle en $C_1$-$C_4$, le trifluorométhyle, le méthoxy, le méthylthio ou --$COOR^j$,

$R^c$ représentant l'hydrogène, un halogène, un alkyle en $C_1$-$C_4$ ou le méthoxy,
$R^d$ représentant l'hydrogène, un halogène, le nitro, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, -COOR$^j$ ou -CONR$^k$R$^m$,
$R^e$ représentant l'hydrogène, un halogène, un alkyle en $C_1$-$C_4$, -COOR$^j$, le trifluorométhyle ou le méthoxy ou R et $R^e$ représentant ensemble un pont alkylène en $C_3$-$C_4$,
$R^f$ représentant l'hydrogène, un halogène ou un alkyle en $C_1$-$C_4$,
$R^x$ et $R^y$ représentant, indépendamment l'un de l'autre, l'hydrogène, un halogène, un alkyle en $C_1$-$C_4$, un alcoxy en $C_1$-$C_4$, un alkyle en $C_1$-$C_4$ thio, -COOR$^4$, le trifluorométhyle, le nitro ou le cyano,
$R^j$, $R^k$ et $R^m$ représentant, indépendamment les uns des autres, l'hydrogène ou un alkyle en $C_1$-$C_4$,
$R^k$ et $R^m$ représentant ensemble un pont alkylène en $C_4$-$C_6$ ou un pont alkylène en $C_4$-$C_6$ interrompu par l'oxygène, NH ou -N(alkyle en $C_1$-$C_4$) et
$R^n$ représentant un alkyle en $C_1$-$C_4$, le phényle ou le phényle substitué par un halogène, un alkyle en $C_1$-$C_4$, le méthoxy, le nitro ou le trifluorométhyle, ainsi que les composés
1-[4-(N-3-chlorométhylbenzoyl-sulfamoyl)-phényl]-3-méthyl-urée,
1-[4-(N-2-éthoxybenzoyl-sulfamoyl)-phényl]-3-méthyl-urée,
1-[4-(N-3-éthoxybenzoyl-sulfamoyl)-phényl]-3-méthyl-urée,
1-[4-(N-4-éthoxybenzoyl-sulfamoyl)-phényl]-3-méthyl-urée,
1-[4-(N-2-méthylsulfonylbenzoyl-sulfamoyl]-phényl-3-méthyl-urée,
1-[4-(N-2-nitrobenzoyl-sulfamoyl)-phényl]-3-méthyl-urée,
1-[4-(N-3-nitrobenzoyl-sulfamoyl)-phényl]-3-méthyl-urée,
1-[4-(N-4-nitrobenzoyl-sulfamoyl)-phényl]-3-méthyl-urée,
1-[4-(N-2-acétylbenzoyl-sulfamoyl)-phényl]-3-méthyl-urée,
1-[4-(N-4-acétylbenzoyl-sulfamoyl)-phényl]-3-méthyl-urée,
1-[4-(N-3-cyanobenzoyl-sulfamoyl)-phényl]-3-méthyl-urée,
1-[4-(N-4-cyanobenzoyl-sulfamoyl)-phényl]-3-méthyl-urée,
1-[4-(N-3,4-dinitrobenzoyl-sulfamoyl)-phényl]-3-méthyl-urée,
1-[4-(N-3,5-dinitrobenzoyl-sulfamoyl)-phényl]-3-méthyl-urée,
1-[4-(N-2-chloro-3-nitrobenzoyl-sulfamoyl)-phényl]-3-méthyl-urée,
1-[4-(N-2-chloro-4-nitrobenzoyl-sulfamoyl)-phényl]-3-méthyl-urée,
1-[4-(N-2-chloro-5-nitrobenzoyl-sulfamoyl)-phényl]-3-méthyl-urée,
1-[4-(N-3-chloro-2-nitrobenzoyl-sulfamoyl)-phényl]-3-méthyl-urée,
1-[4-(N-4-chloro-2-nitrobenzoyl-sulfamoyl)-phényl]-3-méthyl-urée,
1-[4-(N-3-chloro-3-nitrobenzoyl-sulfamoyl)-phényl]-3-méthyl-urée,
1-[4-(N-5-chloro-2-nitrobenzoyl-sulfamoyl)-phényl]-3-méthyl-urée,
1-[4-(N-2-méthyl-3-nitrobenzoyl-sulfamoyl)-phényl]-3-méthyl-urée,
1-[4-(N-2-méthyl-5-nitrobenzoyl-sulfamoyl)-phényl]-3-méthyl-urée,
1-[4-(N-2-méthyl-6-nitrobenzoyl-sulfamoyl)-phényl]-3-méthyl-urée,
1-[4-(N-3-méthyl-2-nitrobenzoyl-sulfamoyl)-phényl]-3-méthyl-urée,
1-[4-(N-3-méthyl-4-nitrobenzoyl-sulfamoyl)-phényl]-3-méthyl-urée,
1-[4-(N-4-méthyl-3-nitrobenzoyl-sulfamoyl)-phényl]-3-méthyl-urée,
1-[4-(N-5-méthyl-2-nitrobenzoyl-sulfamoyl)-phényl]-3-méthyl-urée, et
1-[4-(N-4-fluoro-3-nitrobenzoyl-sulfamoyl)-phényl]-3-méthyl-urée.

2. Composés de formule I selon la revendication 1, caractérisés en ce que A représente un reste provenant du groupe

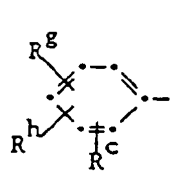 , 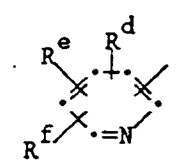 , 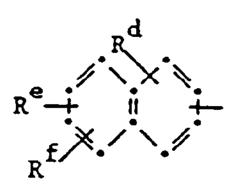 ,

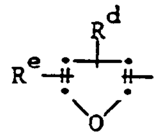 ou 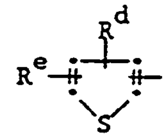 ,

$R^1$ et $R^2$ représentent, indépendamment l'un de l'autre, l'hydrogène, un alkyle en $C_1$-$C_8$, un cycloalkyle en $C_3$-$C_8$, un alcényle en $C_3$-$C_8$, un alcynyle en $C_3$-$C_6$

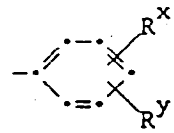

ou un alkyle en $C_1$-$C_4$ substitué par un alcoxy en $C_1$-$C_4$ ou par

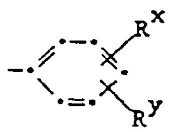

ou
$R^1$ et $R^2$ représentent ensemble un pont alkylène en $C_4$-$C_6$ ou un pont alkylène en $C_4$-$C_6$ interrompu par l'oxygène, le soufre, SO, $SO_2$, NH ou N-(alkyle en $C_1$-$C_4$),
$R^3$ représente l'hydrogène ou un alkyle en $C_1$-$C_4$,
$R^a$ et $R^b$ représentant, indépendamment l'un de l'autre, l'hydrogène, un halogène, un cyano, un nitro, le trifluorométhyle, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, --$COOR^j$, -$CONR^kR^m$, -$COR^m$, -$SO_2NR^kR^m$ ou -$OSO_2$ alkyle en $C_1$-$C_4$ et
$R^g$ et $R^h$ représentent, indépendamment l'un de l'autre, l'hydrogène, un halogène, un alkyle en $C_1$-$C_4$, le trifluorométhyle, le méthoxy, le méthylthio ou --$COOR^j$,
$R^c$ représentant l'hydrogène, un halogène, un alkyle en $C_1$-$C_4$ ou le méthoxy,
$R^d$ représentant l'hydrogène, un halogène, le nitro, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, -$COOR^j$ ou -$CONR^kR^m$,
$R^e$ représentant l'hydrogène, un halogène, un alkyle en $C_1$-$C_4$, -$COOR^j$, le trifluorométhyle ou le méthoxy ou $R^d$ et $R_e$ représentant ensemble un pont alkylène en $C_3$-$C_4$,
$R^f$ représentant l'hydrogène, un halogène ou un alkyle en $C_1$-$C_4$,
$R^x$ et $R^y$ représentant, indépendamment l'un de l'autre, l'hydrogène, un halogène, un alkyle en $C_1$-$C_4$, un alcoxy en $C_1$-$C_4$, un alkyle en $C_1$-$C_4$ thio, -$COOR^4$, le trifluorométhyle, le nitro ou le cyano, $R^j$, $R^k$ et $R^m$ représentant, indépendamment les uns des autres, l'hydrogène ou un alkyle en $C_1$-$C_4$,
$R^k$ et $R^m$ représentant ensemble un pont alkylène en $C_4$-$C_6$ ou un pont alkylène en $C_4$-$C_6$ interrompu par l'oxygène, NH ou -N(alkyle en $C_1$-$C_4$) et
$R^n$ représentant un alkyle en $C_1$-$C_4$, le phényle ou le phényle substitué par un halogène, un alkyle en $C_1$-$C_4$, le méthoxy, le nitro ou le trifluorométhyle.

3. Composés selon la revendication 1, caractérisés en ce que $R^b$ représente l'hydrogène.

4. Composés selon la revendication 1, caractérisés en ce que le groupe sulfamoyle occupe la position 4 sur le noyau phényle.

5. Composés selon la revendication 1, caractérisés en ce que $R^2$ et $R^3$ représentent l'hydrogène.

6. Composés selon la revendication 1, caractérisés en ce que $R^b$ représente l'hydrogène et le groupe sulfamoyle occupe la position 4 sur le noyau phényle.

7. Composés selon la revendication 1, caractérisés en ce que $R^2$, $R^3$ et $R^b$ représentent l'hydrogène et le groupe sulfamoyle occupe la position 4 sur le noyau phényle.

8. Composés selon la revendication 1, caractérisés en ce que A représente le groupe

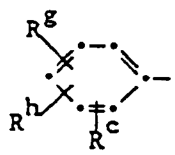

9. Composés selon la revendication 8, caractérisés en ce que $R^2$, $R^3$ et $R^b$ représentent l'hydrogène et le groupe sulfamoyle occupe la position 4 sur le noyau phényle.

10. Composés selon la revendication 1, caractérisés en ce qu'ils appartiennent à la sous-formule Ia

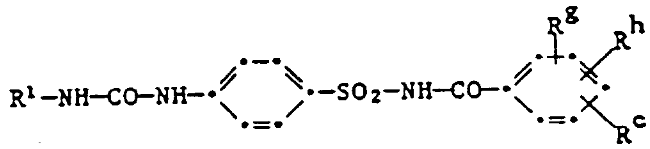

(Ia)

11. Composés selon la revendication 10, caractérisés en ce que $R^c$ représente l'hydrogène.

12. Composés selon la revendication 11, caractérisés en ce que $R^1$, $R^g$ et $R^h$ représentent un alkyle en $C_1$-$C_4$.

13. La 1-[4-(N-4-méthylbenzoyl-sulfamoyl)-phényl]-3-méthylurée selon la revendication 1.

14. La 1-[4-(N-3-méthylbenzoyl-sulfamoyl)-phényl]-3-méthylurée selon la revendication 1.

15. La 1-[4-(N-4-tert-butylbenzoyl-sulfamoyl)-phényl]-3-méthylurée selon la revendication 1.

16. La 1-[4-(N-3-trifluorométhylbenzoyl-sulfamoyl)-phényl]-3-méthylurée selon la revendication 1.

17. La 1-[4-(N-4-nitrobenzoyl-sulfamoyl)-phényl]-3-méthylurée selon la revendication 1.

18. La 1-[4-(N-2,3-diméthylbenzoyl-sulfamoyl)-phényl]-3-méthylurée selon la revendication 1.

19. La 1-[4-(N-3,4-diméthylbenzoyl-sulfamoyl)-phényl]-3-méthylurée selon la revendication 1.

20. La 1-[4-(N-3,4-diméthylbenzoyl-sulfamoyl)-phényl]-3,3-diméthylurée selon la revendication 1.

21. La 1-[4-(N-3,4-diméthylbenzoyl-sulfamoyl)-phényl]-3-éthylurée selon la revendication 1.

22. La 1-[4-(N-2,4-diméthylbenzoyl-sulfamoyl)-phényl]-3-allylurée selon la revendication 1.

23. La 1-[4-(N-3,4-diméthylbenzoyl-sulfamoyl)-phényl]-3-phénylurée selon la revendication 1.

24. La 1-[4-(N-3,5-diméthylbenzoyl-sulfamoyl)-phényl]-3-méthylurée selon la revendication 1.

25. La 1-[4-(N-3,4-dichlorobenzoyl-sulfamoyl)-phényl] -3-méthylurée selon la revendication 1.

26. La 1-[4-(N-3,4-diméthoxybenzoyl-sulfamoyl)-phényl]-3-méthylurée selon la revendication 1.

27. La 1-[4-(N-3,4-diméthoxybenzoyl-sulfamoyl)-phényl]-3,3-diméthylurée selon la revendication 1.

28. La 1-[4-(N-2,4,5-triméthoxybenzoyl-sulfamoyl)-phényl]-3-méthylurée selon la revendication 1.

29. La 1-[4-(N-1-naphtylcarbonyl-sulfamoyl)-phényl]-3-méthylurée selon la revendication 1.

30. La 1-[4-(N-2-furylcarbonyl-sulfamoyl)-phényl]-3-méthylurée selon la revendication 1.

31. La 1-[4-(N-2-furylcarbonyl-sulfamoyl)-phényl]-3,3-diméthylurée selon la revendication 1.

32. La 1-[4-(N-2-thiénylcarbonyl-sulfamoyl)-phényle]-3-méthylurée selon la revendication 1.

33. La 1-[4-(N-pipéronyloyl-sulfamoyl)-phényl]-3-méthylurée selon la revendication 1.

34. La 1-[4-(N-3-méthylbenzoyl-sulfamoyl)-phényl]-3,3-diméthylurée selon la revendication 1.

35. La 1-[4-(N-3-trifluorométhylbenzoyl-sulfamoyl)-phényl]-3-cyclopropylurée selon la revendication 1.

36. La 1-[3-(N-3,4-diméthylbenzoyl-sulfamoyl)-phényl]-3-méthylurée selon la revendication 1.

37. La 1- [3-(N-2-furylcarbonyl-sulfamoyl)-phényle]-3,3-diméthylurée selon la revendication 1.

38. Agent pour protéger les plantes cultivées, contre l'action phytotoxique des herbicides sulfonylurées, des herbicides chloroacétanilides, des herbicides acylcyclohexanediones ou des herbicides acides aryloxyphénoxypropioniques, caractérisé en ce qu'il contient une substance active de formule I selon la revendication 1.

39. Agent pour protéger les plantes cultivées contre l'action phytotoxique des herbicides sulfonylurées, des herbicides chloroacétanilides ou des herbicides acides acyloxyphénoxypropioniques, caractérisé en ce qu'il contient une substance active de formule I selon la revendication 1.

40. Agent selon la revendication 39, caractérisé en ce que les plantes cultivées sont le riz, le maïs, le sorgho, les céréales ou le soja.

41. Agent selon la revendication 40, caractérisé en ce que les plantes cultivées sont le riz, le maïs ou le sorgho.

42. Agent selon la revendication 39, caractérisé en ce que les plantes cultivées sont le sorgho et les herbicides sont les sulfonylurées.

43. Agent selon la revendication 39, caractérisé en ce qu'il est utilisé pour protéger les plantes cultivées contre l'action phytotoxique des herbicides sulfonylurées de formule II

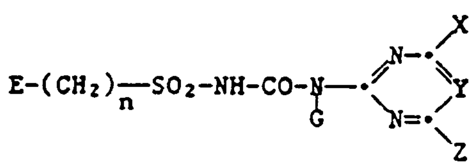

(II)

où
E représente un groupe

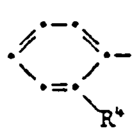
,
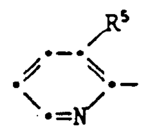
,
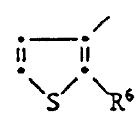
,

ou

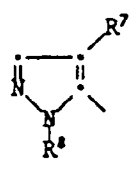
,

n un nombre égal à 0 ou 1,
G l'hydrogène ou le méthyle,
X le méthoxy, l'éthoxy, le difluorométhyle, le méthyle ou le chlore,
Y CH ou N,

Z le méthoxy, le méthyle, le difluorométhoxy, le cyclopropyle ou le méthylamino,

$R^4$ un alcoxy en $C_2$-$C_5$ alcoxy, un halogénoalcoxy en $C_1$-$C_4$, un halogénoalkyle en $C_1$-$C_4$ thio, un halogénoalcényle en $C_1$-$C_4$, le chlore ou un alcoxy en $C_1$-$C_4$ carbonyle,

$R^5$ le trifluorométhyle ou un di(alkyle en $C_1$-$C_4$)carbamoyle,

$R^6$ un alcoxy en $C_1$-$C_4$ carbonyle,

$R^7$ un alcoxy en $C_1$-$C_4$ carbonyle et

$R^8$ un alkyle en $C_1$-$C_4$.

**44.** Agent selon la revendication 43, caractérisé en ce qu'il est choisi dans le groupe constitué par
la N-(3-trifluorométhylpyridin-2-ylsulfonyl)-N'-(4,6-diméthoxypyrimidin-2-yl)-urée,
la N-(3-diméthylcarbamoylpyridin-2-ylsulfonyl)-N'-(4,6-diméthoxypyrimidin-2-yl)-urée,
la N-(1-méthyl-4-éthoxycarbonylpyrazol-2-ylsulfonyl)-N'-(4,6-diméthoxypyrimidin-2-yl)-urée,
la N-(2-méthoxycarbonylthién-3-ylsulfonyl)-N'-(4-méthoxy-6-méthyl-1,3,5-triazin-2-yl)-urée,
la N-(2-méthoxycarbonylbenzylsulfonyl)-N'-(4,6-diméthoxypyrimidin-2-yl)-urée,
la N-(2-méthoxycarbonylphénylsulfonyl)-N'-(4,6-bis-difluorométhoxypyrimidin-2-yl)-urée,
la N-(2-méthoxycarbonylphénylsulfonyl)-N'-(4-éthoxy-6-méthylamino-1,3,5-triazin-2-yl)-urée,
la N-(2-méthoxycarbonylphénylsulfonyl)-N'-(4-méthoxy-6-méthyl-1,3,5-triazin-2-yl)-urée,
la N-(2-éthoxycarbonylphénylsulfonyl)-N'-(4-chloro-6-méthoxypyrimidin-2-yl)-urée,
la N-(2-méthoxycarbonylphénylsulfonyl)-N'-(4-méthoxy-6-méthyl-1,3,5-triazin-2-yl)-N'-méthylurée,
la N-(2-méthoxycarbonylphénylsulfonyl)-N'-(4,6-diméthoxypyrimidin-2-yl)-urée,
la N-(2-chlorophénylsulfonyl)-N'-(4-méthoxy-6-méthyl-1,3,5-triazin-2-yl)-urée,
la N-[2-(2-chloroéthoxy)-phénylsulfonyl]-N'-(4-méthoxy-6-méthyl-1,3,(-triazin-2-yl)-urée et
la N-[2-(2-méthoxyéthoxy)-phénylsulfonyl]-N'-(4,6-diméthoxy-1,3,5-triazin-2-yl)-urée.

**45.** Agent selon la revendication 39, caractérisé en ce qu'il est utilisé pour protéger les plantes cultivées contre l'action phytotoxique des herbicides chloroacétanilides de formule III

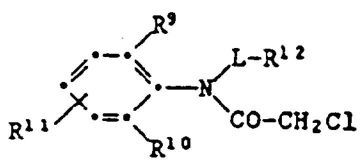

(III)

où

L représente un pont alkylène en $C_1$-$C_4$,

$R^9$, $R^{10}$ et $R^{11}$ représentent, indépendamment les uns des autres, l'hydrogène, un halogène, un alkyle en $C_1$-$C_4$, un alcoxy en $C_1$-$C_4$, un halogéno-alkyle en $C_1$-$C_4$, un alcoxy en $C_2$-$C_5$ alkyle ou un alkyle en $C_2$-$C_5$ thioalkyle et

$R^{12}$ représente un alcoxy en $C_1$-$C_4$, -COOH, un alcoxy en $C_1$-$C_4$ carbonyle, $-CONH_2$, un alkyle en $C_1$-$C_4$ carbamoyle, un dialkyle en $C_1$-$C_4$ carbamoyle,
le cyano, un alkyle en $C_1$-$C_4$ carbonyle,
le benzoyle éventuellement substitué,
le furyle éventuellement substitué,
le thiényle éventuellement substitué,
le pyrrolyle éventuellement substitué,
le pyrazolyle éventuellement substitué,
le 1,3,4-oxadiazol-2-yle éventuellement substitué,
le 1,3,4-thiadiazol-2-yle éventuellement substitué,
le 1,2,4-triazol-3-yle éventuellement substitué,
le dioxolanyle éventuellement substitué,
le dioxanyle éventuellement substitué,
le 1,3,4-triazol-2-yle éventuellement substitué ou
le tétrahydrofuryle éventuellement substitué.

**46.** Agent selon la revendication 45, caractérisé en ce que les chloroacétanilides de formule III sont choisis dans le groupe constitué par
la N-éthoxyméthyl-N-chloroacétyl-2-éthyl-6-méthylaniline,

la N-chloroacétyl-N-méthoxyméthyl-2,6-diéthylaniline,
la N-chloroacétyl-N-(2-méthoxyéthyl)-2,6-diméthylaniline,
la N-chloroacétyl-N-(2-n-propoxyéthyl)-2,6-diméthylaniline,
la N-chloroacétyl-N-(2-isopropoxyéthyl)-2,6-diméthylaniline,
la N-chloroacétyl-N-(2méthoxyéthyl)-2-éthyl-6-méthylaniline,
la N-chloroacétyl-N-(méthoxyéthyl)-2,6-diéthylaniline,
la N-(2-éthoxyéthyl)-N-chloroacétyl-2-éthyl-6-méthylaniline,
la N-chloroacétyl-N-(2-méthoxy-1-méthyléthyl)-2-méthylaniline,
la N-chloroacétyl-N-(2-méthoxy-1-méthyléthyl)-2,6-diméthylaniline,
la N-chloroacétyl-N-(2-méthoxy-1-méthyléthyl)-2,6-diéthylaniline,
la N-chloroacétyl-N-(2-méthoxy-1-méthyléthyl)-2-éthyl-6-méthylaniline,
la N-(2-éthoxyéthyl)-N-chloroacétyl-2,6-diéthylaniline,
la N-chloroacétyl-N-(2-n-propoxyéthyl)-2-éthyl-6-méthylaniline,
la N-chloroacétyl-N-(2-n-propoxyéthyl)-2,6-diéthylaniline,
la N-chloroacétyl-N-(2-isopropoxyéthyl)-2-éthyl-6-méthylaniline,
la N-éthoxycarbonylméthyl-N-chloroacétyl-2,6-diméthyl-aniline,
la N-éthoxycarbonylméthyl-N-chloroacétyl-2,6-diéthyl-aniline,
la N-chloroacétyl-N-méthoxycarbonylméthyl-2,6-diméthyl-aniline,
la N-chloroacétyl-N-(2,2-diéthoxyéthyl)-2,6-diméthyl-aniline,
la N-chloroacétyl-N-(2-méthoxy-1-méthyléthyl)-2,3-diméthylaniline,
la N-(2-éthoxyéthyl)-N-chloroacétyl-2-méthylaniline,
la N-chloroacétyl-N-(2-méthoxyéthyl)-2-méthylaniline,
la N-chloroacétyl-N-(2-méthoxy-2-méthyléthyl)-2,6-diméthylaniline,
la N-(2-éthoxy-2-méthyléthyl)-N-chloroacétyl-2-éthyl-6-méthylaniline,
la N-chloroacétyl-N-(1-éthyl-1-méthoxyéthyl)-2,6-diméthylaniline,
la N-chloroacétyl-N-(2-méthoxyéthyl)-2-méthoxy-6-méthylaniline,
la N-n-butoxyméthyl-N-chloroacétyl-2-tert-butylaniline,
la N-(2-éthoxyéthyl-2-méthyléthyl)-2,6-diméthylaniline,
la N-chloroacétyl-N-(2-méthoxyéthyl)-2-chloro-6-méthylaniline,
la N-(2-éthoxyéthyl)-N-chloroacétyl-2-chloro-6-méthylaniline,
la N-(2-éthoxyéthyl)-N-chloroacétyl-2,3,6-triméthylaniline,
la N-chloroacétyl-1-(2-méthoxyéthyl)-2,3,6-triméthylaniline,
la N-chloroacétyl-N-cyanométhyl-2,6-diméthylaniline,
la N-chloroacétyl-N-(1,3-dioxolan-2-ylméthyl)-2,6-diméthylaniline,
la N-chloroacétyl-N-(1,3-dioxolan-2-ylméthyl)-2-éthyl-6-méthylaniline,
la N-chloroacétyl-N-(1,3-dioxan-2-ylméthyl)-2-éthyl-6-méthylaniline,
la N-chloroacétyl-N-(2-furylméthyl)-2,6-diméthylaniline,
la N-chloroacétyl-N-(2-furylméthyl)-2-éthyl-6-méthylaniline,
la N-chloroacétyl-N-(2-tétrahydrofurylméthyl)-2,6-diméthylaniline,
la N-chloroacétyl-N-(N,N-diméthylcarbamoylméthyl)-2,6-diméthylaniline,
la N-(n-butoxyméthyl)-N-chloroacétyl-2,6-diéthylaniline,
la N-(2-n-butoxyéthyl)-N-chloroacétyl-2,6-diéthylaniline,
la N-chloroacétyl-N-(2-méthoxy-1,2-diméthyléthyl)-2,6-diméthylaniline,
la N-chloroacétyl-N-isopropyl-2,3-diméthylaniline,
la N-chloroacétyl-N-isopropyl-2-chloroaniline,
la N-chloroacétyl-N-(1H-pyrazol-1-ylméthyl)-2,6-diméthylaniline,
la N-chloroacétyl-N-(1H-pyrazol-1-ylméthyl)-2-éthyl-6-méthylaniline,
la N-chloroacétyl-N-(1H-1,2,4-triazol-1-ylméthyl)-2,6-diméthylaniline,
la N-chloroacétyl-N-(1H-1,2,4-triazol-1-ylméthyl)-2,6-diéthylaniline,
la N-benzoylméthyl-N-chloroacétyl-2,6-diéthylaniline,
la N-benzoylméthyl-N-chloroacétyl-2-éthyl-6-méthylaniline,
la N-chloroacétyl-N-(5-méthyl-1,3,4-oxadiazol-2-yl)-2,6-diéthylaniline,
la N-chloroacétyl-N-(5-méthyl-1,3,4-oxadiazol-2-yl)-2-éthyl-6-méthylaniline,
la N-chloroacétyl-N-(5-méthyl-1,3,4-oxadiazol-2-yl)-2-tert-butylaniline,
la N-chloroacétyl-N-(4-chlorobenzoylméthyl)-2,6-diméthylaniline et
la N-chloroacétyl-N-(1-méthyl-5-méthylthio-1,3,4-triazol-2-ylméthyl)-2,6-diéthylaniline.

**47.** Agent selon la revendication 39, caractérisé en ce qu'il est utilisé pour protéger les plantes cultivées contre

l'action phytotoxique des herbicides acides aryloxyphénoxypropioniques de formule IV

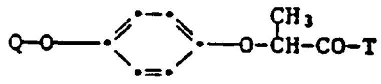

(IV)

où

Q représente le reste

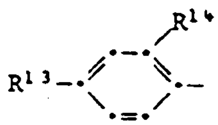 , 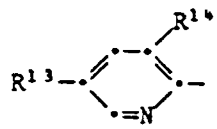 , 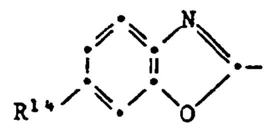 ,

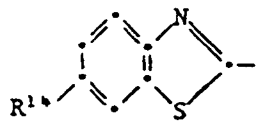 ou 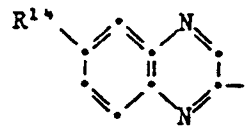

et T représente $-NR^{15}R^{66}$, $-N(CN)R^{17}$, $OR^{18}$, $SR^{18}$ ou $-O-N=CR^{19}R^{20}$

$R^{13}$ représentant un halogène ou le trifluorométhyle,

$R^{14}$ représentant l'hydrogène ou un halogène,

$R^{15}$ et $R^{16}$ représentant, indépendamment l'un de l'autre, l'hydrogène, un alcoxy en $C_1$-$C_8$, un alkyle en $C_1$-$C_8$, le phényle ou le benzyle, $R^{15}$ et $R^{16}$ représentant ensemble avec l'atome d'azote les portant, un hétérocycle azoté saturé à 5 ou 6 chaînons, pouvant être interrompu par un atome d'oxygène ou de soufre,

$R^{17}$ représentant un alkyle en $C_1$-$C_4$, un alcényle en $C_3$-$C_4$, un alcynyle en $C_3$-$C_4$ ou un alcoxy en $C_2$-$C_4$ alkyle,

$R^{18}$ représentant l'hydrogène ou un équivalent d'un ion d'un métal alcalin, d'un métal alcalino-terreux, du cuivre ou du fer ; un reste alkyle en $C_1$-$C_4$ ammonium ou hydroxyalkyle en $C_1$-$C_4$ ammonium quaternaire ; un reste alkyle en $C_1$-$C_9$ éventuellement substitué une ou plusieurs fois par un amino, un halogène, l'hydroxyle, le cyano, le nitro, le phényle, un alcoxy en $C_1$-$C_4$, un polyéthoxy ayant 2 à 6 unités d'oxyde d'éthylène, $-COOR^{21}$, $-COSR^{21}$, $-CONH_2$, -CON(alcoxy en $C_1$-$C_4$)alkyle en $C_1$-$C_4$, -CO-N-dialkyle en $C_1$-$C_4$, CONH-alkyle en $C_1$-$C_4$, -N(alcoxy en $C_1$-$C_4$)alkyle en $C_1$-$C_4$ ou un dialkyle en $C_1$-$C_4$ amino ; un reste alcényle en $C_3$-$C_9$ éventuellement substitué par un halogène ou par un alcoxy en $C_1$-$C_4$ ; un reste alcynyle en $C_3$-$C_9$ éventuellement substitué par un halogène ou par un alcoxy en $C_1$-$C_4$ ; un cycloalkyle en $C_3$-$C_9$ ; ou un phényle éventuellement substitué par le cyano, un alkyle en $C_1$-$C_4$, alcoxy en $C_1$-$C_4$, l'acétyle, $-COOR^{21}$, $COSR^{21}$, $-CONH_2$, -CON(alcoxy en $C_1$-$C_4$)alkyle en $C_1$-$C_4$, -CO-N-dialkyle en $C_1$-$C_4$ ou -CONH-alkyle en $C_1$-$C_4$,

$R^{19}$ et $R^{20}$ représentant, indépendamment l'un de l'autre, un alkyle en $C_1$-$C_4$ ou ensemble une chaîne alkylène à 3 à 6 chaînons et

$R^{21}$ représentant l'hydrogène, un alkyle en $C_1$-$C_6$, un halogénoalkyle en $C_1$-$C_6$, un alcoxy en $C_2$-$C_6$ alkyle, un alcényle en $C_3$-$C_6$, un halogénoalkyle en $C_3$-$C_6$, un alcynyle en $C_3$-$C_6$ ou un halogénoalcynyle en $C_3$-$C_6$.

**48.** Agent selon la revendication 47, caractérisé en ce que les dérivés acides aryloxyphénoxypropioniques de formule IV sont choisis dans le groupe constitué par
l'ester propargylique de l'acide 2-[4-(3,5-dichloro-pyridin-2-yloxy)-phénoxy]-propionique,
l'ester propargylique de l'acide 2-[4-(3,5-dichloro-pyridin-2-yloxy)-phénoxy]-thiopropionique,
l'ester méthylique de l'acide 2-[4-(5-chloro-3-fluoropyridin-2-yloxy)-phénoxy]-propionique,
l'ester butylique de l'acide 2-[4-(5-chloro-3-fluoro-pyridin-2-yloxy)-phénoxy]-propionique,
l'ester butylique de l'acide 2- [4-(5-trifluorométhyl-pyridin-2-yloxy)-phénoxy]-propionique,
l'ester méthylique de l'acide 2- [4-(5-trifluorométhyl-pyridin-2-yloxy)-phénoxy]-propionique,
l'ester éthylique de l'acide 2- [4-(6-chloroquinoxalin-2-yloxy)-phénoxy]-propionique et
l'ester éthylique de l'acide 2-[4-(6-chlorobenzoxazolin-2-yloxy)-phénoxy]-propionique.

**49.** Agent selon la revendication 38, caractérisé en ce qu'il est utilisé pour protéger les plantes cultivées contre

l'action phytotoxique des herbicides acylcyclohexanediones de formule IX

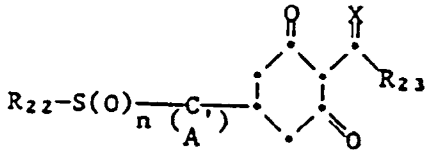

(IX)

où A' représente un pont alkylène ayant 2 à 7 chaînons, un pont alcénylène ayant 3 à 7 chaînons, pouvant être une ou plusieurs fois insaturé,

n' est égal à zéro, un ou deux,

$R_{22}$ représente un alkyle en $C_1$-$C_4$ ou le benzyle,

$R_{23}$ représente un alkyle en $C_1$-$C_6$, un alcoxy en $C_1$-$C_4$ non substitué ou substitué par un halogène, un alkyle en $C_1$-$C_4$ thio ; un cycloalkyle en $C_3$-$C_6$ ; le phényle, le benzyle ou le phényléthyle, le noyau phényle pouvant être substitué par un halogène, un alkyle en $C_1$-$C_4$, un alcoxy en $C_1$-$C_4$, un alkyle en $C_1$-$C_4$ thio, un halogénoalkyle en $C_1$-$C_4$, un halogénoalcoxy en $C_1$-$C_4$, le cyano ou le nitro ;

X' représente l'oxygène ou un reste -$NOR_{24}$ et

$R_{24}$ représente un alkyle en $C_1$-$C_4$, un halogénoalkyle en $C_1$-$C_6$, un alcényle en $C_3$-$C_6$, un halogénoalcényle en $C_3$-$C_6$ ou un alcynyle en $C_3$-$C_6$.

**50.** Agent selon la revendication 49, caractérisé en ce que les herbicides acylcyclohexanediones sont choisis dans le groupe constitué par
la 5-(1-méthylthiocyclobutan-1-yl)-2-(2,4-dichloro-benzoyl)-cyclohexane-1,3-dione,
la 5-(1-méthylthiocyclobutan-1-yl)-2-n-butyryl-cyclohexane-1,3-dione,
la 5-(1-méthylthiocyclobutan-1-yl)-2-cyclopropylcarbonyl-cyclohexane-1,3-dione,
la 5-(1-méthylthiocyclobutan-1-yl)-2-(2,3-dichloro-benzoyl)-cyclohexane-1,3-dione,
la 5-(1-méthylsulfonylcyclobutan-1-yl)-2-n-butyryl-cyclohexane-1,3-dione,
la 5-(1-méthylthiocyclopropan-1-yl)-2-propionyl-cyclohexane-1,3-dione,
la 5-(1-éthylthiocyclopropan-1-yl)-2-propionyl-cyclohexane-1,3-dione et
la 5-(1-méthylthiocyclopropan-1-yl)-2-n-butyryl-cyclohexane-1,3-dione,
la 5-(1-méthylthiocyclobutan-1-yl)-2-(1-éthoximino-n-butyryl)-cyclohexane-1,3-dione,
la 5-(1-méthylthiocyclopropan-1-yl)-2-(1-éthoximino-n-butyryl)-cyclohexane-1,3-dione,
la 5-(1-méthylthiocyclohexan-1-yl)-2-(1-éthoximino-n-butyryl)-cyclohexane-1,3-dione,
la 5-(1-méthylthiocyclobutan-1-yl)-2-(1-allyloximino-n-butyryl)-cyclohexane-1,3-dione et
la 5-(1-méthylthiocyclopentan-1-yl)-2-(1-éthoximino-n-butyryl)-cyclohexane-1,3-dione,
la 5-(1-méthylthiocyclopropan-1-yl)-2- [1-(trans-3-chloroallyloximino)-n-butyryl]-cyclohexane-1,3-dione,
la 5-(1-méthylthiocyclopropan-1-yl)-2- [1-(trans-3-chloroallyloximino)-propionyl]-cyclohexane-1,3-dione,
la 5-(1-méthylthiocyclopropan-1-yl)-2- [1-(cis-3-chloroallyloximino)-propionyl]-cyclohexane-1,3-dione et
la 5-(1-éthylthiocyclopropan-1-yl)-2- [1-(trans-3-chloroallyloximino)-propionyl]-cyclohexane-1,3-dione.

**51.** Utilisation des sulfamoylphénylurées de formule I selon la revendication 1 pour protéger les plantes cultivées contre l'action nuisible des herbicides sulfonylurées, des herbicides chloroacétanilides, des herbicides acylcyclohexanediones ou des herbicides acides aryloxyphénoxypropioniques.

**52.** Utilisation de sulfamoylphénylurées de formule I selon la revendication 1 pour protéger les plantes cultivées contre l'action nuisible des herbicides sulfonylurées, des herbicides chloroacétanilides ou des herbicides acides aryloxyphénoxypropioniques.

**53.** Utilisation selon la revendication 52, caractérisée en ce que les herbicides sulfonylurées sont des composés de formule II

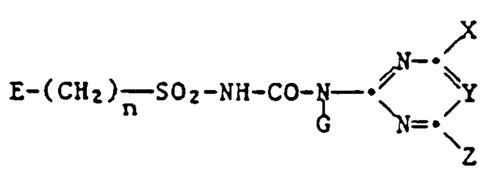

(II)

où
E représente un groupe

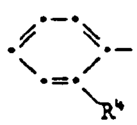 , 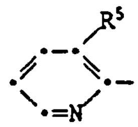 , 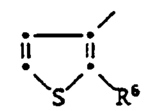 ,

ou

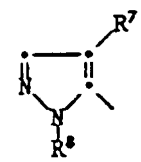 ,

n un nombre égal à 0 ou 1,
G l'hydrogène ou le méthyle,
X le méthoxy, l'éthoxy, le difluorométhyle, le méthyle ou le chlore,
Y CH ou N,
Z le méthoxy, le méthyle, le difluorométhoxy, le cyclopropyle ou le méthylamino,
$R^4$ un alcoxy en $C_2$-$C_5$ alcoxy, un halogénoalcoxy en $C_1$-$C_4$, un halogénoalkyle en $C_1$-$C_4$ thio, un halogénoalcényle en $C_2$-$C_4$, le chlore ou un alcoxy en $C_1$-$C_4$ carbonyle,
$R^5$ le trifluorométhyle ou un di(alkyle en $C_1$-$C_4$) carbamoyle,
$R^6$ un alcoxy en $C_1$-$C_4$ carbonyle,
$R^7$ un alcoxy en $C_1$-$C_4$ carbonyle et
$R^8$ un alkyle en $C_1$-$C_4$.

**54.** Utilisation selon la revendication 52, caractérisée en ce que les herbicides chloroacétanilides sont des composés de formule III

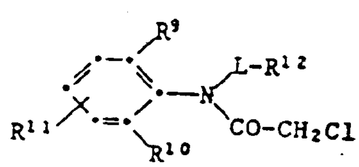 (III)

où
L représente un pont alkylène en $C_1$-$C_4$,
$R^9$, $R^{10}$ et $R^{11}$ représentent, indépendamment les uns des autres, l'hydrogène, un halogène, un alkyle en $C_1$-$C_4$, un alcoxy en $C_1$-$C_4$, un halogénoalkyle en $C_1$-$C_4$, un alcoxy en $C_2$-$C_5$ alkyle ou un alkyle en $C_2$-$C_5$ thioalkyle et
$R^2$ représente un alcoxy en $C_1$-$C_4$, -COOH, un alcoxy en $C_1$-$C_4$ carbonyle, $-CONH_2$, un alkyle en $C_1$-$C_4$ carbamoyle, un dialkyle en $C_1$-$C_4$ carbamoyle, le cyano, un alkyle en $C_1$-$C_4$ carbonyle,
le benzoyle éventuellement substitué,
le furyle éventuellement substitué,
le thiényle éventuellement substitué,
le pyrrolyle éventuellement substitué,
le pyrazolyle éventuellement substitué,
le 1,3,4-oxadiazol-2-yle éventuellement substitué,
le 1,3,4-thiadiazol-2-yle éventuellement substitué,
le 1,2,4-triazol-3-yle éventuellement substitué,
le dioxolanyle éventuellement substitué,
le dioxanyle éventuellement substitué,

le 1,3,4-triazol-2-yle éventuellement substitué ou le tétrahydrofuryle éventuellement substitué.

**55.** Utilisation selon la revendication 52, caractérisée en ce que les herbicides acides aryloxyphénoxypropioniques sont des composés de formule IV

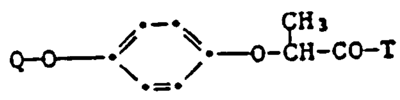

(IV)

où

Q représente le reste

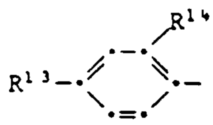

,

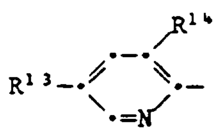

,

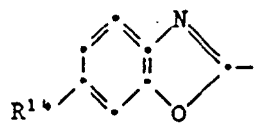

,

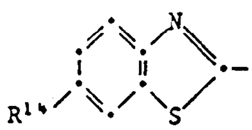

ou

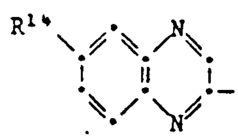

et T représente $-NR^{15}R^{16}$, $-N(CN)R^{17}$, $OR^{18}$, $SR^{18}$ ou $-O-N=CR^{19}R^{20}$,

$R^{13}$ représentant un halogène ou le trifluorométhyle,

$R^{14}$ représentant l'hydrogène ou un halogène,

$R^{15}$ et $R^{16}$ représentant, indépendamment l'un de l'autre, l'hydrogène, un alcoxy en $C_1$-$C_8$, un alkyle en $C_1$-$C_8$, le phényle ou le benzyle, $R^{15}$ et $R^{16}$ représentant ensemble avec l'atome d'azote les portant, un hétérocycle azoté saturé à 5 ou 6 chaînons, pouvant être interrompu par un atome d'oxygène ou de soufre,

$R^{17}$ représentant un alkyle en $C_1$-$C_4$, un alcényle en $C_3$-$C_4$, un alcynyle en $C_3$-$C_4$ ou un alcoxy en $C_2$-$C_4$ alkyle,

$R^{18}$ représentant l'hydrogène ou un équivalent d'un ion d'un métal alcalin, d'un métal alcalino-terreux, du cuivre ou du fer ; un reste alkyle en $C_1$-$C_4$ ammonium ou hydroxyalkyle en $C_1$-$C_4$ ammonium quaternaire ; un reste alkyle en $C_1$-$C_9$ éventuellement substitué une ou plusieurs fois par un amino; un halogène, l'hydroxyle, le cyano, le nitro, le phényle, un alcoxy en $C_1$-$C_4$, un polyéthoxy ayant 2 à 6 unités d'oxyde d'éthylène, $-COOR^{21}$, $-COSR^{21}$, $-CONH_2$, -CON(alcoxy en $C_1$-$C_4$)alkyle en $C_1$-$C_4$, -CO-N-dialkyle en $C_1$-$C_4$, CONH-alkyle en $C_1$-$C_4$, -N(alcoxy en $C_1$-$C_4$)alkyle en $C_1$-$C_4$ ou un dialkyle en $C_1$-$C_4$ amino; un reste alcényle en $C_3$-$C_9$ éventuellement substitué par un halogène ou par un alcoxy en $C_1$-$C_4$ ; un reste alcynyle en $C_3$-$C_9$ éventuellement substitué par un halogène ou par un alcoxy en $C_1$-$C_4$ ; un cycloalkyle en $C_3$-$C_9$ : ou un phényle éventuellement substitué par le cyano, un alkyle en $C_1$-$C_4$, un alcoxy en $C_1$-$C_4$, l'acétyle, -$COOR^{21}$, $COSR^{21}$, $-CONH_2$, CON(alcoxy en $C_1$-$C_4$)alkyle en $C_1$-$C_4$, -CO-N-dialkyle en $C_1$-$C_4$ ou -CONH-alkyle en $C_1$-$C_4$;

$R^{19}$ et $R^{20}$ représentant, indépendamment l'un de l'autre, un alkyle en $C_1$-$C_4$ ou ensemble une chaîne alkylène à 3 à 6 chaînons et

$R^{21}$ représentant l'hydrogène, un alkyle en $C_1$-$C_6$, un halogénoalkyle en $C_1$-$C_6$, un alcoxy en $C_2$-$C_6$ alkyle, un alcényle en $C_3$-$C_6$, un halogénoalkyle en $C_3$-$C_6$, un alcynyle en $C_3$-$C_6$ ou un halogénoalcynyle en $C_3$-$C_6$.

**56.** Utilisation selon la revendication 51, caractérisée en ce que les herbicides acylcyclohexanediones sont des composés de formule IX

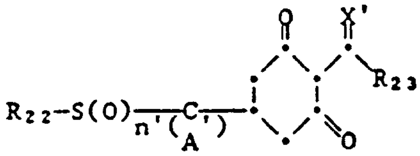

(IX)

où A' représente un pont alkylène ayant 2 à 7 chaînons, un pont alcénylène ayant 3 à 7 chaînons, pouvant être une ou plusieurs fois insaturé,
n' est égal à zéro, un ou deux,
$R_{22}$ représente un alkyle en $C_1$-$C_4$ ou le benzyle,
$R_{23}$ représente un alkyle en $C_1$-$C_6$, non substitué ou substitué par un halogène, un alcoxy en $C_1$-$C_4$, allyle en $C_1$-$C_4$ thio ; un cycloalkyle en $C_3$-$C_6$ ; le phényle, le benzyle ou le phényléthyle, le noyau phényle pouvant être substitué par un halogène, un alkyle en $C_1$-$C_4$, un alcoxy en $C_1$-$C_4$, un alkyle en $C_1$-$C_4$ thio, un halogénoalkyle en $C_1$-$C_4$, un halogénoalcoxy en $C_1$-$C_4$; le cyano ou le nitro ;
X' représente l'oxygène ou un reste -$NOR_{24}$ et
$R_{24}$ représente un alkyle en $C_1$-$C_6$, un halogénoalkyle en $C_1$-$C_6$, un alcényle en $C_3$-$C_6$, un halogénoalcényle en $C_3$-$C_6$ ou un alcynyle en $C_3$-$C_6$.

**57.** Agent herbicide pour lutter sélectivement contre les mauvaises herbes dans des cultures de plantes utiles, caractérisé en ce qu'il contient, comme substances actives, aussi bien un herbicide sulfonylurée, un herbicide acylcyclohexanedione, un herbicide chloroacétanilide ou un herbicide acide aryloxyphénoxypropionique qu'un antidote de formule I selon la revendication 1.

**58.** Agent herbicide pour lutter sélectivement contre les mauvaises herbes dans les cultures de plantes utiles, caractérisé en ce qu'il contient, comme substances actives, aussi bien un herbicide sulfonylurée, un herbicide chloroacétanilide ou un herbicide acide aryloxyphénoxypropionique qu'un antidote de formule I selon la revendication 1.

**59.** Agent selon la revendication 57, caractérisé en ce que l'herbicide acylcyclohexanedione est un composé de formule IX

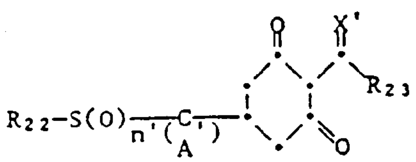

(IX)

où A' représente un pont alkylène ayant 2 à 7 chaînons, un pont alcénylène ayant 3 à 7 chaînons, pouvant être une ou plusieurs fois insaturé,
n' est égal à zéro, un ou deux,
$R_{22}$ représente un alkyle en $C_1$-$C_4$ ou le benzyle,
$R_{23}$ représente un alkyle en, $C_1$-$C_6$, non substitué ou substitué par un halogène, un alcoxy en $C_1$-$C_4$, alkyle en $C_1$-$C_4$ thio ; un cycloalkyle en $C_3$-$C_6$; le phényle, le benzyle ou le phényléthyle, le noyau phényle pouvant être substitué par un halogène, un alkyle en $C_1$-$C_4$, un alcoxy en $C_1$-$C_4$, un alkyle en $C_1$-$C_4$ thio, un halogénoalkyle en $C_1$-$C_4$, un halogénoalcoxy en $C_1$-$C_4$; le cyano ou le nitro ;
X' représente l'oxygène ou un reste -$NOR_{24}$ et
$R_{24}$ représente un alkyle en $C_1$-$C_6$, un halogénoalkyle en $C_1$-$C_6$, un alcényle en $C_3$-$C_6$, un halogénoalcényle en $C_3$-$C_6$ ou un alcynyle en $C_3$-$C_6$.

**60.** Agent selon la revendication 58, caractérisé en ce que l'herbicide sulfonylurée est un composé de formule II

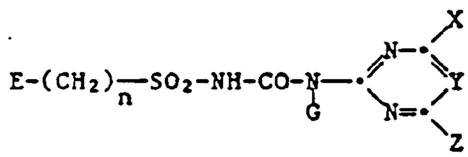 (II)

où

E représente un groupe

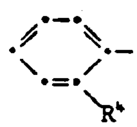 , 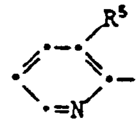 , 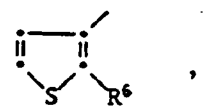 ,

ou

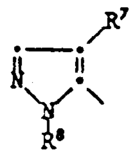 ,

n un nombre égal à 0 ou 1,
G l'hydrogène ou le méthyle,
X le méthoxy, l'éthoxy, le difluorométhyle, le méthyle ou le chlore,
Y CH ou N
Z le méthoxy, le méthyle, le difluorométhoxy, le cyclopropyle ou le méthylamino,
$R^4$ un alcoxy en $C_2$-$C_5$ alcoxy, un halogénoalcoxy en $C_1$-$C_4$, un halogénoalkyle en $C_1$-$C_4$ thio, un halogénoalcényle en $C_2$-$C_4$, le chlore ou un alcoxy en $C_1$-$C_4$ carbonyle,
$R^5$ le trifluorométhyle ou un di(alkyle en $C_1$-$C_4$) carbamoyle,
$R^6$ un alcoxy en $C_1$-$C_4$ carbonyle,
$R^7$ un alcoxy en $C_1$-$C_4$ carbonyle et
$R^8$ un alkyle en $C_1$-$C_4$.

**61.** Agent selon la revendication 58, caractérisé en ce que l'herbicide chloroacétanilide est un composé de formule III

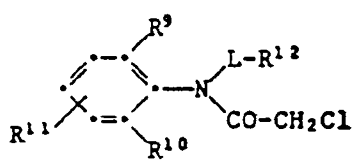 (III)

où

L représente un pont alkylène en $C_1$-$C_4$,

$R^9$, $R^{10}$ et $R^{11}$ représentent, indépendamment les uns des autres, l'hydrogène, un halogène, un alkyle en $C_1$-$C_4$, un alcoxy en $C_1$-$C_4$, un halogénoalkyle en $C_1$-$C_4$, un alcoxy en $C_2$-$C_5$ alkyle ou un alkyle en $C_2$-$C_5$ thioalkyle et

$R^{12}$ représente un alcoxy en $C_1$-$C_4$, -COOH, un alcoxy en $C_1$-$C_4$ carbonyle, -$CONH_2$, un alkyle en $C_1$-$C_4$ carbamoyle, un dialkyle en $C_1$-$C_4$ carbamoyle, le cyano, un alkyle en $C_1$-$C_4$ carbonyle,
le benzoyle éventuellement substitué,
le furyle éventuellement substitué,
le thiényle éventuellement substitué,
le pyrrolyle éventuellement substitué,

le pyrazolyle éventuellement substitué,
le 1,3,4-oxadiazol-2-yle éventuellement substitué,
le 1,3,4-thiadiazol-2-yle éventuellement substitué,
le 1,2,4-triazol-3-yle éventuellement substitué,
le dioxolanyle éventuellement substitué,
le dioxanyle éventuellement substitué,
le 1,3,4-triazol-2-yle éventuellement substitué ou le tétrahydrofuryle éventuellement substitué.

**62.** Agent selon la revendication 58, caractérisé en ce que l'herbicide acides aryloxyphénoxypropioniques est un composé de formule IV

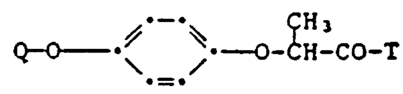

(IV)

où
Q représente le reste

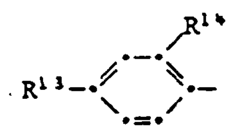
,
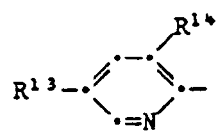
,
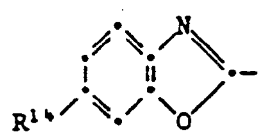
,
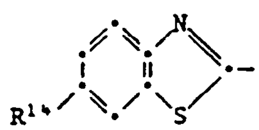
ou
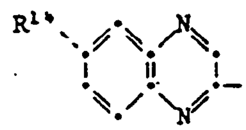

et T représente $-NR^{15}R^{16}$, $-N(CN)R^{17}$, $OR^{18}$, $SR^{18}$ ou $-O-N=CR^{19}R^{20}$

$R^{13}$ représentant un halogène ou le trifluorométhyle,

$R^{14}$ représentant l'hydrogène ou un halogène,

$R^{15}$ et $R^{16}$ représentant, indépendamment l'un de l'autre, l'hydrogène, un alcoxy en $C_1$-$C_8$, un alkyle en $C_1$-$C_8$, le phényle ou le benzyle, $R^{15}$ et $R^{16}$ représentant ensemble avec l'atome d'azote les portant, un hétérocycle azoté saturé à 5 ou 6 chaînons, pouvant être interrompu par un atome d'oxygène ou de soufre,

$R^{17}$ représentant un alkyle en $C_1$-$C_4$, un alcényle en $C_3$-$C_4$, un alcynyle en $C_3$-$C_4$ ou un alcoxy en $C_2$-$C_4$ alkyle,

$R^{18}$ représentant l'hydrogène ou un équivalent d'un ion d'un métal alcalin, d'un métal alcalino-terreux, du cuivre ou du fer ; un reste alkyle en $C_1$-$C_4$ ammonium ou hydroxyalkyle en $C_1$-$C_4$ ammonium quaternaire ; un reste alkyle en $C_1$-$C_9$ éventuellement substitué une ou plusieurs fois par un amino, un halogène, l'hydroxyle, le cyano, le nitro, le phényle, un alcoxy en $C_1$-$C_4$, un polyéthoxy ayant 2 à 6 unités d'oxyde d'éthylène, $-COOR^{21}$, $-COSR^{21}$, $-CONH_2$, -CON(alcoxy en $C_1$-$C_4$, -CO-N-dialkyle en $C_1$-$C_4$, CONH-alkyle en $C_1$-$C_4$, -N(alcoxy en $C_1$-$C_4$) alkyle en $C_1$-$C_4$ ou un dialkyle en $C_1$-$C_4$ amino ; un reste alcényle en $C_3$-$C_9$ éventuellement substitué par un halogène ou par un alcoxy en $C_1$-$C_4$ ; un reste alcynyle en $C_3$-$C_9$ éventuellement substitué par un halogène ou par un alcoxy en $C_1$-$C_4$ ; un cycloalkyleen $C_3$-$C_9$ ; ou un phényle éventuellement substitué par le cyano, un ,alkyle en $C_1$-$C_4$, un alcoxy en $C_1$-$C_4$, l'acétyle, -$COOR^{21}$, $COSR^{21}$, $-CONH_2$, -CON(alcoxy en $C_1$-$C_4$) alkyle en $C_1$-$C_4$, -CO-N-dialkyle en $C_1$-$C_4$ ou -CONH-alkyle en $C_1$-$C_4$,

$R^{19}$ et $R^{20}$ représentant, indépendamment l'un de l'autre, un alkyle en $C_1$-$C_4$ ou ensemble une chaîne alkylène à 3 à 6 chaînons et

$R^{21}$ représentant l'hydrogène, un alkyle en $C_1$-$C_6$, un halogénoalkyle en $C_1$-$C_6$, un alcoxy en $C_2$-$C_6$ alkyle, un alcényle en $C_3$-$C_6$, un halogénoalkyle en $C_3$-$C_6$, un alcynyle en $C_3$-$C_6$ ou un halogéno-alcynyle en $C_3$-$C_6$.

**63.** Procédé pour lutter sélectivement contre les mauvaises herbes dans les cultures de plantes utiles, caractérisé en ce que l'on traite les plantes cultivées ou les surfaces cultivées avec ces plantes aussi bien avec

un herbicide sulfonylurée, un herbicide acylcyclohexanedione, un herbicide chloroacétanilide ou un herbicide acide aryloxyphénoxypropionique qu'avec une quantité efficace de N-acylsulfamoyl-phénylurée de formule I selon la revendication 1.

**64.** Procédé pour protéger les plantes cultivées contre les dommages intervenant lors de l'application des herbicides sulfonylurées, des herbicides acylcyclohexanediones, des herbicides chloroacétanilides ou des herbicides acides aryloxyphénoxypropioniques, caractérisé en ce que l'on traite, ou la surface cultivée pour la plante avant ou pendant l'application de l'herbicide ou les semences ou les boutures ou les plantes elles-mêmes avec une quantité efficace d'une N-acylsulfamoylphénylurée de formule I selon la revendication 1.

**65.** Procédé pour lutter sélectivement contre les mauvaises herbes dans les cultures de plantes utiles, caractérisé en ce que l'on traite les plantes cultivées ou les surfaces cultivées avec ces plantes aussi bien avec un herbicide sulfonylurée, un herbicide chloroacétanilide ou un herbicides acides aryloxyphénoxypropioniques qu'avec une quantité efficace d'une N-acylsulfamoylphénylurée de formule I selon la revendication 1 comme antidote.

**66.** Procédé pour protéger les plantes cultivées contre les dommages intervenant lors de l'application des herbicides sulfonylurées, des herbicides chloroacétanilides ou des herbicides acides aryloxyphénoxypropioniques, caractérisé en ce que l'on traite, ou la surface cultivée pour la plante avant ou pendant l'application de l'herbicide ou les semences ou les boutures des plantes ou les plantes elles-mêmes avec une quantité efficace d'une N-acylsulfamoylphénylurée de formule I selon la revendication 1.

**67.** Semences de plantes utiles, caractérisées en ce qu'elles ont été traitées avec une quantité efficace en tant qu'antagoniste d'une N-acylsulfamoyl-phénylurée de formule I selon la revendication 1.

**68.** Semences selon la revendication 67, caractérisées en ce que les semences sont des semences de céréales, soja, riz, maïs ou sorgho.

**69.** Semences selon la revendication 67, caractérisées en ce que les semences sont des semences de sorgho, maïs ou riz.

**70.** Procédé pour la préparation des sulfamoylphénylurées de formule I où $R^1$ a les significations données sous la formule I dans la revendication 1, à l'exception de l'hydrogène, et $R^2$ représente l'hydrogène, caractérisé en ce que l'on fait réagir une sulfamoylaniline de formule V

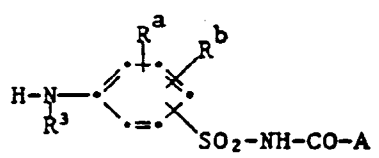

(V)

où A, $R^3$, $R^a$ et $R^b$ ont les significations données sous la formule I, avec un isocyanate de formule VI

$R^1$-N=C=O (VI)

où $R^1$ a les significations données sous la formule I, à l'exception de l'hydrogène.

**71.** Procédé pour la préparation des composés de formule I selon la revendication 1, caractérisé en ce que l'on acyle une sulfamoylphénylurée de formule VII

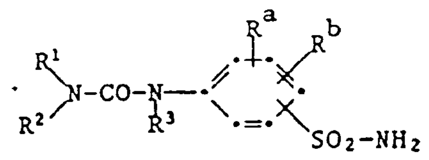

(VII)

où $R^1$, $R^2$, $R^3$, $R^a$ et $R^b$ ont les significations données sous la formule I, avec un halogénure d'acide carboxylique de formule VIII

Hal-CO-A (VIII)

où A a les significations données sous la formule I et Hal représente le chlore ou le brome.

**72.** La 1-[4-(N-2-éthoxybenzoyl-sulfamoyl)-phényl]-3-méthylurée.

## Revendications pour l'Etat Contractant suivant:ES

**1.** Procédé pour la préparation des composés de formule I

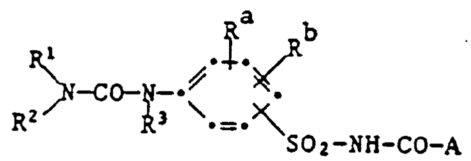

(I)

où
A représente un reste provenant du groupe

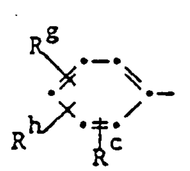
,
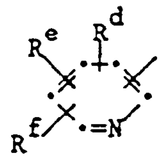
,
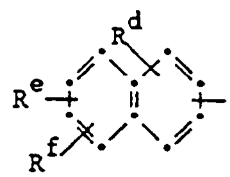
,
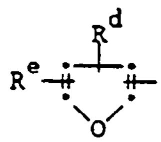
ou
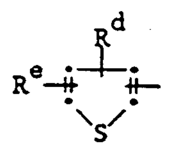
,

$R^1$ et $R^2$ représentent, indépendamment l'un de l'autre, l'hydrogène, un alkyle en $C_1$-$C_8$, un cycloalkyle en $C_3$-$C_8$, un alcényle en $C_3$-$C_6$, un alcynyle en $C_3$-$C_6$,

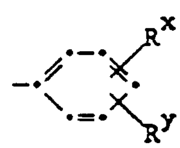

ou un alkyle en $C_1$-$C_4$ substitué par un alcoxy en $C_1$-$C_4$ ou par

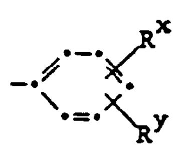

ou

$R^1$ et $R^2$ représentent ensemble un pont alkylène en $C_4$-$C_6$ ou un pont alkylène en $C_4$-$C_6$ interrompu par l'oxygène le soufre, SO, $SO_2$, NH ou N-(alkyle en $C_1$-$C_4$),
$R^3$ représente l'hydrogène ou un alkyle en $C_1$-$C_4$,
$R^a$ et $R^b$ représentent, indépendamment l'un de l'autre, l'hydrogène, un halogène, un cyano, un nitro, le trifluorométhyle, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, $-COOR^j$, $-CONR^4R^m$, $-COR^m$, $-SO_2NR^kR^m$ ou $-OSO_2$ alkyle en $C_1$-$C_4$, ou $R^a$ et $R^b$ représentent ensemble un pont alkylène en $C_3$-$C_4$, pouvant être substitué par un halogène ou par un alkyle en $C_1$-$C_4$ ou un pont alcénylène en $C_3$-$C_4$ pouvant être substitué par un halogène ou par un alkyle en $C_3$-$C_4$ ou un pont alcadiénylène en $C_4$ pouvant être substitué par un halogène ou par un alkyle en $C_1$-$C_4$,
$R^g$ et $R^h$ représentent, infépendamment l'un de l'autre, l'hydrogène, un halogène, un alkyle en $C_1$-$C_4$, le

trifluorométhyle, le méthoxy, le méthylthio ou --COOR$^j$,
$R^c$ représentant l'hydrogène, un halogène, un alkyle en $C_1$-$C_4$ ou le méthoxy,
$R^d$ représentant l'hydrogène, un halogène, le nitro, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, -COOR$^j$ ou -CONR$^k$R$^m$,
$R^e$ représentant l'hydrogène, un halogène, un alkyle en $C_1$-$C_4$, -COOR$^j$, le trifluorométhyle ou le méthoxy ou $R^d$ et $R^e$ représentant ensemble un pont alkylène en $C_3$-$C_4$,
$R_f$ représentant l'hydrogène, un halogène ou un alkyle en $C_1$-$C_4$,
$R^x$ et $R^y$ représentant, indépendamment l'un de l'autre, l'hydrogène, un halogène, un alkyle en $C_1$-$C_4$, un alcoxy en $C_1$-$C_4$, un alkyle en $C_1$-$C_4$ thio, -COOR$^4$, le trifluorométhyle, le nitro ou le cyano,
$R^j$, $R^k$ et $R^m$ représentant, indépendamment les uns des autres, l'hydrogène ou un alkyle en $C_1$-$C_4$,
$R^k$ et $R^m$ représentant ensemble un pont alkylène en $C_4$-$C_6$ ou un pont alkylène en $C_4$-$C_6$ interrompu par l'oxygène, NH ou -N(alkyle en $C_1$-$C_4$) et
Rn représentant un alkyle en $C_1$-$C_4$, le phényle ou le phényle substitué par un halogène, un alkyle en $C_1$-$C_4$, le méthoxy, le nitro ou le trifluorométhyle, caractérisé en ce que l'on acyle une sulfamoylphénylurée de formule VII

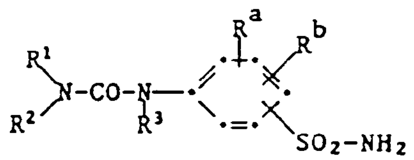

(VII)

où $R^1$, $R^2$, $R^3$, $R^a$ et $R^b$ ont les significations données sous la formule I, avec un halogénure d'acide carboxylique de formule VIII

Hal-CO-A (VIII)

où A a les définitions données sous la formule I et Hal représente le chlore ou le brome.

2. Procédé selon la revendication 1 pour la préparation des composés de formule I dans laquelle A représente un reste provenant du groupe

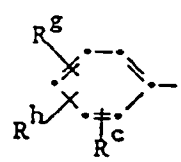
,
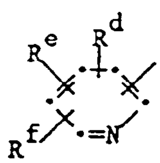
,
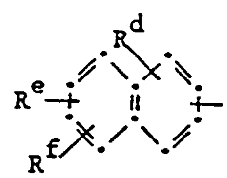
,
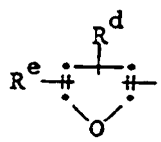
ou
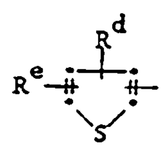
,

$R^1$ et $R^2$ représentent, indépendamment l'un de l'autre, l'hydrogène, un alkyle en $C_1$-$C_8$, un cycloalkyle en $C_3$-$C_8$, un alcényle en $C_3$-$C_6$, un alcynyle en $C_3$-$C_6$,

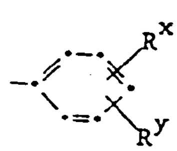

ou un alkyle en $C_1$-$C_4$ substitué par un alcoxy en $C_1$-$C_4$ ou par

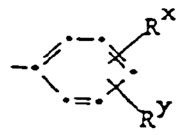

ou

$R^1$ et $R^2$ représentent ensemble un pont alkylène en $C_4$-$C_6$ ou un pont alkylène en $C_4$-$C_6$ interrompu par l'oxygène, le soufre, SO, $SO_2$, NH ou N-(alkyle en $C_1$-$C_4$),
$R^3$ représente l'hydrogène ou un alkyle en $C_1$-$C_4$,
$R^a$ et $R^b$ représentent, indépendamment l'un de l'autre, l'hydrogène, un halogène, un cyano, un nitro, le trifluorométhyle, un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, $-COOR^j$, $-CONR^kR^m$, $-COR^m$, $-SO_2NR^kR^m$ ou $-OSO_2$ alkyle en $C_1$-$C_4$ et
$R^g$ et $R^h$ représentent, indépendamment l'un de l'autre, l'hydrogène, un halogène, un alkyle en $C_1$-$C_4$, le trifluorométhyle, le méthoxy, le méthylthio ou $--COOR^j$,
$R^c$ représentant l'hydrogène, un halogène, un alkyle en $C_1$-$C_4$ ou le méthoxy,
$R^d$ représentant l'hydrogène, un halogène, le nitro un alkyle en $C_1$-$C_4$, un alcoxy en $C_1$-$C_4$, un alkyle en $C_1$-$C_4$ thio, un alkyle en $C_1$-$C_4$ sulfinyle, un alkyle en $C_1$-$C_4$ sulfonyle, $-COOR^j$ ou $CON^kR^m$,
$R^e$ représentant l'hydrogène, un halogène, un alkyle en $C_1$-$C_4$, $-COOR^j$, le trifluorométhyle ou le méthoxy ou $R^d$ et $R^e$ représentant ensemble un pont alkylène en $C_3$-$C_4$,
$R^f$ représentant l'hydrogène, un halogène ou un alkyle en $C_1$-$C_4$,
$R^x$ et $R^y$ représentant, indépendamment l'un de l'autre, l'hydrogène, un halogène, un alkyle en $C_1$-$C_4$, un alcoxy en $C_1$-$C_4$, un alkyle en $C_1$-$C_4$ thio, $-COOR^4$, le trifluorométhyle, le nitro ou le cyano,
$R^j$, $R^k$ et $R^m$ représentant, indépendamment les uns des autres, l'hydrogène ou un alkyle en $C_1$-$C_4$,
$R^k$ et $R^m$ représentant ensemble un pont alkylène en $C_4$-$C_6$ ou un pont alkylène en $C_4$-$C_6$ interrompu par l'oxygène, NH ou -N(alkyle en $C_1$-$C_4$) et
$R^n$ représentant un alkyle en $C_1$-$C_4$, le phényle ou le phényle substitué par un halogène, un alkyle en $C_1$-$C_4$, le méthoxy, le nitro ou le trifluorométhyle.

3. Procédé selon la revendication 1 pour la préparation des composés de formule I dans laquelle $R^b$ représente l'hydrogène.

4. Procédé selon la revendication 1 pour la préparation des composés de formule I dans laquelle le groupe sulfamoyle occupe la position 4 sur le noyau phényle.

5. Procédé selon la revendication 1 pour la préparation des composés de formule I dans laquelle $R^2$ et $R^3$ représentent l'hydrogène.

6. Procédé selon la revendication 1 pour la préparation des composés de formule I dans laquelle $R^b$ représente l'hydrogène et le groupe sulfamoyle occupe la position 4 sur le noyau phényle.

7. Procédé selon la revendication 1 pour la préparation des composés de formule I dans laquelle $R^2$, $R^3$ et $R^b$ représentent l'hydrogène et le groupe sulfamoyle occupe la position 4 sur le noyau phényle.

8. Procédé selon la revendication 1 pour la préparation des composés de formule I dans laquelle A représente le groupe

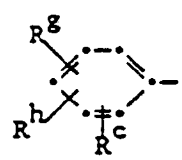

9. Procédé selon la revendication 8 pour la préparation des composés de formule I dans laquelle $R^2$, $R^3$ et $R^b$ représentent l'hydrogène et le groupe sulfamoyle occupe la position 4 sur le noyau phényle.

10. Procédé selon la revendication 1 pour la préparation des composés de formule I selon lequel ils appartiennent à la sous-formule Ia

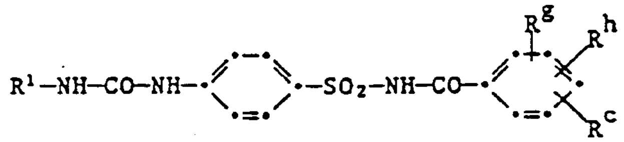

(Ia)

.

11. Procédé selon la revendication 10 pour la préparation des composés de sous-formule Ia dans laquelle $R^c$ représente l'hydrogène.

12. Procédé selon la revendication 11 pour la préparation des composés de sous-formule I dans laquelle $R^1$, $R^g$ et $R^h$ représentent un alkyle en $C_1$-$C_4$.

13. Procédé selon la revendication 1 pour la préparation de la 1-[4-(N-4-méthylbenzoyl-sulfamoyl)-phényl]-3-méthylurée.

14. Procédé selon , la revendication 1 pour la préparation de la 1-[4-(N-3-méthylbenzoyl-sulfamoyl)-phényl]-3-méthylurée.

15. Procédé selon la revendication 1 pour la préparation de la 1-[4-(N-4-tert-butyl-benzoyl-sulfamoyl)-phényl]-3-méthylurée.

16. Procédé selon la revendication 1 pour la préparation de la 1-[4-(N-3-trifluorométhyl-benzoyl-sulfamoyl)-phényl]-3-méthylurée.

17. Procédé selon la revendication 1 pour la préparation de la 1-[4-(N-4-nitrobenzoyl-sulfamoyl)-phényl]-3-méthylurée.

18. Procédé selon la revendication 1 pour la préparation de la 1-[4-(N-2,3-diméthylbenzoyl-sulfamoyl)-phényl]-3-méthylurée.

19. Procédé selon la revendication 1 pour la préparation de la 1-[4-(N-3,4-diméthylbenzoyl-sulfamoyl)-phényl]-3-méthylurée.

20. Procédé selon la revendication 1 pour la préparation de la 1-[4-(N-3,4-diméthylbenzoyl-sulfamoyl)-phényl]-3,3-diméthylurée.

21. Procédé selon la revendication 1 pour la préparation de la 1-[4-(N-3,4-diméthylbenzoyl-sulfamoyl)-phény]-3-éthylurée.

22. Procédé selon la revendication 1 pour la préparation de la 1-[4-(N-3,4-diméthylbenzoyl-sulfamoyl)-phényl]-3-allylurée.

23. Procédé selon la revendication 1 pour la préparation de la 1-[4-(N-3,4-diméthylbenzoyl-sulfamoyl)-phényl]-3-phénylurée.

24. Procédé selon la revendication 1 pour la préparation de la 1-[4-(N-3,5-diméthylbenzoyl-sulfamoyl)-phényl]-3-méthylurée.

25. Procédé selon la revendication 1 pour la préparation de la 1-[4-(N-3,4-dichlorobenzoyl-sulfamoyl)-phényl]-3-méthylurée.

26. Procédé selon la revendication 1 pour la préparation de la 1-[4-(N-3,4-diméthoxybenzoyl-sulfamoyl)-phényl]-3-méthylurée.

27. Procédé selon la revendication 1 pour la préparation de la 1-[4-(N-3,4-diméthoxybenzoyl-sulfamoyl)-phényl]-3,3-diméthylurée.

28. Procédé selon la revendication 1 pour la préparation de la 1-[4-(N-2,4,5-triméthoxy-benzoyl-sulfamoyl)-phényl]-3-méthylurée.

29. Procédé selon la revendication 1 pour la préparation de la 1-[4-(N-1-naphtylcarbonyl-sulfamoyl)-phényl]-3-méthylurée.

**30.** Procédé selon la revendication 1 pour la préparation de la 1-[4-(N-2-furylcarbonyl-sulfamoyl)-phényl]-3-méthylurée.

**31.** Procédé selon la revendication 1 pour la préparation de la 1-[4-(N-2-furylcarbonyl-sulfamoyl)-phényl]-3,3-diméthylurée.

**32.** Procédé selon la revendication 1 pour la préparation de la 1-[4-(N-2-thiénylcarbonyl-sulfamoyl)-phényl]-3-méthylurée.

**33.** Procédé selon la revendication 1 pour la préparation de la 1-[4-(N-pipéronyloyl-sulfamoyl)-phényl]-3-méthylurée.

**34.** Procédé selon la revendication 1 pour la préparation de la 1-[4-(N-3-méthylbenzoyl-sulfamoyl)-phényl]-3,3-diméthylurée.

**35.** Procédé selon la revendication 1 pour la préparation de la 1-[4-(N-3-trifluorométhyl-benzoyl-sulfamoyl)-phényl]-3-cyclopropylurée.

**36.** Procédé selon la revendication 1 pour la préparation de la 1-[3-(N-3,4-diméthylbenzoyl-sulfamoyl)-phényl]-3-méthylurée.

**37.** Procédé selon la revendication 1 pour la préparation de la 1-[3-(N-2-furylcarbonyl-sulfamoyl)-phényl]-3,3-diméthylurée.

**38.** utilisation des sulfamoylphénylurées de formule I selon la revendication 1 pour protéger les plantes cultivées contre l'action nuisible des herbicides sulfonylurées, des herbicides chloroacétanilides, des herbicides acylcyclohexanediones ou des herbicides acides aryloxyphénoxypropioniques.

**39.** Utilisation des sulfamoylphénylurées de formule I selon la revendication 1 pour protéger les plantes cultivées contre l'action nuisible des herbicides sulfonylurées, des herbicides chloroacétanilides ou des herbicides acides aryloxyphénoxypropioniques

**40.** Utilisation selon la revendication 39, caractérisée en ce que les herbicides sulfonylurées sont des composés de formule II

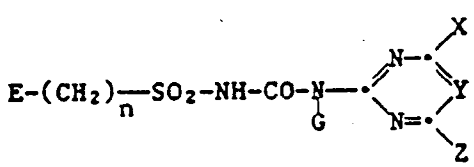

(II)

où
E représente un groupe

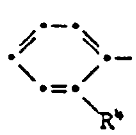 , 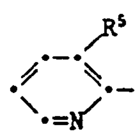 , 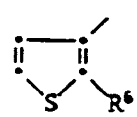 ,

ou

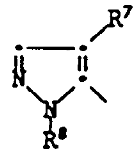

n un nombre égal à 0 ou 1,
G l'hydrogène ou le méthyle,
X le méthoxy, l'éthoxy, le difluorométhyle, le méthyle ou le chlore,
Y CH ou N,
Z le méthoxy, le méthyle, le difluorométhoxy, le cyclopropyle ou le méthylamino,
$R^4$ un alcoxy en $C_2$-$C_5$ alcoxy, un halogénoalcoxy en $C_1$-$C_4$, un halogénoalkyle en $C_1$-$C_4$ thio, un halogénoalcényle en $C_2$-$C_4$, le chlore ou un alcoxy en $C_1$-$C_4$ carbonyle,
$R^5$ le trifluorométhyle ou un di(alkyle en $C_1$-$C_4$) carbamoyle,
$R^6$ un alcoxy en $C_1$-$C_4$ carbonyle,
$R^7$ un alcoxy en $C_1$-$C_4$ carbonyle et
$R^8$ un alkyle en $C_1$-$C_4$.

**41.** Utilisation selon la revendication 39, caractérisée en ce que les herbicides chloroacétanides sont des composés de formule III

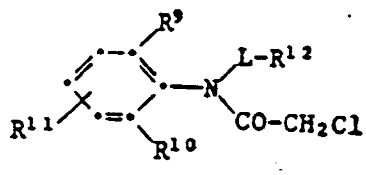

(III)

où
L représente un pont alkylène en $C_1$-$C_4$,
$R^9$, $R^{10}$ et $R^{11}$ représentent, indépendamment les uns des autres, l'hydrogène, un halogène, un alkyle en $C_1$-$C_4$, un alcoxy en $C_1$-$C_4$, un halogénoalkyle en $C_1$-$C_4$, un alcoxy en $C_2$-$C_5$ alkyle ou un alkyle en $C_2$-$C_5$ thioalkyle et représente un alcoxy en $C_1$-$C_4$, --COOH, un alcoxy en $C_1$-$C_4$ carbonyle, -$CONH_2$, un alkyle en $C_1$-$C_4$ carbamoyle, un dialkyle en $C_1$-$C_4$ carbamoyle,
le cyano, un alkyle en $C_1$-$C_4$ carbonyle,
le benzoyle éventuellement substitué,
le furyle éventuellement substitué,
le thiényle éventuellement substitué,
le pyrrolyle éventuellement substitué,
le pyrazolyle éventuellement substitué,
le 1,3,4-oxadiazol-2-yle éventuellement substitué,
le 1,3,4-thiadiazol-2-yle éventuellement substitué,
le 1,2,4-triazol-3-yle éventuellement substitué,
le dioxolanyle éventuellement substitué,
le dioxanyle éventuellement substitué,
le 1,3,4-triazol-2-yle éventuellement substitué ou le tétrahydrofuryle éventuellement substitué.

**42.** Utilisation selon la revendication 39, caractérisée en ce que les herbicides acides aryloxyphénoxypropioniques sont des composés de formule IV

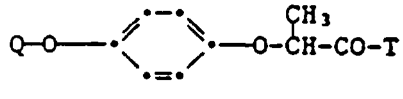

(IV)

où
Q représente le reste

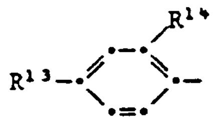 , 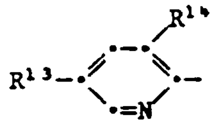 , 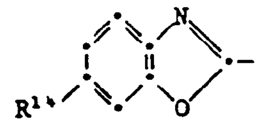 ,

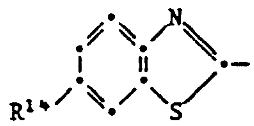 ou 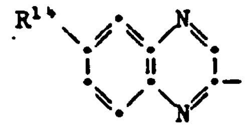

et T représente $-NR^{15}R^{16}$, $-N(CN)R^{17}$, $OR^{18}$, $SR^{18}$ ou $-O-N=CR^{19}R^{20}$,

$R^{13}$ représentant un halogène ou le trifluorométhyle,

$R^{14}$ représentant l'hydrogène ou un halogène,

$R^{15}$ et $R^{16}$ représentant, indépendamment l'un de l'autre, l'hydrogène, un alcoxy en $C_1$-$C_8$, un alkyle en $C_1$-$C_8$, le phényle ou le benzyle, $R^{15}$ et $R^{16}$ représentant ensemble avec l'atome d'azote les portant, un hétérocycle azoté saturé à 5 ou 6 chaînons, pouvant être interrompu par un atome d'oxygène ou de soufre,

$R^{17}$ représentant un alkyle en $C_1$-$C_4$, un alcényle en $C_3$-$C_4$, un alcynyle en $C_3$-$C_4$ ou un alcoxy en $C_2$-$C_4$ alkyle,

$R^{18}$ représentant l'hydrogène ou un équivalent d'un ion d'un métal alcalin, d'un métal alcalino-terreux, du cuivre ou du fer ; un reste alkyle en $C_1$-$C_4$ ammonium ou hydroxyalkyle en $C_1$-$C_4$ ammonium quaternaire ; un reste alkyle en $C_1$-$C_9$ éventuellement substitué une ou plusieurs fois par un amino, un halogène, l'hydroxyle, le cyano, le nitro, le phényle, un alcoxy en $C_1$-$C_4$, un polyéthoxy ayant 2 à 6 unités d'oxyde d'éthylène, $-COOR^{21}$, $-COSR^{21}$, $-CONH_2$, -CON(alcoxy en $C_1$-$C_4$)alkyle en $C_1$-$C_4$, -CO-N-dialkyle en $C_1$-$C_4$, CONH-alkyle en $C_1$-$C_4$, -N(alcoxy en $C_1$-$C_4$) alkyle en $C_1$-$C_4$ ou un dialkyle en $C_1$-$C_4$ amino ; un reste alcényle en $C_3$-$C_9$ éventuellement substitué par un halogène ou par un alcoxy en $C_1$-$C_4$ ; un reste alcynyle en $C_3$-$C_9$ éventuellement substitué par un halogène ou par un alcoxy en $C_1$-$C_4$ ; un cycloalkyle en $C_3$-$C_9$ ; ou un phényle éventuellement substitué par le cyano, un alkyle en $C_1$-$C_4$, un alcoxy en $C_1$-$C_4$, l'acétyle, -$COOR^{21}$, $COSR^{21}$, $-CONH_2$, -CON(alcoxy en $C_1$-$C_4$)alkyle en $C_1$-$C_4$, -CO-N-dialkyle en $C_1$-$C_4$ ou -CONH-alkyle en $C_1$-$C_4$,

$R^{19}$ et $R^{20}$ représentant, indépendamment l'un de l'autre un alkyle en $C_1$-$C_4$ ou ensemble une chaîne alkylène à 3 ou 6 chaînons et

$R^{21}$ représentant l'hydrogène, un alkyle en $C_1$-$C_6$, un halogénoalkyle en $C_1$-$C_6$, un alcoxy en $C_2$-$C_6$ alkyle, un alcényle en $C_3$-$C_6$, un halogénoalkyle en $C_3$-$C_6$, un alcynyle en $C_3$-$C_6$ ou un halogénoalcynyle en $C_3$-$C_6$.

**43.** Utilisation selon la revendication 38, caractérisée en ce que les herbicides acylcyclohexanediones sont des composés de formule IX

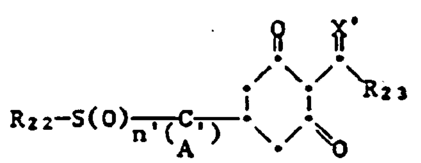

(IX)

où A' représente un pont alkylène ayant 2 à 7 chaînons, un pont alcénylène ayant 3 à 7 chaînons, pouvant être une ou plusieurs fois insaturé,

n' est égal à zéro, un ou deux,

$R_{22}$ représente un alkyle en $C_1$-$C_4$ ou le benzyle,

$R_{23}$ représente un alkyle en $C_1$-$C_6$, non substitué ou substitué par un halogène, un alcoxy en $C_1$-$C_4$ alkyle en $C_1$-$C_4$ thio ; un cycloalkyle en $C_3$-$C_6$ ; le phényle, le benzyle ou le phényléthyle, le noyau phényle pouvant être substitué par un halogène, un alkyle en $C_1$-$C_4$, un alcoxy en $C_1$-$C_4$, un alkyle en $C_1$-$C_4$ thio, un halogénoalkyle en $C_1$-$C_4$, un halogéno-alcoxy en $C_1$-$C_4$ : le cyano ou le nitro ;

X' représente l'oxygène ou un reste $-NOR_{24}$ et

$R_{24}$ représente un alkyle en $C_1$-$C_6$, un halogénoalkyle en $C_1$-$C_6$, un alcényle en $C_3$-$C_6$, un halogénoalcényle en $C_3$-$C_6$ ou un alcynyle en $C_3$-$C_6$.

**44.** Procédé selon la revendication 1 pour la préparation de la 1-[4-(N-2-éthoxybenzoyl-sulfamoyl)-phényl]-3-méthylurée.